# EUROPEAN PATENT APPLICATION

(11) **EP 4 768 481 A1**
(43) Date of publication of application: **01.07.2026**
(21) Application number: 24884444.1
(22) Date of filing: 17.10.2024
(51) Int. Cl.: C07D 413/12, C09K 11/06, H10K 85/60, H10K 50/11

(54) **DEUTERATED COMPOUND, LIGHT-EMITTING HOST MATERIAL COMPOSITION AND LIGHT-EMITTING DEVICE COMPRISING SAME, AND USE**

(30) Priority: 31.10.2023 CN 202311444258
(71) Applicant: Ningbo Lumilan Advanced Materials Co., Ltd., Ningbo, Zhejiang 315000 (CN)
(72) Inventor: WEI, Ting-Wei, Ningbo, Zhejiang 315000 (CN); TANG, Cong, Ningbo, Zhejiang 315000 (CN); LIU, Fengjiao, Ningbo, Zhejiang 315000 (CN); XIAO, Yang, Ningbo, Zhejiang 315000 (CN); XU, Binyu, Ningbo, Zhejiang 315000 (CN); DING, Huanda, Ningbo, Zhejiang 315000 (CN); CHEN, Zhikuan, Ningbo, Zhejiang 315000 (CN)
(74) Representative: Murgitroyd & Company
(86) International application number: PCT/CN2024/125458
(87) International publication number: WO 2025/092440

(57) **Abstract**

The present application relates to the technical field of displays, and specifically relates to a deuterated compound, a light-emitting host material composition and a light-emitting device comprising the same, and uses thereof. The deuterated compound provided in the present application has the structure as shown below: 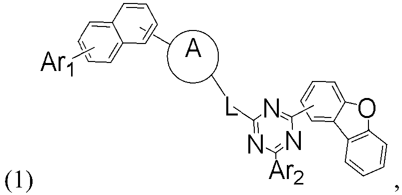 and a substituent comprises deuterium,, such that an organic electroluminescent device comprising the deuterated compound can have a relatively high light-emitting efficiency and a relatively long service time.

## Description

### CROSS REFERENCE TO RELATED APPLICATIONS

This application claims the priority of Chinese Patent Application NO. 202311444258.6 filed on 31 October 2023, titled "DEUTERATED COMPOUND, LIGHT-EMITTING HOST MATERIAL COMPOSITION AND LIGHT-EMITTING DEVICE COMPRISING THE SAME, AND USES THEREOF," the contents of which are incorporated by reference in its entirety herein.

### TECHNICAL FIELD

The present application relates to the technical field of displays, and specifically to deuterated compound, as well as light-emitting host material composition and light-emitting device comprising the same, and uses thereof.

### BACKGROUND

An organic light-emitting diode (OLED) is a device that converts electrical energy into light by applying an electric current to organic electroluminescent materials, and typically has a structure comprising an anode, a cathode, and an organic layer between the anode and cathode. The organic layer of the organic electroluminescence (EL) device can be composed of a hole injection layer, a hole transport layer, an electron blocking layer, a light-emitting layer (containing a host material and a dopant material), an electron buffer layer, a hole blocking layer, an electron transport layer, an electron injection layer and the like. Materials used for the organic layer are classified according to their functions as hole injection materials, hole transport materials, electron blocking materials, light-emitting materials, electron buffer materials, hole blocking materials, electron transport materials, electron injection materials and the like. In the organic EL device, upon application of voltage, holes are injected from the anode into the light-emitting layer, and electrons are injected from the cathode into the light-emitting layer, forming high-energy excitons through recombination of holes and electrons. This energy excites an organic luminescent compound to an excited state, and light is emitted when the excited state of the organic luminescent compound returns to the ground state, resulting in luminescence.

The most critical factor determining the luminance efficiency of an organic EL device is the light-emitting material. The light-emitting material must exhibit high quantum efficiency and high electron and hole mobility, and the formed light-emitting material layer must be uniform and stable. The light-emitting materials are categorized by the color of emitted light into blue-emitting materials, green-emitting materials, red-emitting materials, as well as yellow-emitting or orange-emitting materials. Additionally, the light-emitting materials can be categorized by function into host materials and dopant materials.

However, existing organic electroluminescent materials suffer from low stability and low quantum yield of the molecular, leading to low luminance efficiency and short lifetime in organic electroluminescent devices comprising the organic electroluminescent materials, severely limiting their applications.

### SUMMARY

The objective of this application is to overcome the drawbacks of existing organic electroluminescent materials, such as low stability and low molecular quantum yield, leading to low luminance efficiency and short lifetime in organic electroluminescent devices comprising the organic electroluminescent materials. To this end, the present application provides a deuterated compound, as well as a light-emitting host material composition and a light-emitting device comprising the same, and uses thereof.

The definitions of terms related to substituents in the present application are as follows:
As used herein, the term "halogen" can include fluorine, chlorine, bromine, or iodine.

As used herein, the term "C1-C30 alkyl" refers to a monovalent substituent derived from a straight or branched saturated hydrocarbon having 1 to 30 carbon atoms. Examples include, but not limited to, methyl, ethyl, propyl, isobutyl, sec-butyl, tert-butyl, pentyl, isopentyl, and hexyl.

As used herein, the term "C3-C30 cycloalkyl" refers to a monocyclic or polycyclic hydrocarbon group derived from a ring containing 3 to 30 carbon atoms on the main chain thereof. The cycloalkyl group may include cyclopropyl, cyclobutyl, adamantyl, and the like.

Aryl and arylene in this application encompass monocyclic, polycyclic, or fused-ring aromatic groups, wherein the rings may be interrupted by short non-aromatic units and may contain spiro structures. Aryl groups include, but not limited to, phenyl, biphenyl, terphenyl, naphthyl, phenanthryl, anthryl, fluorenyl, spiro-bifluorenyl and the like. Arylene groups include, but not limited to, phenylene, biphenylene, terphenylene, naphthylene, phenanthrylene, anthrylene, fluorenylene, spiro-bifluorenylene and the like.

Heteroaryl and heteroarylene in this application encompass monocyclic, polycyclic, or fused-ring heteroaromatic groups, wherein the rings may be interrupted by short non-aromatic units, and the heteroatoms include nitrogen, oxygen, and sulfur. Heteroaryl groups include, but not limited to, furanyl, phenylsulfanyl, pyrrolyl, imidazolyl, pyrazolyl, thiazolyl, thiadiazolyl, isothiazolyl, isoxazolyl, oxazolyl, oxadiazolyl, triazinyl, tetrazinyl, triazolyl, tetrazolyl, furazanyl, pyridinyl, pyrazinyl, pyrimidinyl, pyridazinyl, benzofuranyl, benzothienyl, isobenzofuranyl, dibenzofuranyl, dibenzothienyl, benzimidazolyl, benzothiazolyl, benzoisothiazolyl, benzoisoxazolyl, benzoxazolyl, isoindolyl, indolyl, indazolyl, benzothiadiazolyl, quinolinyl, isoquinolinyl, cinnolinyl, quinazolinyl, quinoxalinyl, carbazolyl, phenoxazinyl, phenothiazinyl, phenanthridinyl, benzodioxolyl, dihydroacridinyl, and derivatives thereof. Heteroarylene groups include, but not limited to, furylene, phenylsulfanylene, pyrrolylene, imidazolylene, pyrazolylene, thiazolylene, thiadiazolylene, isothiazolylene, isoxazolylene, oxazolylene, oxadiazolylene, triazinylene, tetrazinylene, triazolylene, tetrazolylene, furazanylene, pyridinylene, pyrazinylene, pyrimidinylene, pyridazinylene, benzofuranylene, benzothienylene, isobenzofuranylene, dibenzofuranylene, dibenzothienylene, benzimidazolylene, benzothiazolylene, benzoisothiazolylene, benzoisoxazolylene, benzoxazolylene, isoindolylene, indolylene, indazolylene, benzothiadiazolylene, quinolinylene, isoquinolinylene, cinnolinylene, quinazolinylene, quinoxalinylene, carbazolylene, phenoxazinylene, phenothiazinylene, phenanthridinylene, benzodioxolylene, dihydroacridinylene, and derivatives thereof.

As used herein, the term "substituted" means that one or more hydrogen atoms of a compound are replaced by one or more substituents. Such substitution may occur at any available position where a hydrogen atom is replaceable, and is not limited to a specific site. When two or more substituents are present, they may be the same or different.

As used herein, unless otherwise specified, the hydrogen atom includes protium, deuterium, and tritium.

In this application, when a group is defined with a range for the number of carbon atoms, the number of carbon atoms may be any integer within the specified range; for example, a C6-C30 aryl group means that the number of carbon atoms thereof may be any integer in a range of 6 to 60, e.g., 6, 8, 10, 13, 15, 17, 20, 22, 25, or 30.

As used herein, "organic electroluminescent material" refers to a material that can be used in an organic electroluminescent device and may comprise at least one compound. Such materials may be incorporated into any layer constituting the organic electroluminescent device as needed. For example, the organic electroluminescent material may be a hole injection material, hole transport material, hole assisting material, emission assisting material, electron blocking material, light-emitting material (comprising a host material and a dopant material), electron buffer material, hole blocking material, electron transport material, electron injection material and the like.

As used herein, "multi-host material" means that an organic electroluminescent material comprises a combination of at least two host materials. It may refer to both the material before incorporation into the organic electroluminescent device (e.g., prior to vapor deposition) and the material after incorporation (e.g., after vapor deposition). The multi-host material of this application may be included in any light-emitting layer constituting the organic electroluminescent device. Two or more compounds contained in the multi-host material disclosed herein may be included in a single light-emitting layer, or may be separately included in different light-emitting layers. For example, when two or more host materials are included in one layer, the layer may be formed by mixing and co-evaporation, or simultaneously by individual co-evaporation. In the present application, 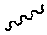 represents a linking bond.

The solution adopted in the present application is as follows:

The present application provides a deuterated compound having the structure shown in Formula (1) below: 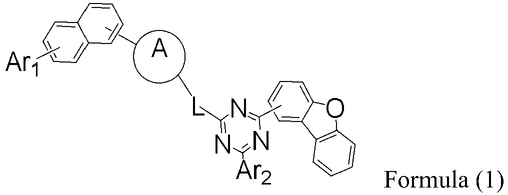
in Formula (1), Ar₁ is selected from hydrogen, deuterium, halogen, cyano, a substituted or unsubstituted C6-C30 aryl, and a substituted or unsubstituted C3-C30 heteroaryl;
Ar₂ is selected from a substituted or unsubstituted C6-C30 aryl, and a substituted or unsubstituted C3-C30 heteroaryl;
Ring A is naphthylene, wherein at least one hydrogen on Ring A or Ar₂ is substituted with deuterium;
L is selected from a linking bond, a substituted or unsubstituted C6-C30 arylene, and a substituted or unsubstituted C3-C30 heteroarylene; and
the substituents in the substituted C6-C30 arylene, substituted C3-C30 heteroarylene, substituted C6-C30 aryl, and substituted C3-C30 heteroaryl are selected from one or two of a combination of deuterium, halogen, cyano, C1-C6 alkyl, C3-C30 cycloalkyl, C6-C30 aryl, and C3-C30 heteroaryl.

Optionally, Ar₁ is selected from hydrogen, deuterium, a substituted or unsubstituted C6-C20 aryl, and a substituted or unsubstituted C3-C20 heteroaryl;
Ar₂ is selected from a substituted or unsubstituted C6-C20 aryl, and a substituted or unsubstituted C3-C20 heteroaryl;
the substituents on the substituted C6-C20 aryl, and substituted C3-C20 heteroaryl are selected from one or two of a combination of deuterium, halogen, cyano, C1-C6 alkyl, C3-C30 cycloalkyl, C6-C30 aryl, and C3-C30 heteroaryl; and
optionally, at least one hydrogen on Ring A is substituted with deuterium, and at most 5 hydrogens are substituted with deuterium.

Optionally, the Formula (1) is selected from one of structures below: 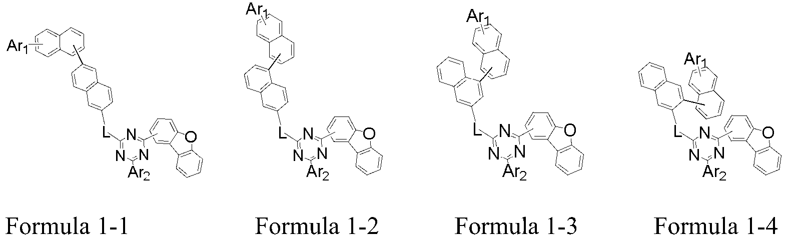 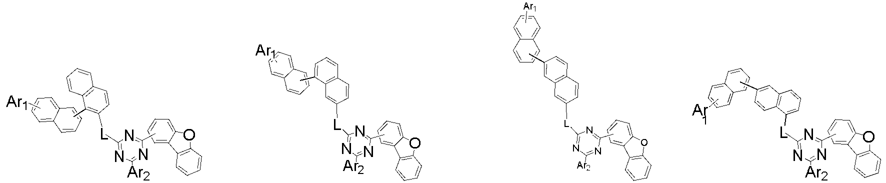 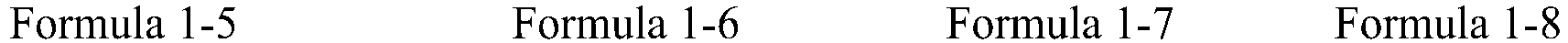 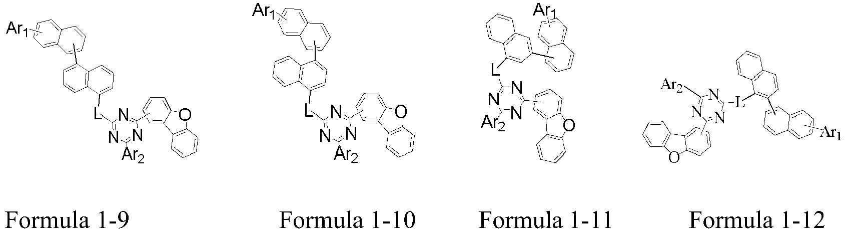 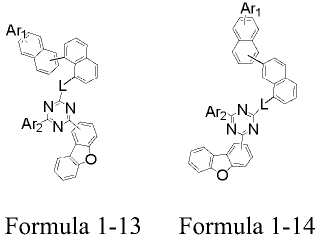 wherein Ar₁, Ar₂, and L are defined as described above.

Optionally, Ar₁ is selected from hydrogen, deuterium, and a substituted or unsubstituted B group,
Ar₂ is selected from a substituted or unsubstituted C group,
wherein the B group is selected from phenyl, naphthyl, biphenyl, terphenyl, carbazolyl, and dimethylfluorenyl;
the C group is selected from phenyl, naphthyl, biphenyl, terphenyl, phenanthrenyl, fluoranthenyl, triphenylene, dimethylfluorenyl, diphenylfluorenyl, spiro-bifluorenyl, benzodimethylfluorenyl, benzodiphenylfluorenyl, benzospirobifluorenyl, benzofuranyl, dibenzofuranyl, benzothienyl, dibenzothienyl, carbazolyl, benzocarbazolyl, and dibenzocarbazolyl; and
the substituents on the substituted B group and substituted C group are selected from one or two of a combination of deuterium, halogen, cyano, C1-C6 alkyl, C3-C30 cycloalkyl, C6-C30 aryl, and C3-C30 heteroaryl.

Optionally, Ar₂ is selected from a substituted or unsubstituted C group;
wherein the C group is selected from phenyl, naphthyl, biphenyl, terphenyl, phenanthrenyl, fluoranthenyl, triphenylene, dimethylfluorenyl, diphenylfluorenyl, spiro-bifluorenyl, benzodimethylfluorenyl, benzodiphenylfluorenyl, benzospirobifluorenyl, benzofuranyl, dibenzofuranyl, benzothienyl, dibenzothienyl, carbazolyl, benzocarbazolyl, and dibenzocarbazolyl; and
wherein the substituent on the substituted C group is selected from deuterium;
optionally, Ar₂ is selected from phenyl, phenyl substituted with at least one deuterium and naphthyl substituted with at least one deuterium;
optionally, Ar₂ is selected from phenyl, phenyl substituted with at least one deuterium and naphthyl substituted with at least one deuterium, wherein the number of deuteriums on the phenyl substituted with at least one deuterium is 1 to 2, and the number of deuteriums on the naphthyl substituted with at least one deuterium is 1 to 6;
optionally, Ar₁ is selected from hydrogen, deuterium, phenyl, naphthyl, and phenanthrenyl; and
optionally, Ar₁ is selected from hydrogen, deuterium, and phenyl.
Optionally, L is selected from a linking bond and C6-C12 arylene;
Optionally, L is selected from a linking bond, phenylene, and naphthylene; and
optionally, Ar₂ is selected from phenyl substituted with 1 or 2 deuteriums, and naphthyl substituted with 1 to 6 deuteriums.

Optionally, the deuterated compound is selected from one of M-1 to M-100:

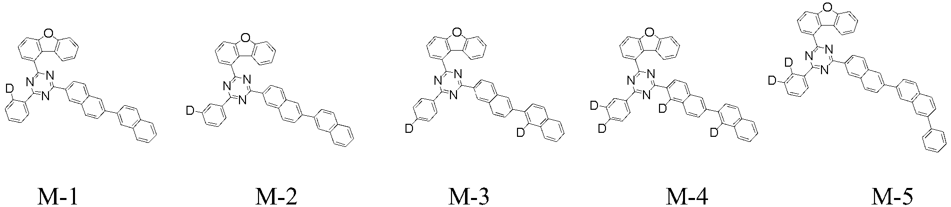

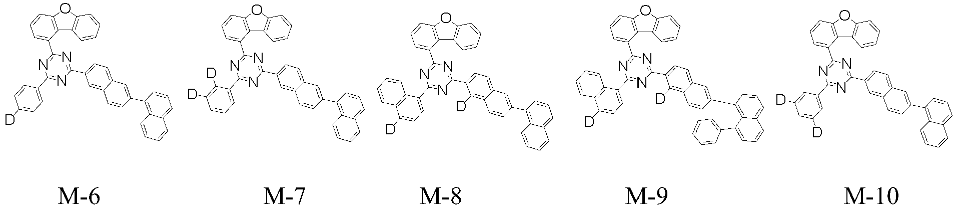

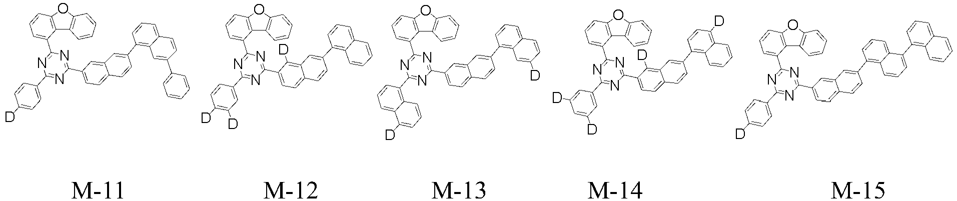

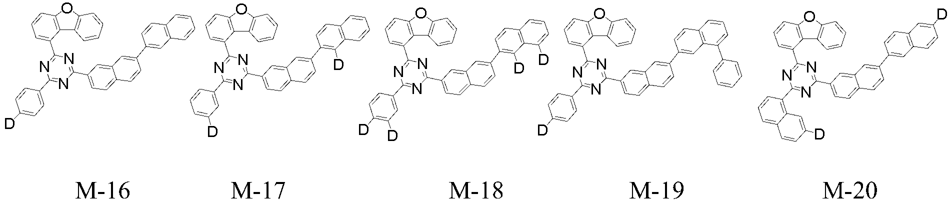

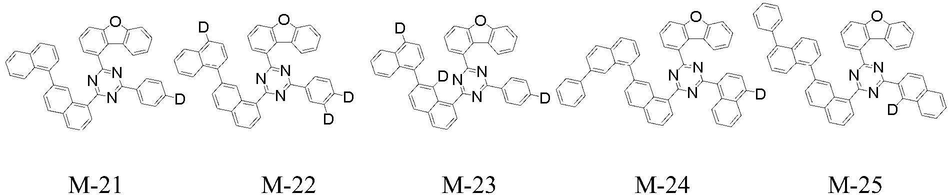

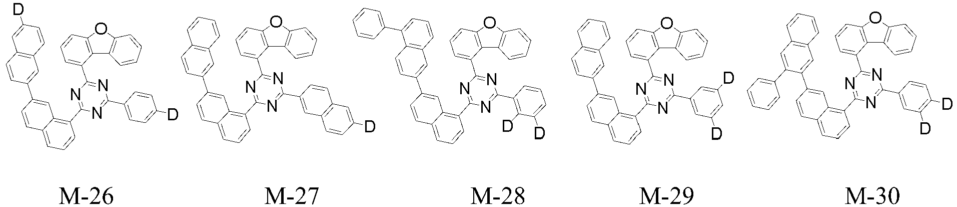

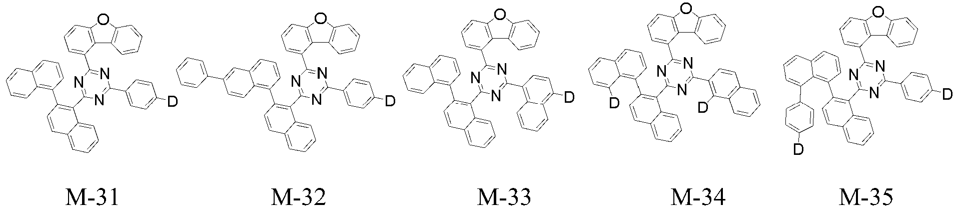

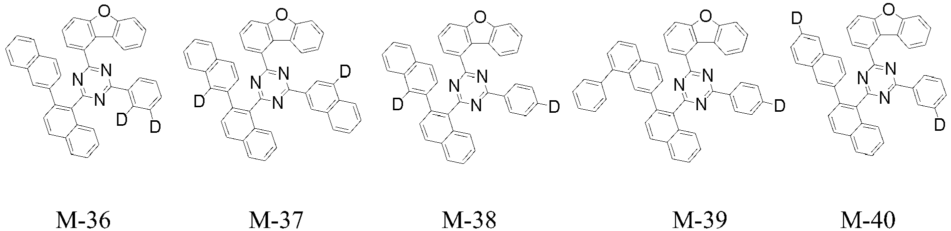

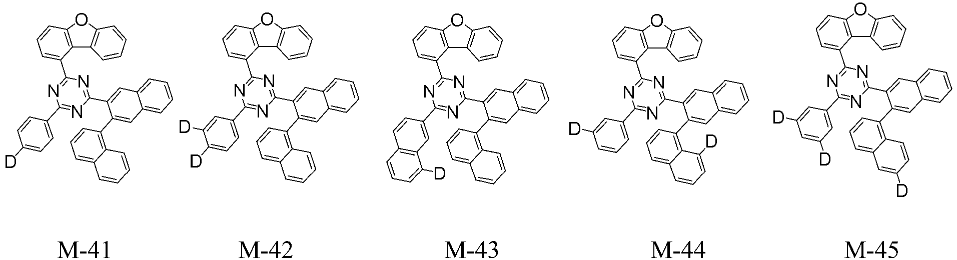

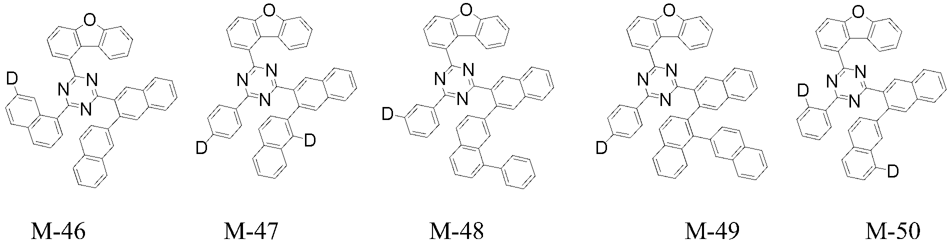

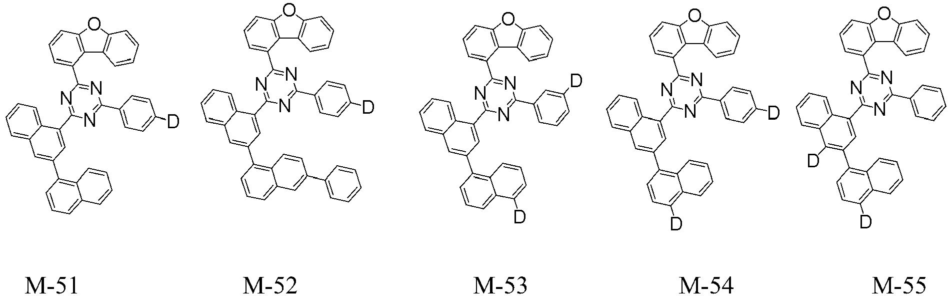

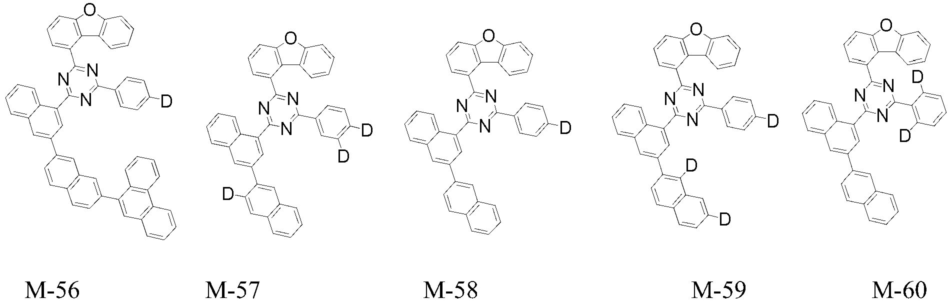

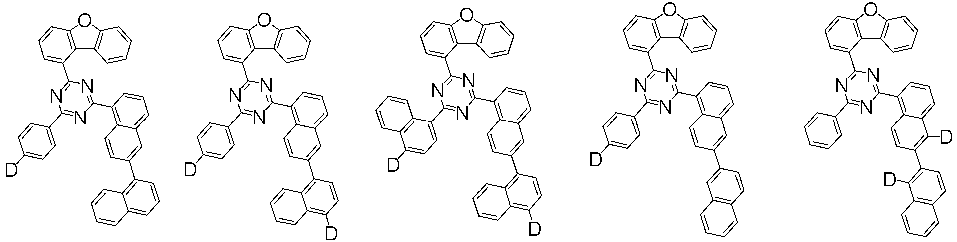

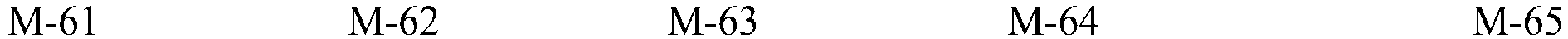

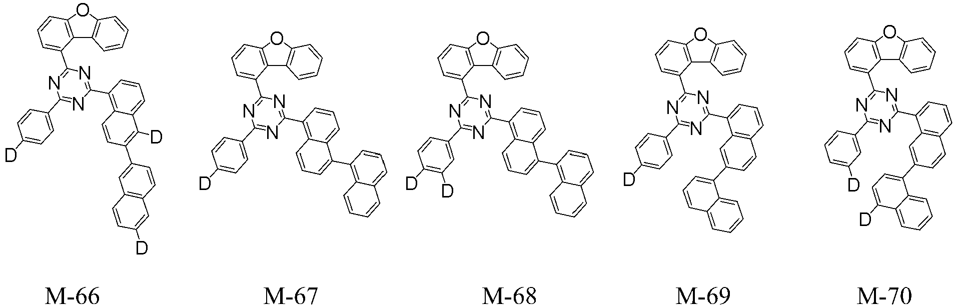

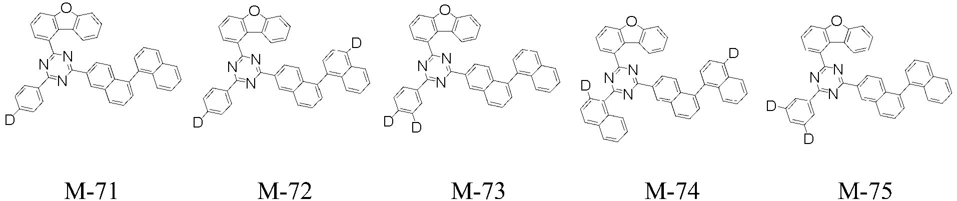

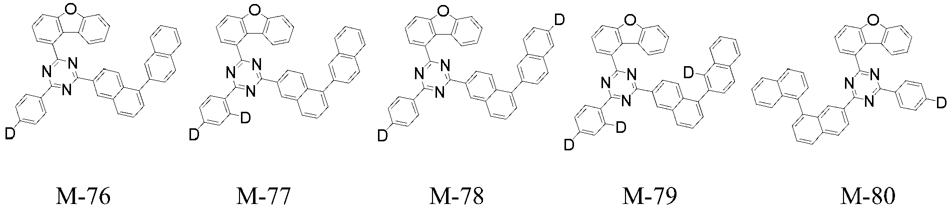

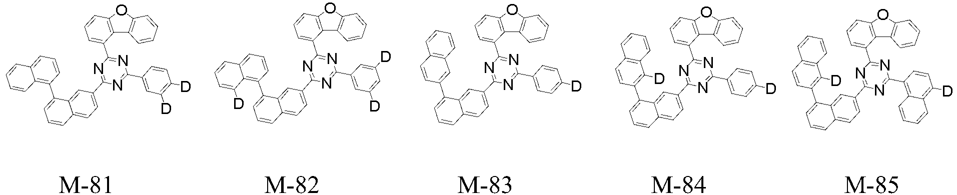

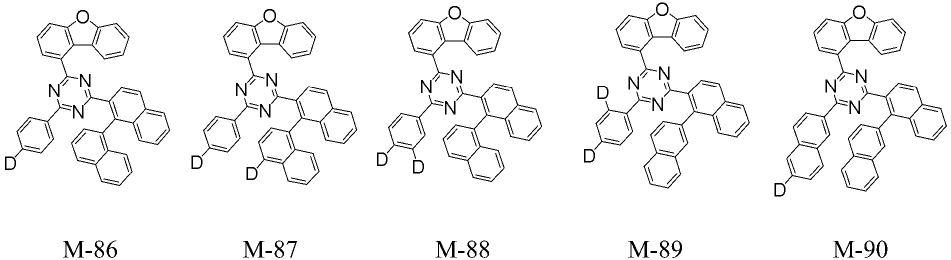

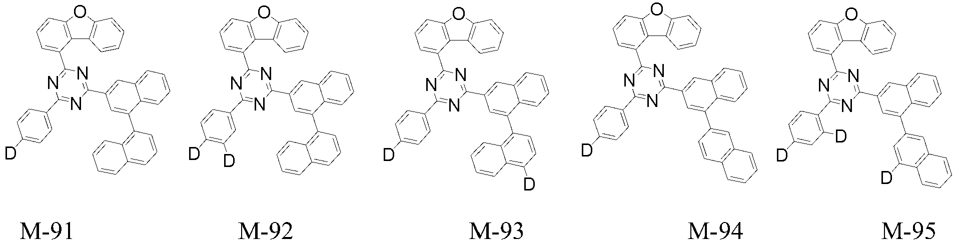

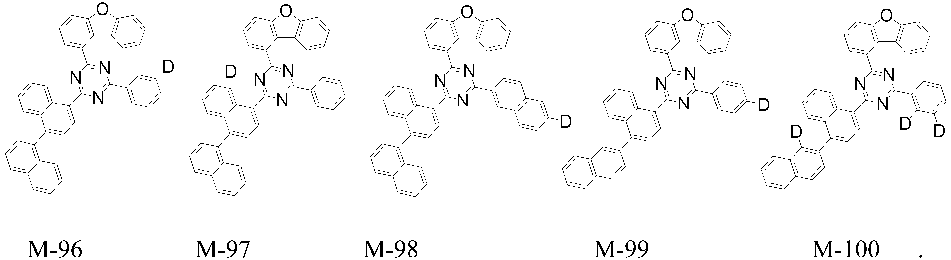

The present application also provides a light-emitting host material composition, comprising a first host material and a second host material, the first host material comprises the deuterated compound of any one of claims 1 to 7, and the second host material comprises a compound shown in Formula (2) below: 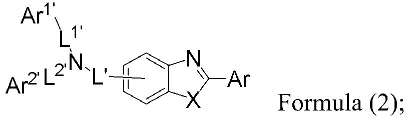
wherein X is selected from O and S;
L', L^{1'}, and L^{2'} are each independently selected from a linking bond, a substituted or unsubstituted C6-C30 arylene, and a substituted or unsubstituted C3-C30 heteroarylene, and L', L^{1'}, and L^{2'} are each independently present; and
wherein Ar^{1'}, Ar^{2'}, and Ar are each independently selected from hydrogen, deuterium, halogen, cyano, a substituted or unsubstituted C6-C60 aryl, a substituted or unsubstituted C6-C60 arylamino, a substituted or unsubstituted C3-C60 heteroarylamino, and a substituted or unsubstituted C3-C60 heteroaryl; and
the substituents on the substituted C6-C30 arylene, C3-C30 heteroarylene, substituted C6-C30 aryl, substituted C3-C30 heteroaryl, substituted C6-C60 arylamino, and substituted C3-C60 heteroarylamino are each independently selected from one or at least two of a combination of deuterium, halogen, cyano, C1-C12 alkyl, C3-C12 cycloalkyl, C6-C30 aryl, C3-C30 heteroaryl, C6-C60 arylamino, and C3-C60 heteroarylamino.

Optionally, Ar^{1'}, Ar^{2'}, and Ar are each independently selected from hydrogen, deuterium, halogen, and a substituted or unsubstituted E group, the E group is selected from: phenyl, naphthyl, biphenyl, phenanthrenyl, fluoranthenyl, chrysenyl, terphenyl, triphenylene, phenylnaphthyl, naphthylphenyl, dimethylfluorenyl, phenylmethylfluorenyl, diphenylfluorenyl, pyridinyl, pyridinylphenyl, phenylpyridinyl, spiro-bifluorenyl, benzodimethylfluorenyl, benzodiphenylfluorenyl, benzospirobifluorenyl, dibenzofuranyl, benzonaphthofuranyl, benzonaphthothienyl and dibenzothienyl;
the substituents on the substituted E group are each independently selected from one or at least two of a combination of C1-C6 alkyl, C3-C12 cycloalkyl, C6-C25 aryl, C3-C25 heteroaryl, C6-C60 arylamino, and C3-C60 heteroarylamino;
optionally, L', L^{1'}, and L^{2'} are each independently selected from a linking bond, a substituted or unsubstituted C6-C12 arylene, and a substituted or unsubstituted C3-C12 heteroarylene;
the substituents on the substituted C6-C12 arylene, and substituted C3-C12 heteroarylene are each independently selected from one or at least two of a combination of C1-C6 alkyl, C3-C12 cycloalkyl, C6-C25 aryl, C3-C25 heteroaryl, C6-C60 arylamino, and C3-C60 heteroarylamino;
optionally, Ar^{1'}-Ar^{2'} are each independently selected from phenyl, naphthyl, biphenyl, chrysenyl, terphenyl, phenylnaphthyl, naphthylphenyl, dimethylfluorenyl, diphenylfluorenyl, pyridinyl, pyridinylphenyl, phenylpyridinyl, phenylcarbazolyl, phenylbenzocarbazolyl, phenylphenanthrocarbazolyl, spirobifluorenyl, spiro[fluorene-9,9'-xanthene]yl, phenylmethylfluorenyl, dibenzofuranyl, and benzonaphthofuranyl;
optionally, Ar is selected from phenyl or naphthyl;
optionally, L' is selected from naphthylene; and
optionally, L^{1'} and L^{2'} are each independently selected from a linking bond, phenylene, or naphthylene.

Optionally, the second host material comprises one of the compounds selected from N-1 to N-654: 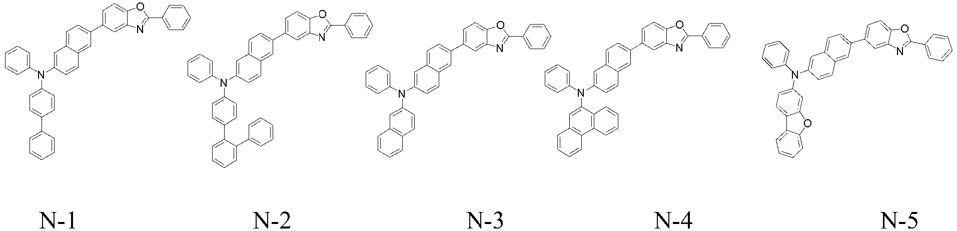 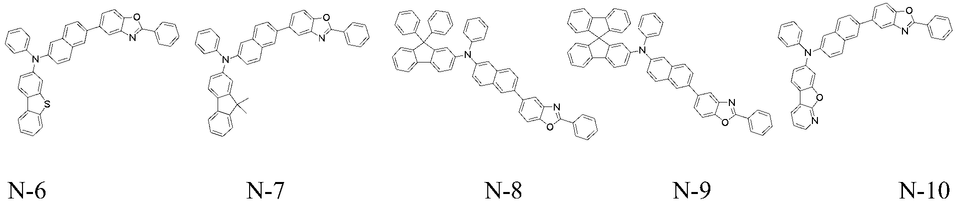 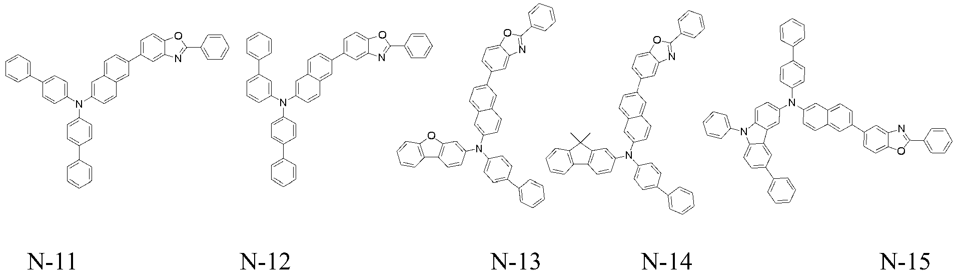 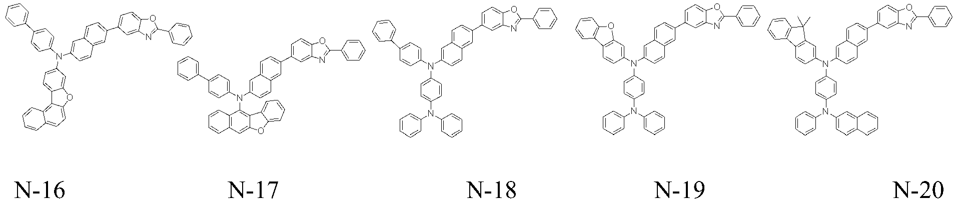 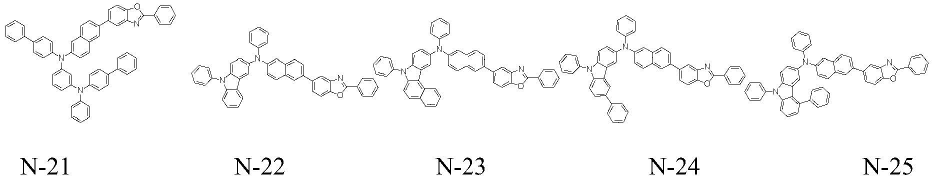 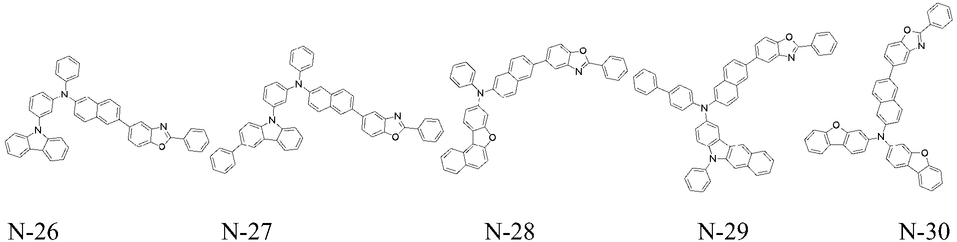 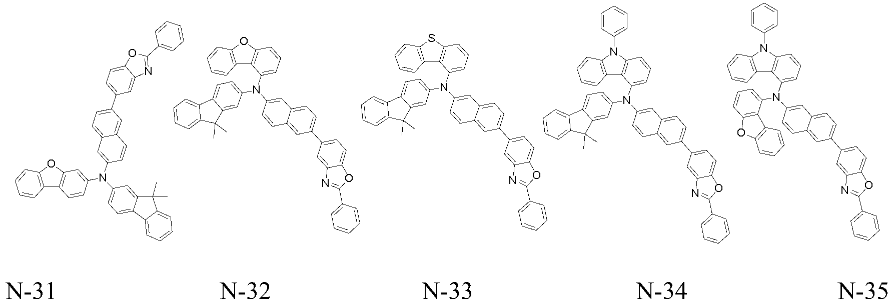 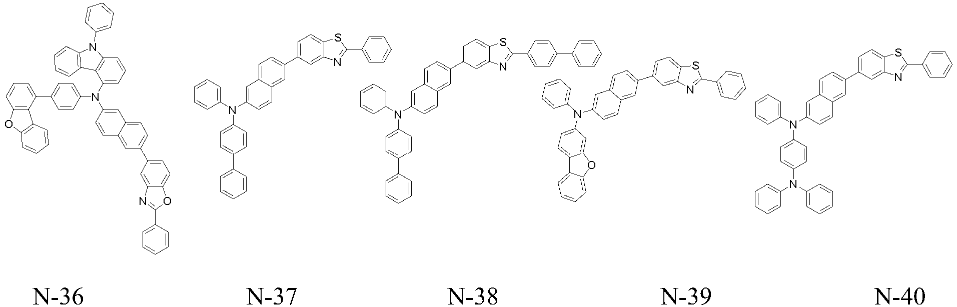 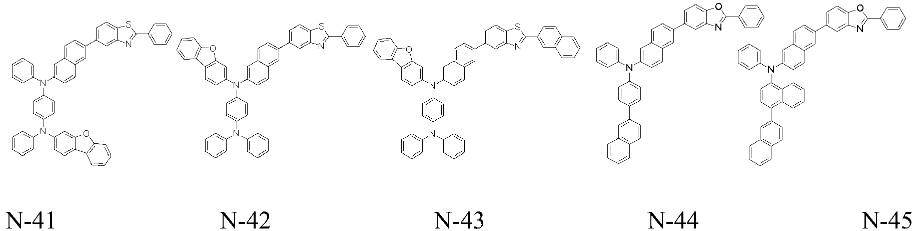 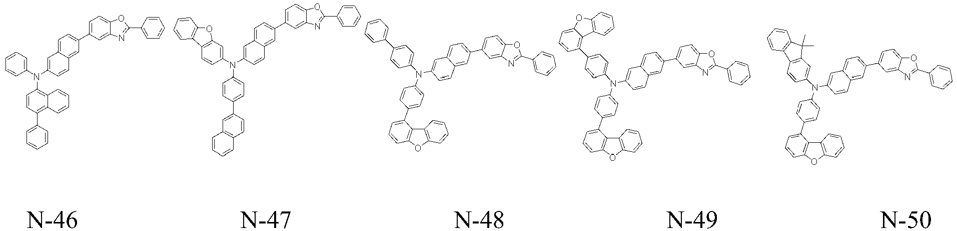 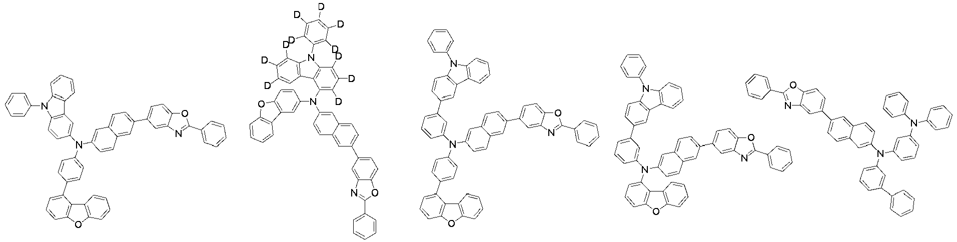 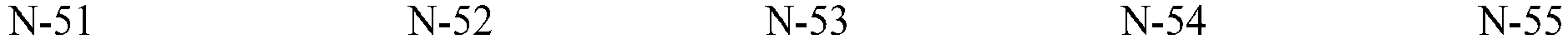 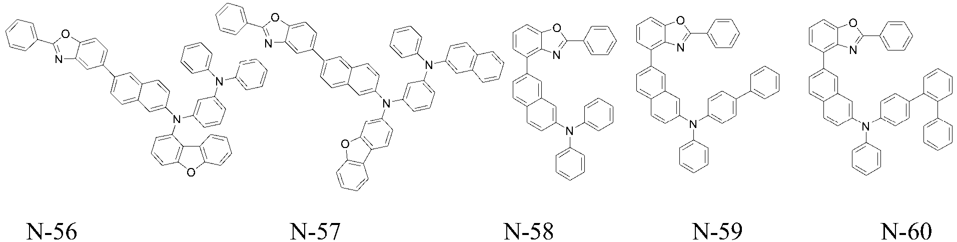 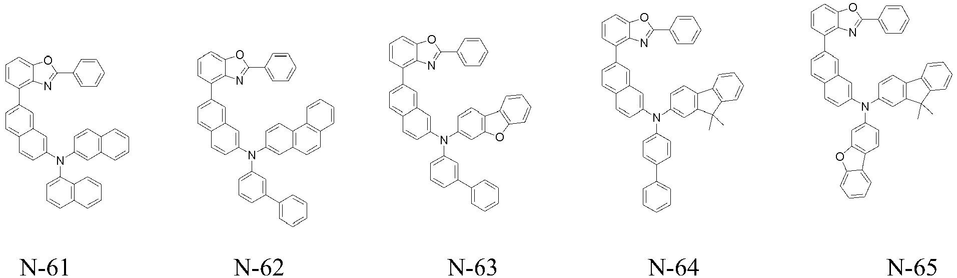 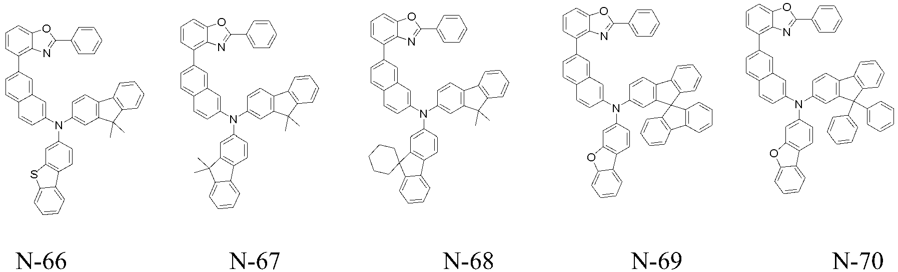 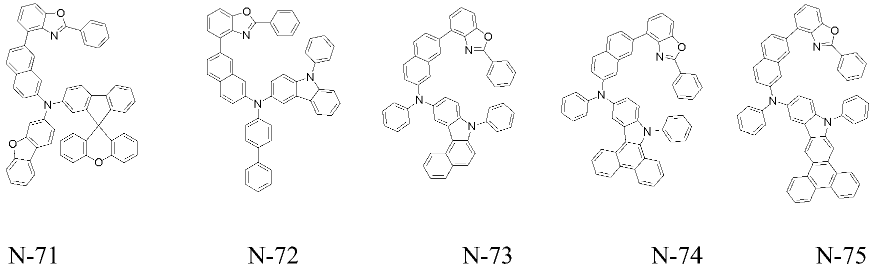 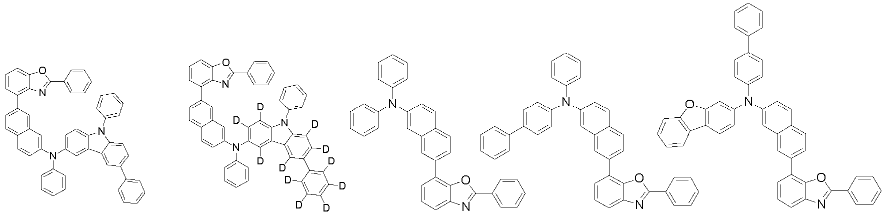 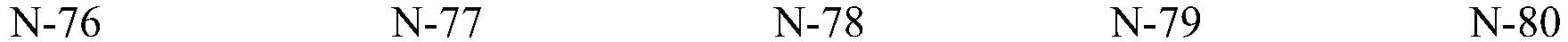 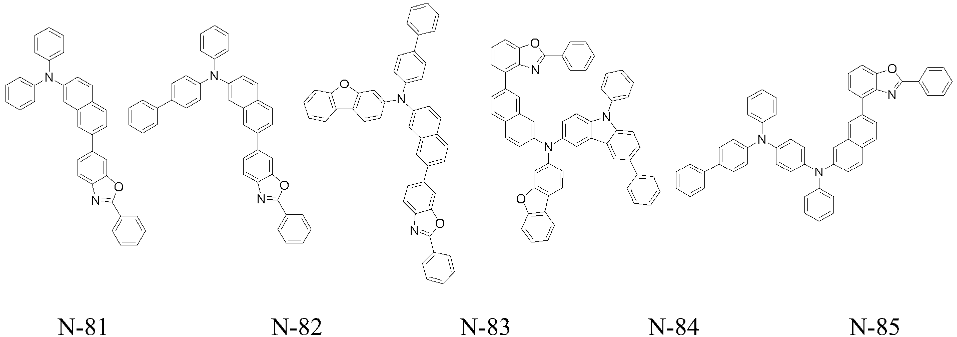 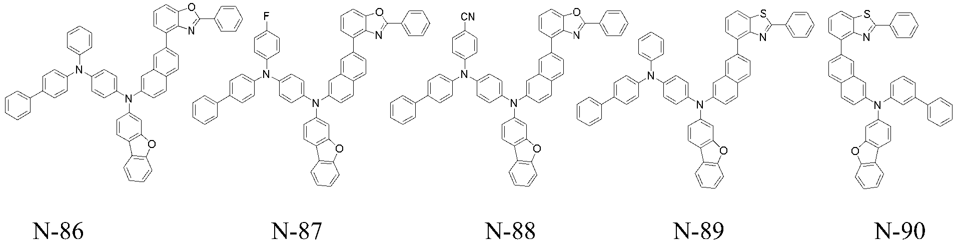 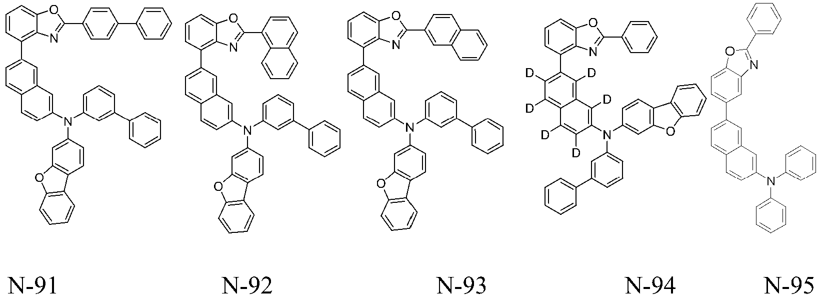 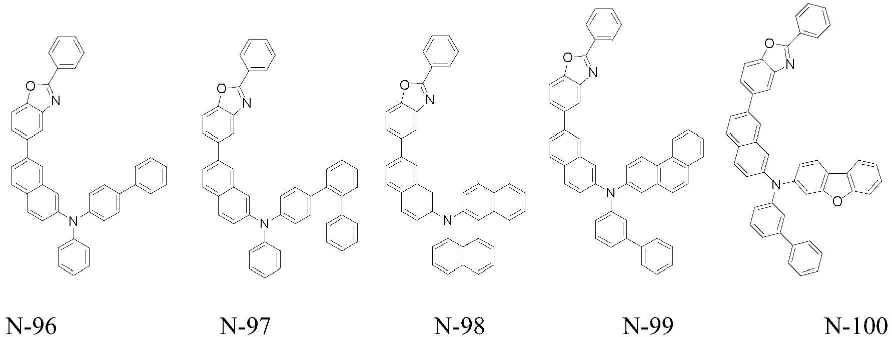 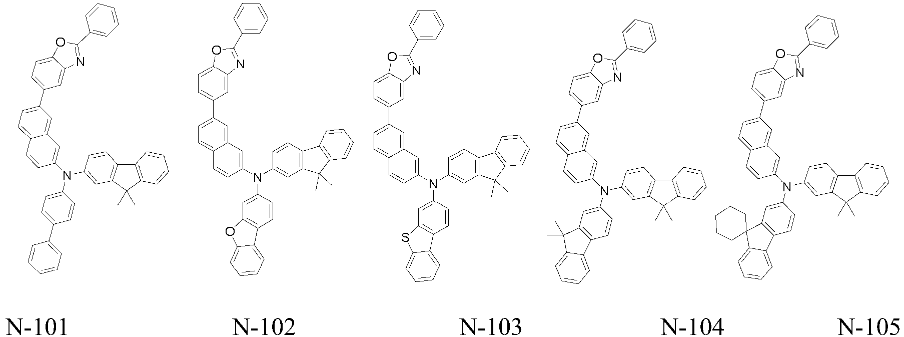 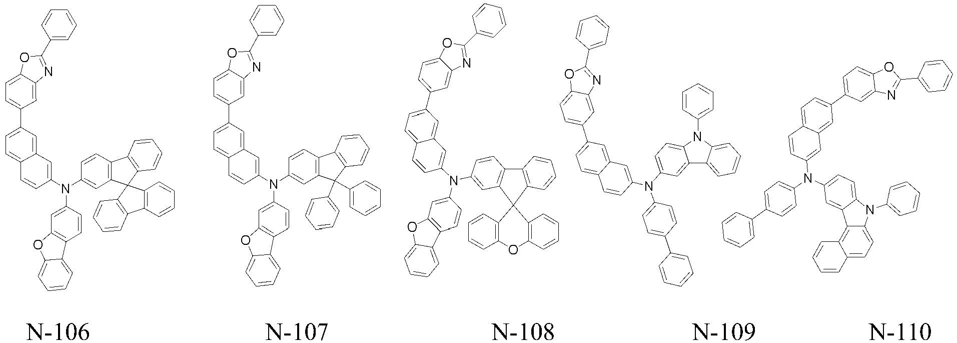 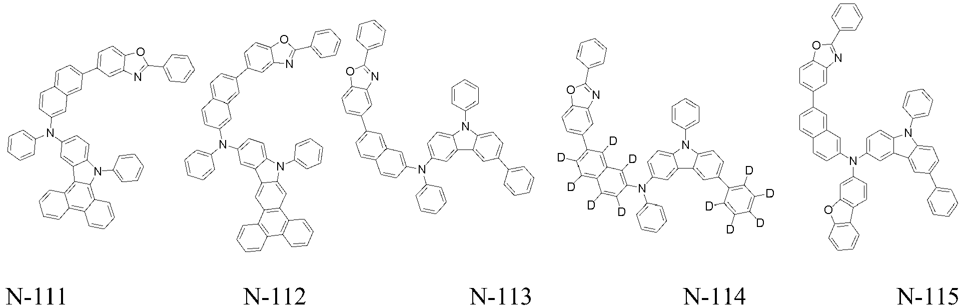 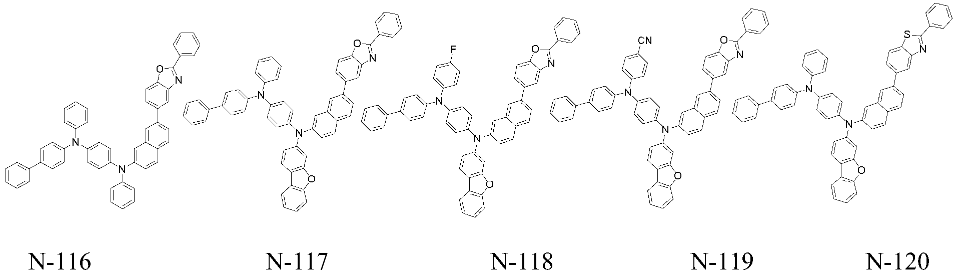 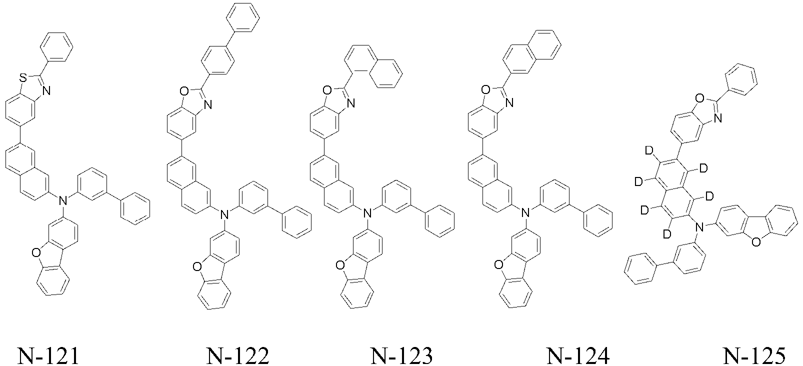 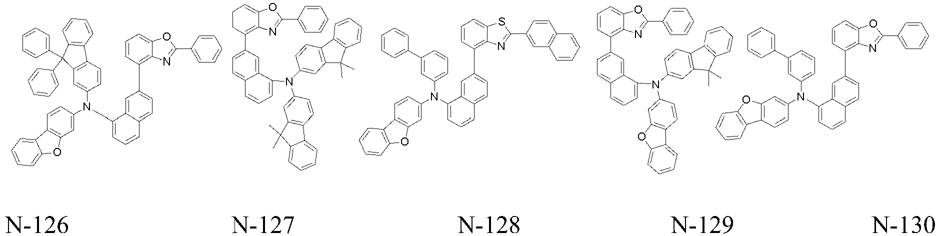 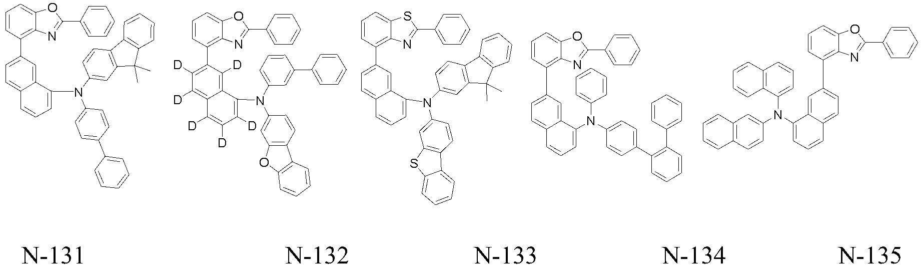 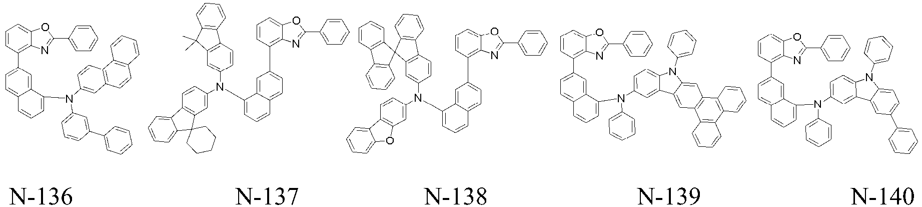 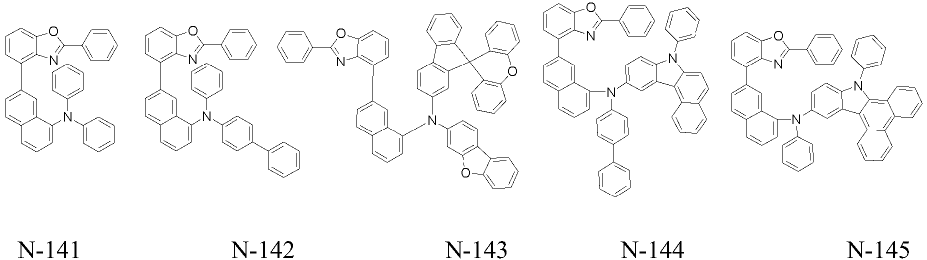 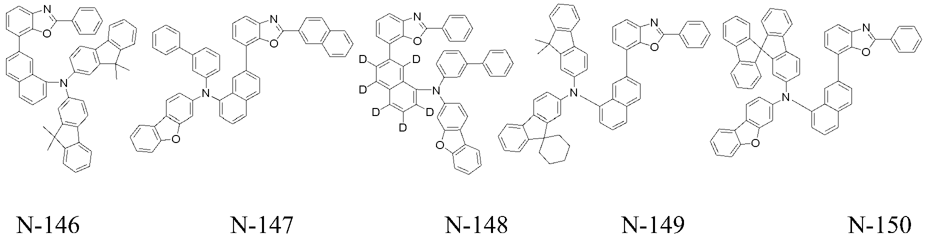 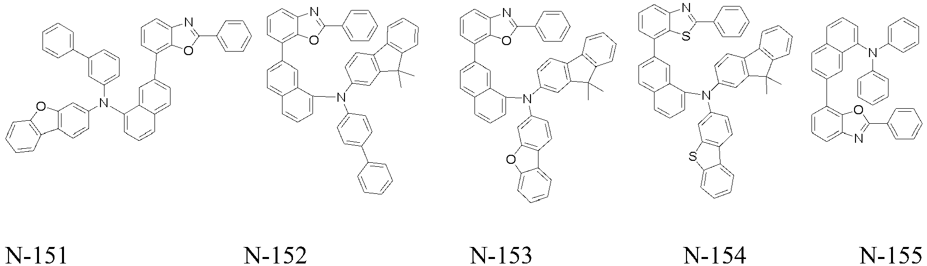 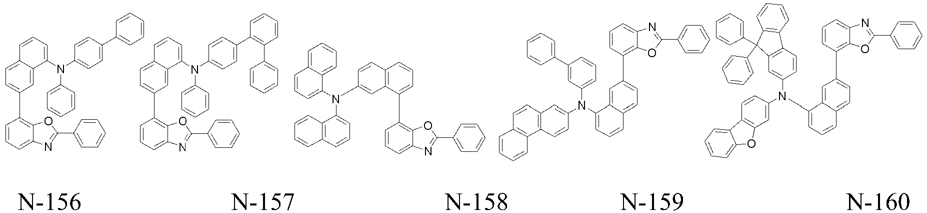 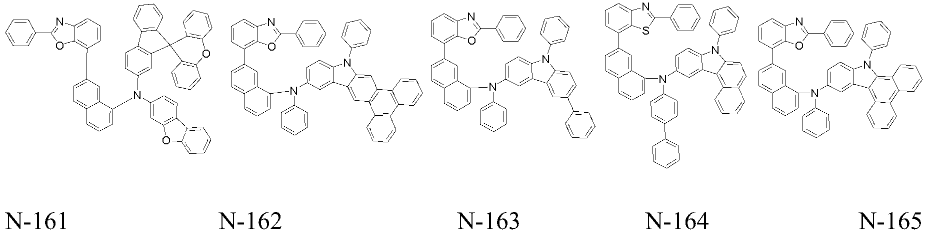 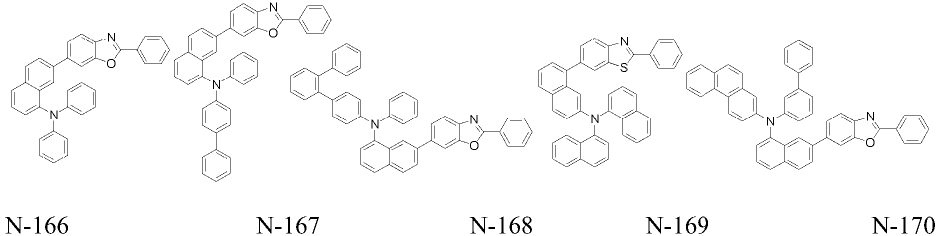 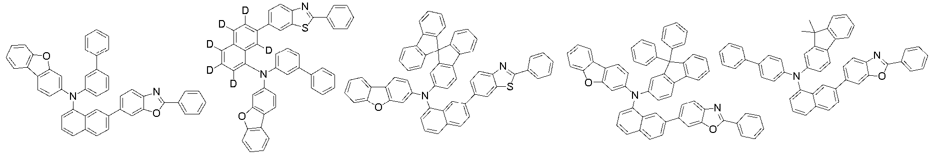 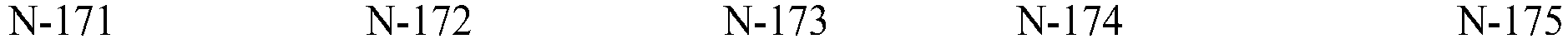 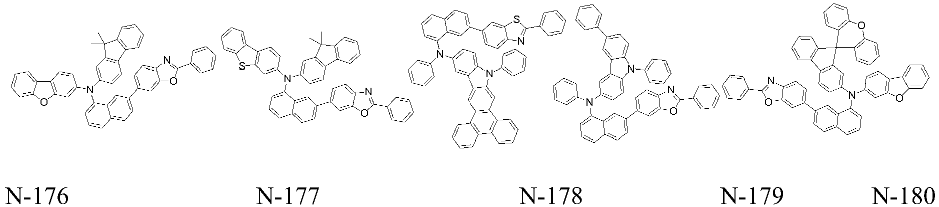 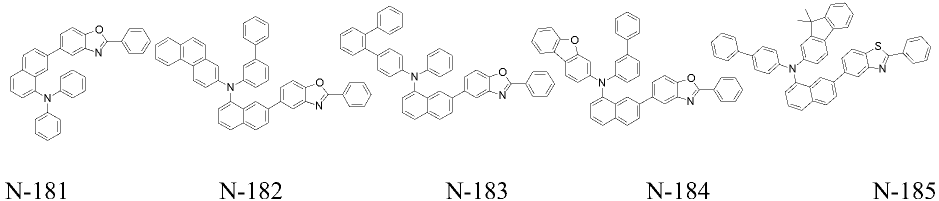 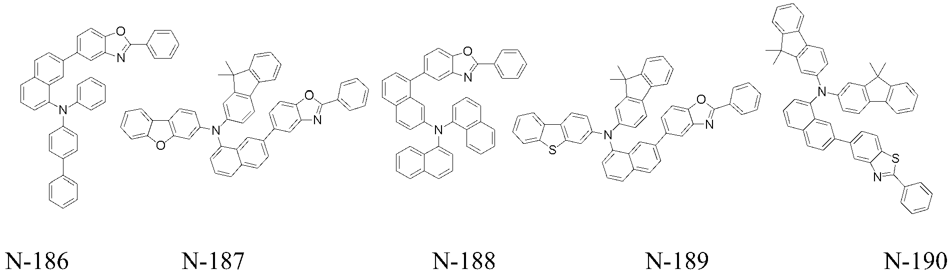 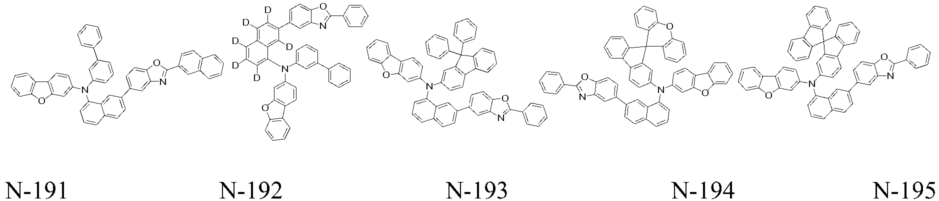 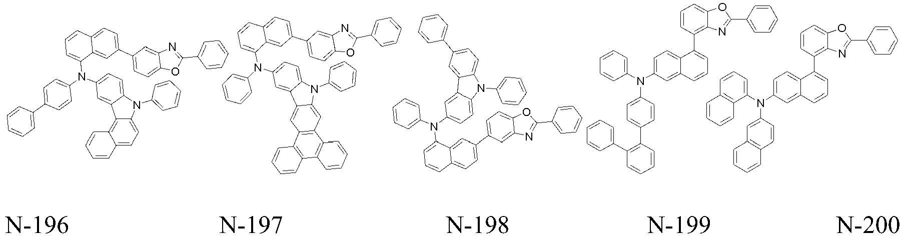 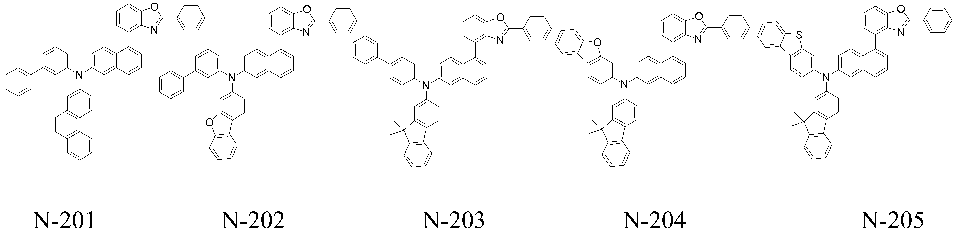 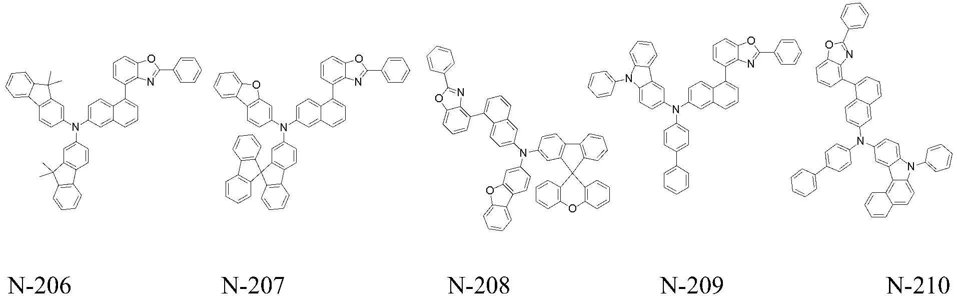 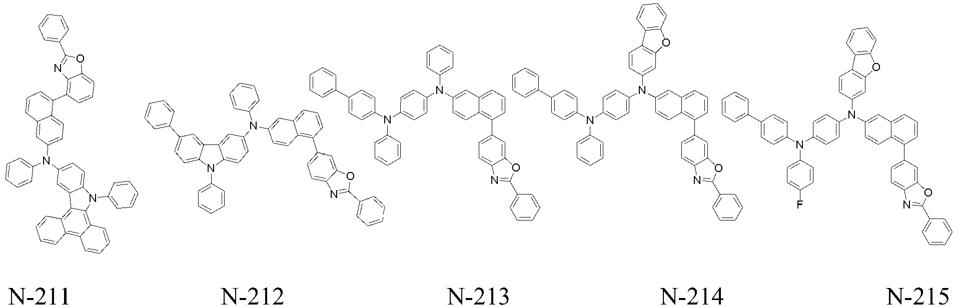 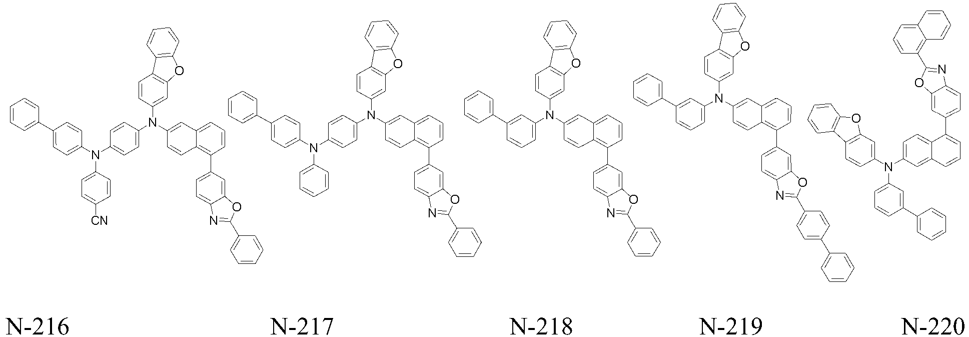 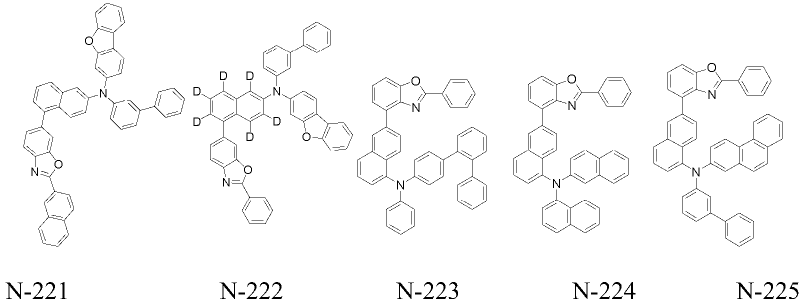 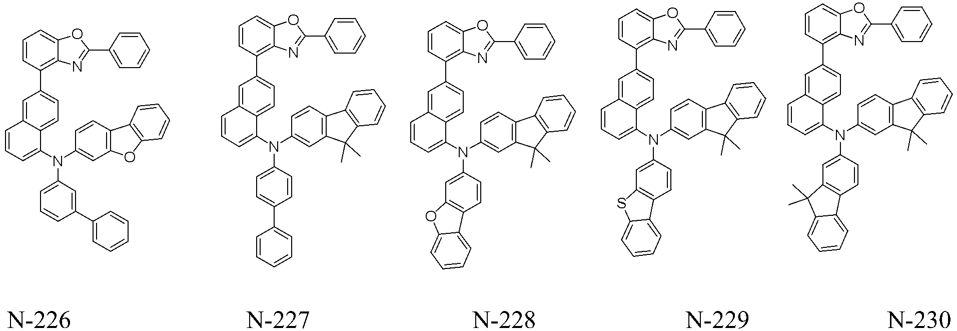 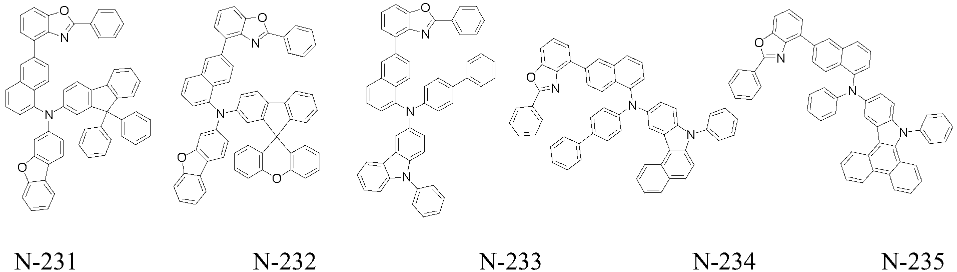 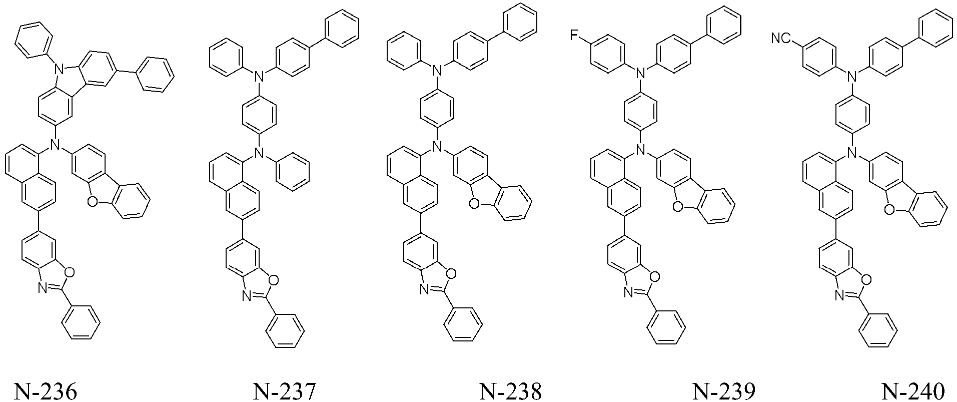 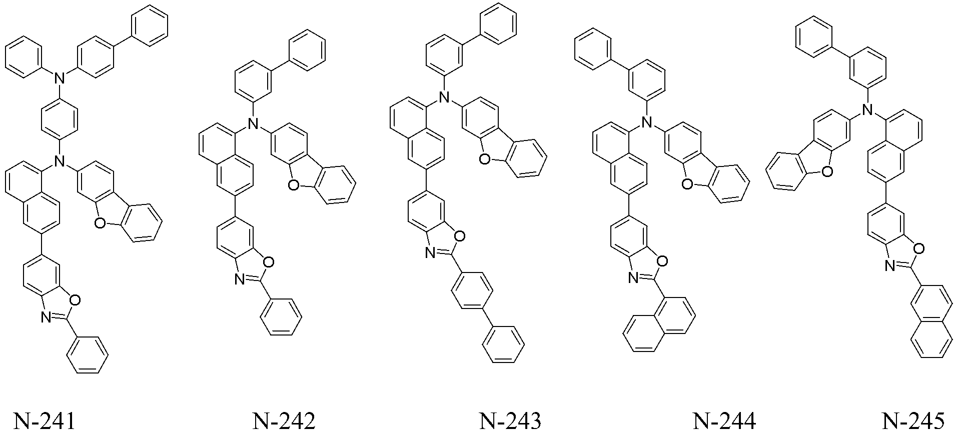 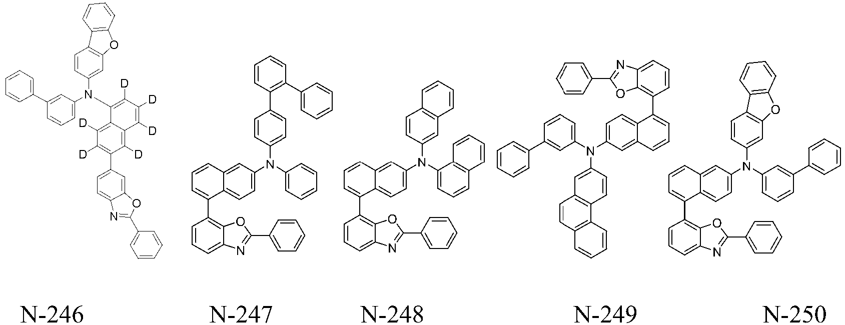 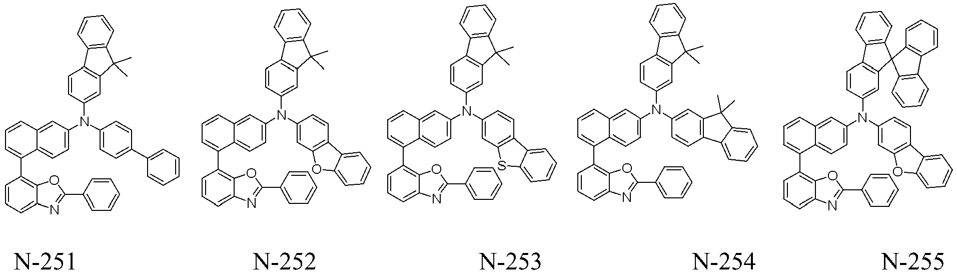 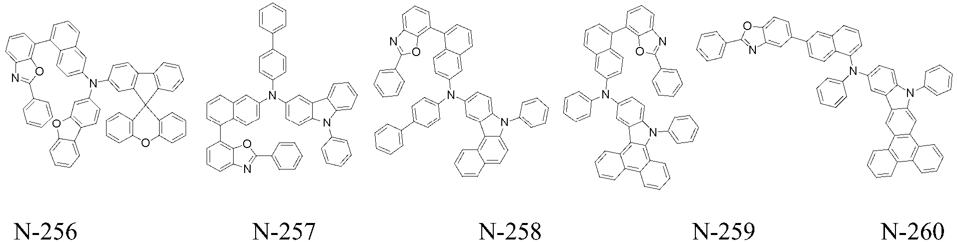 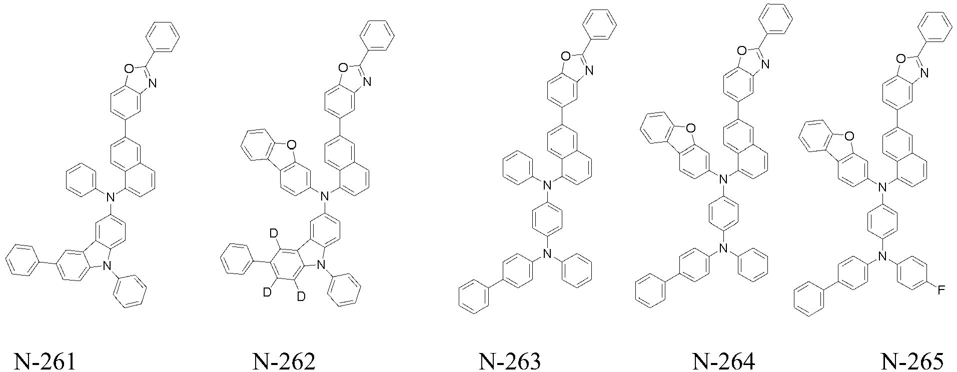 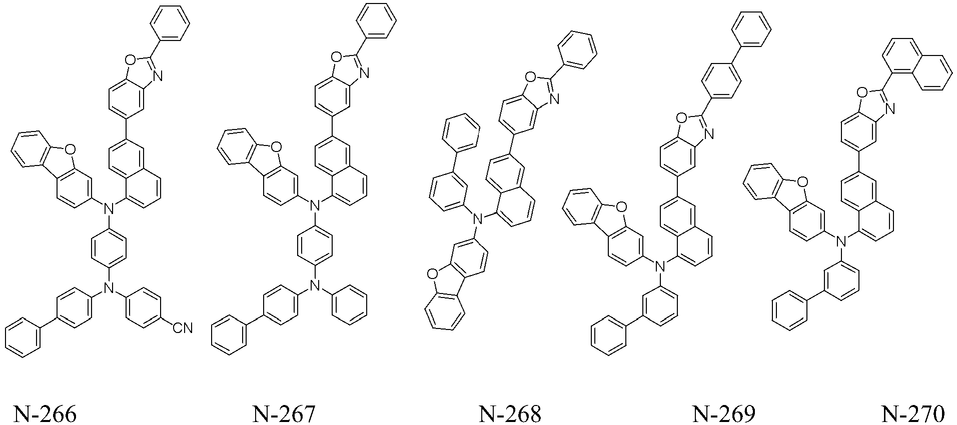 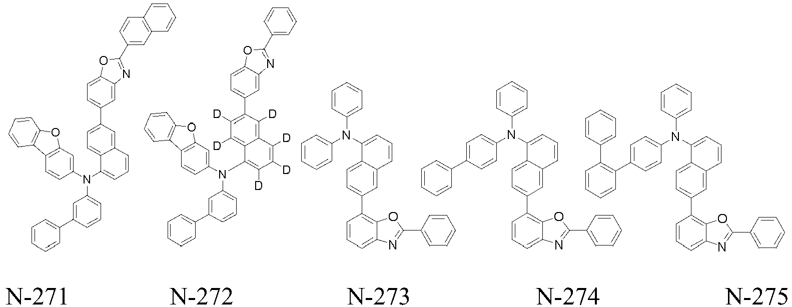 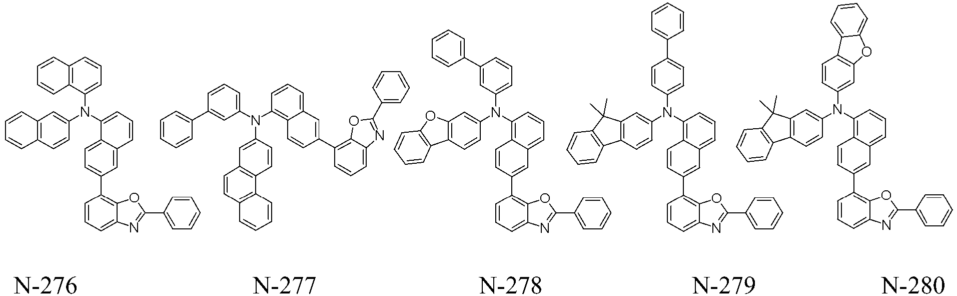 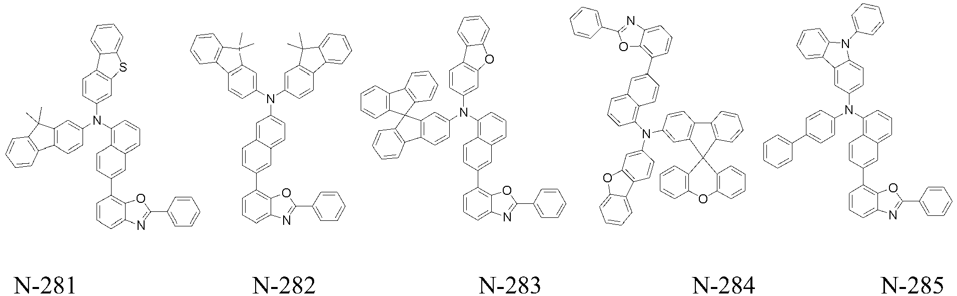 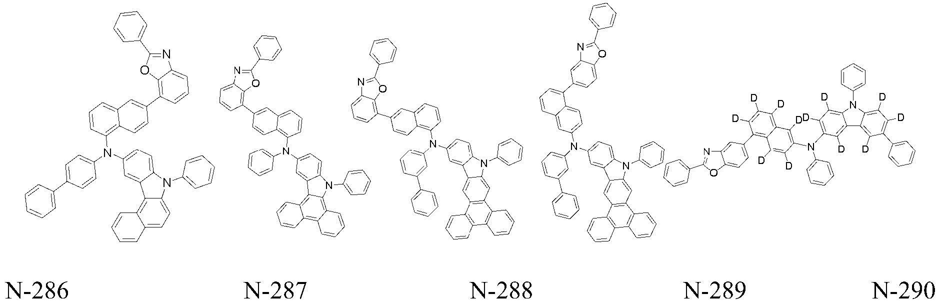 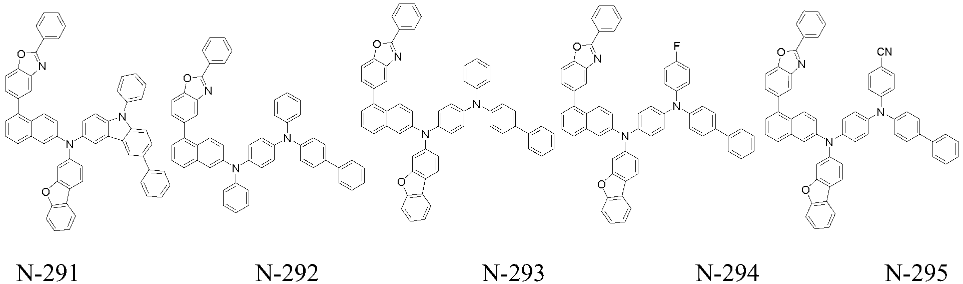 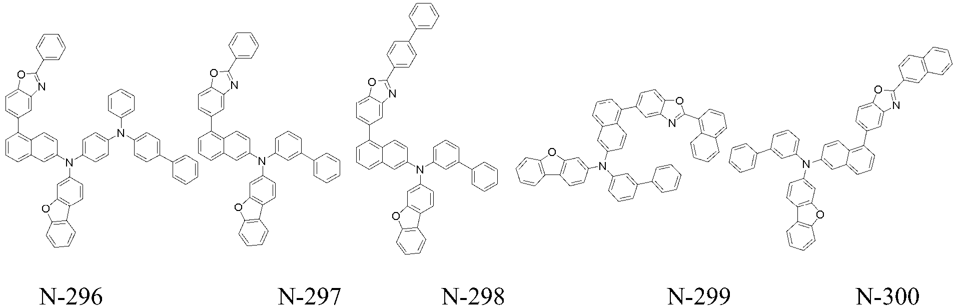 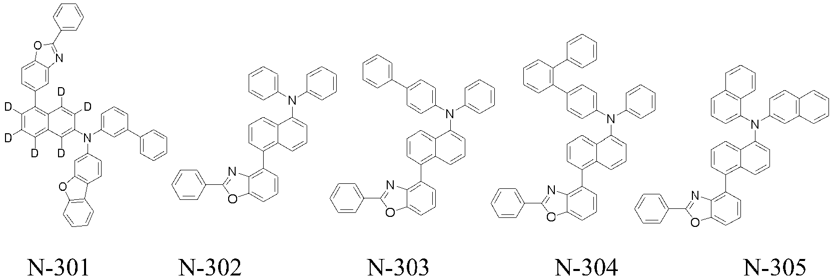 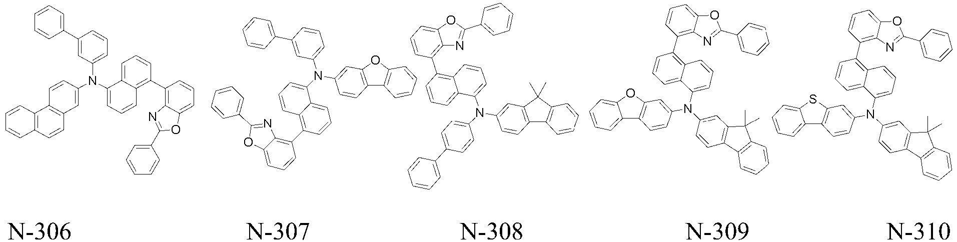 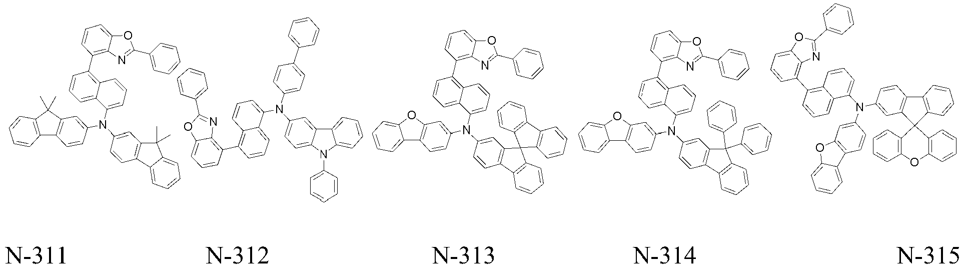 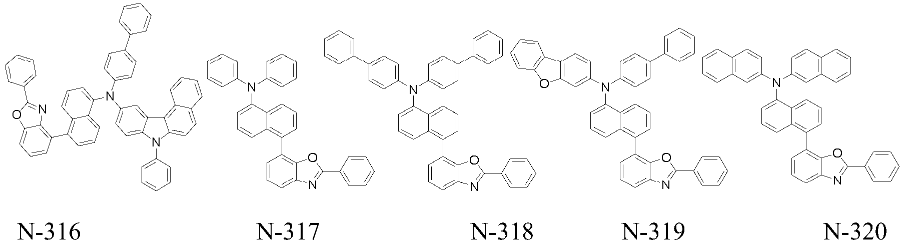 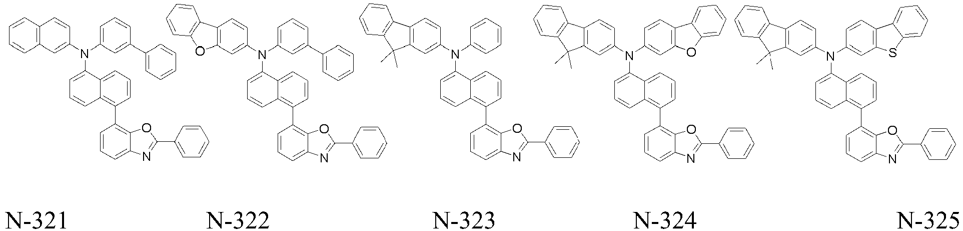 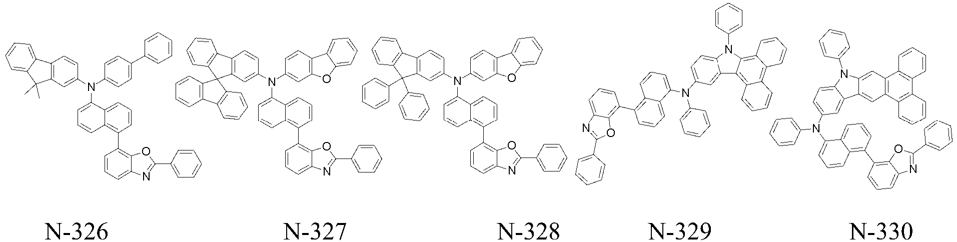 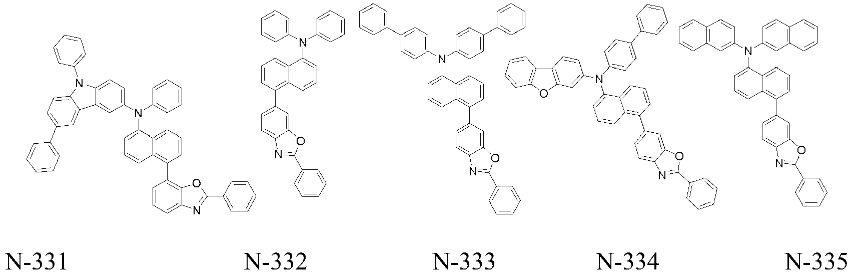 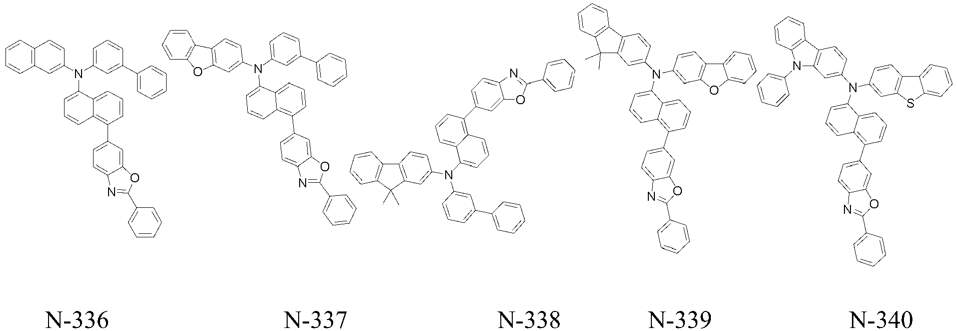 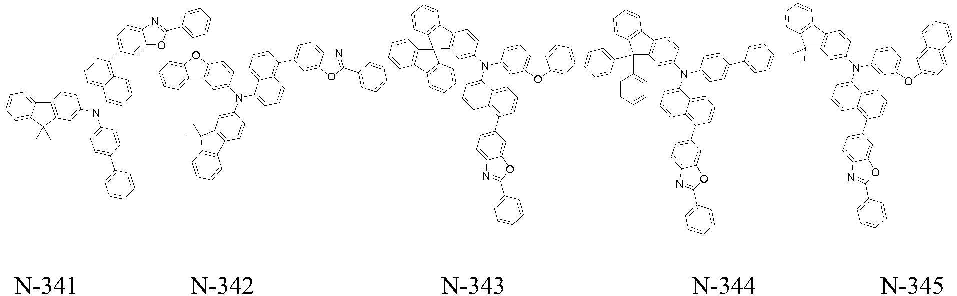 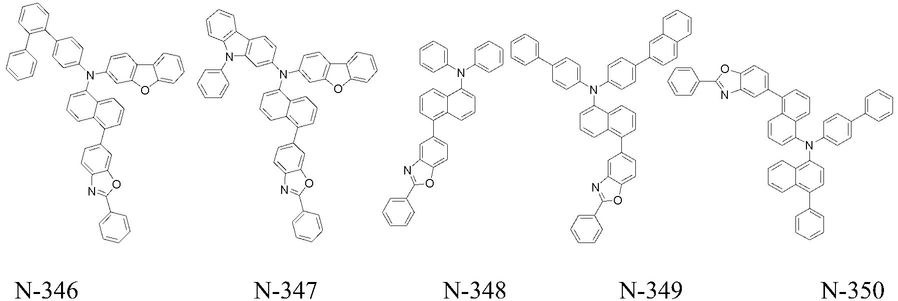 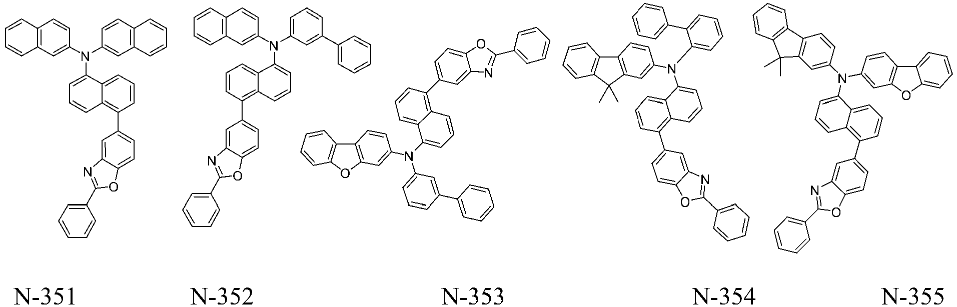 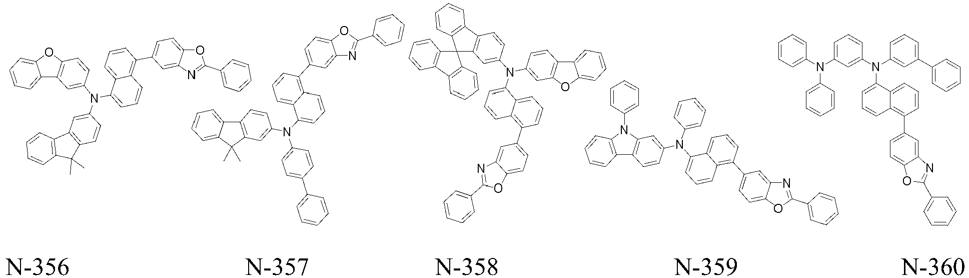 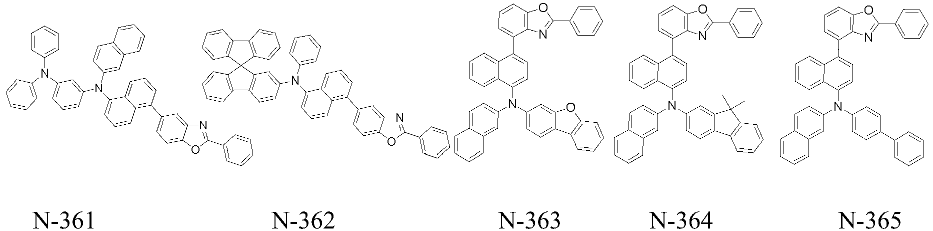 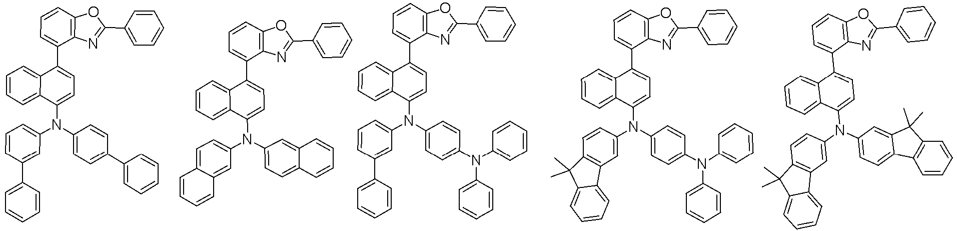 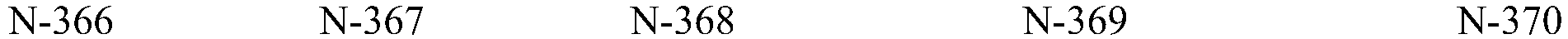 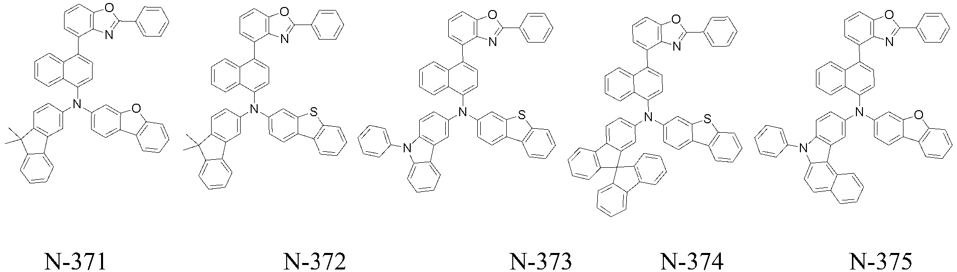 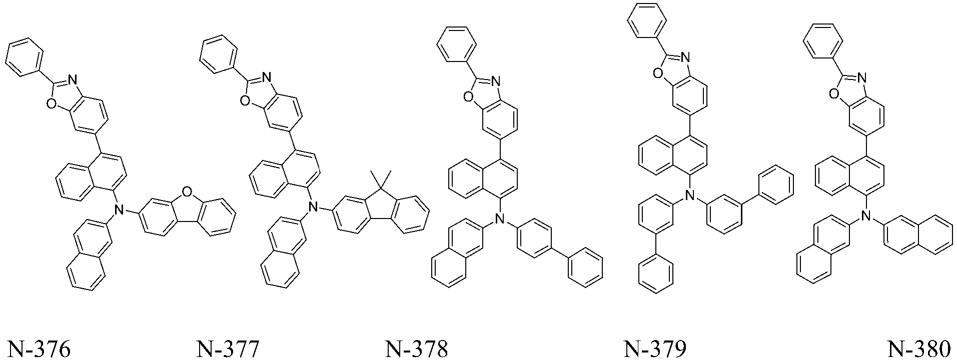 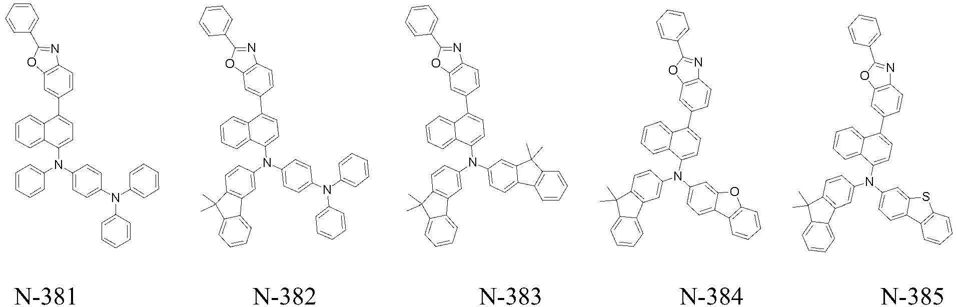 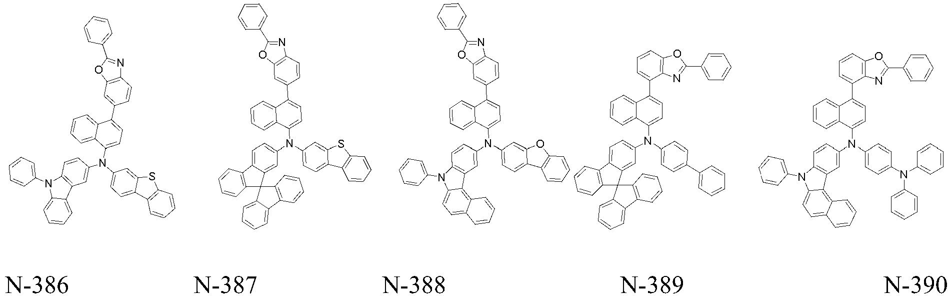 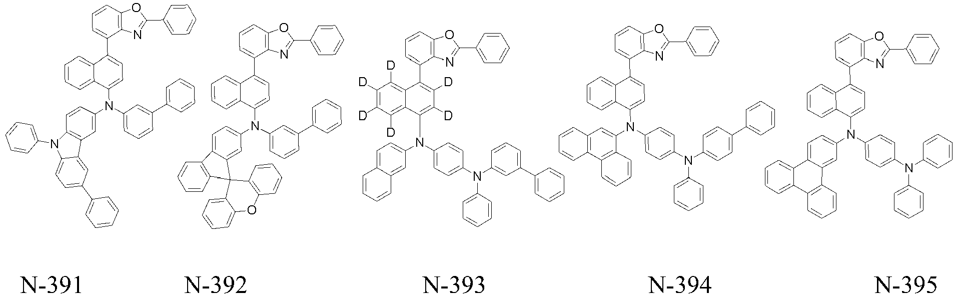 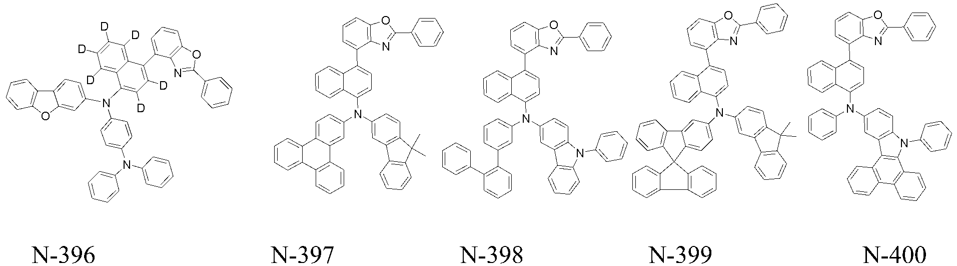 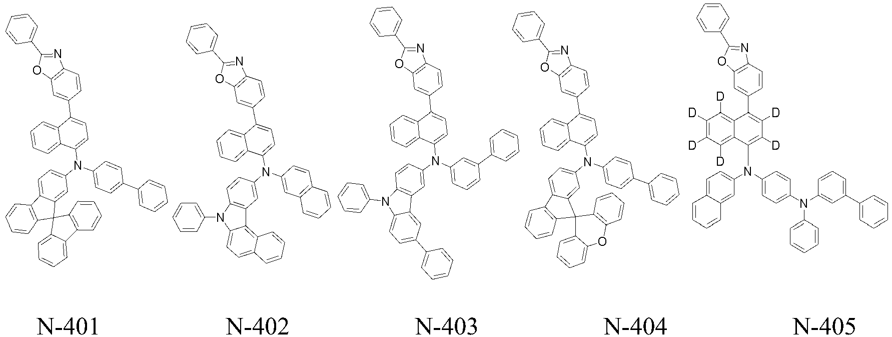 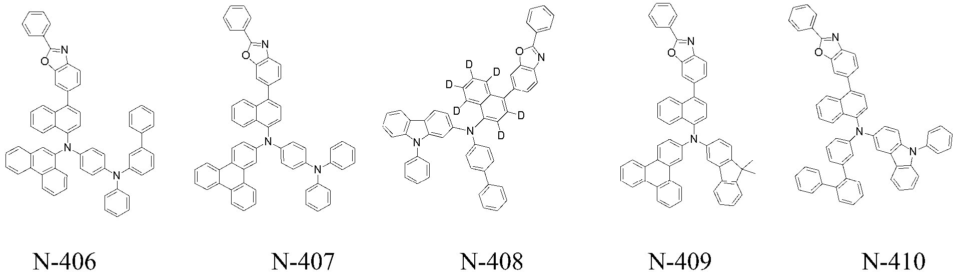 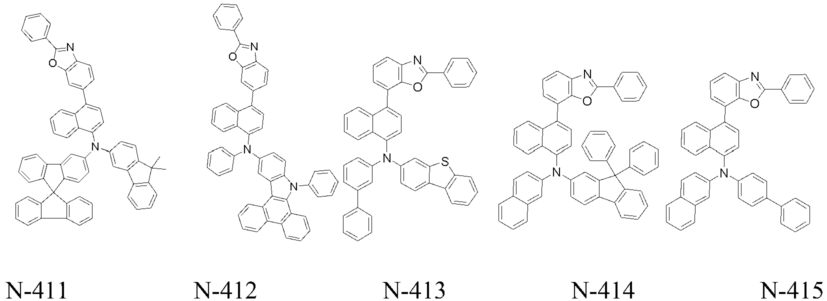 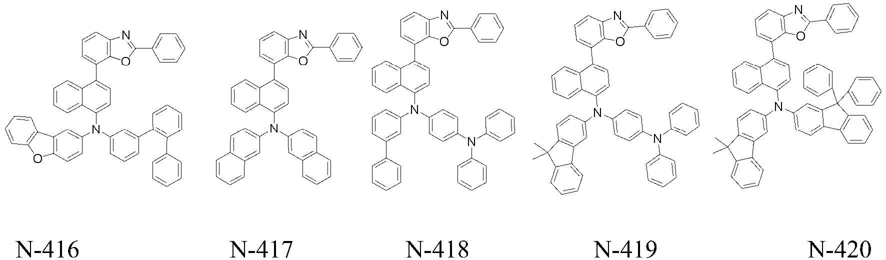 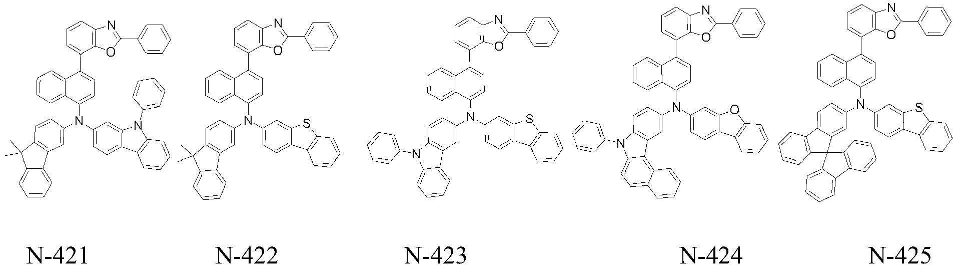 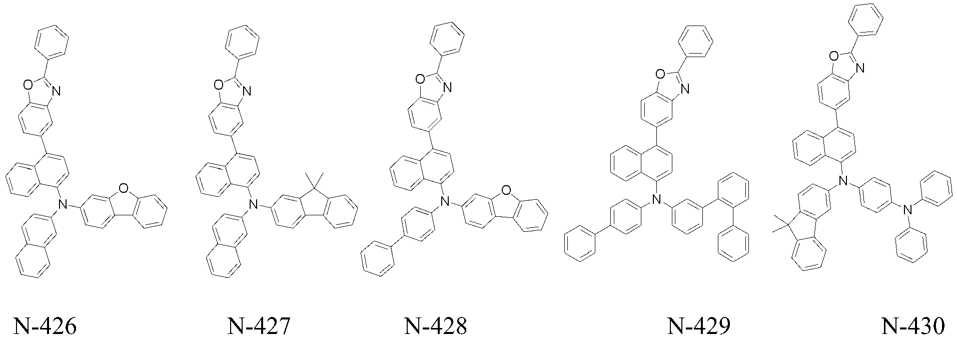 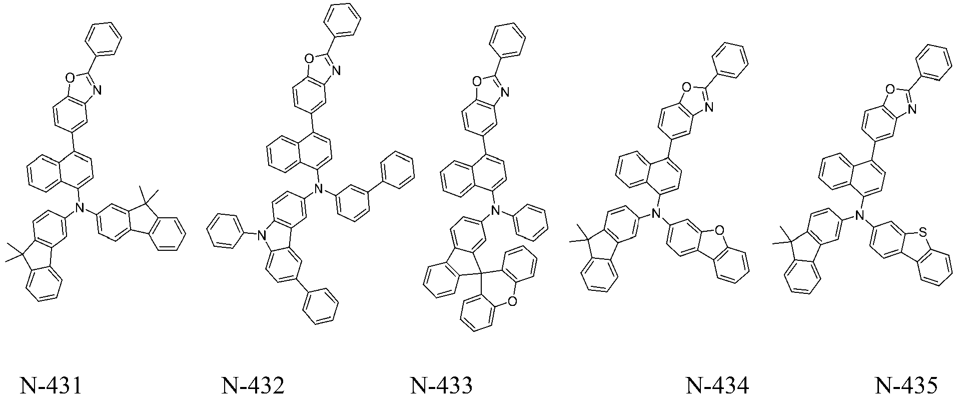 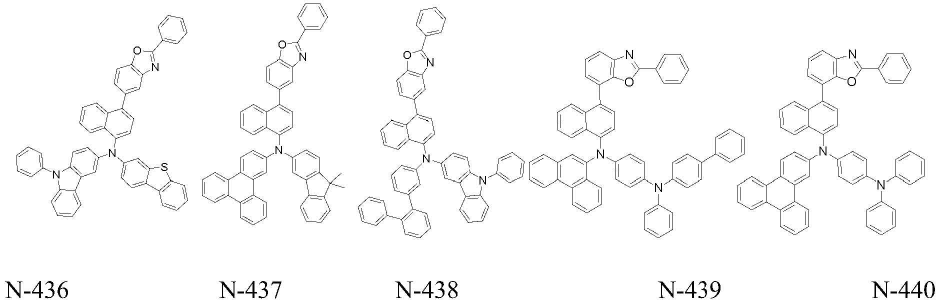 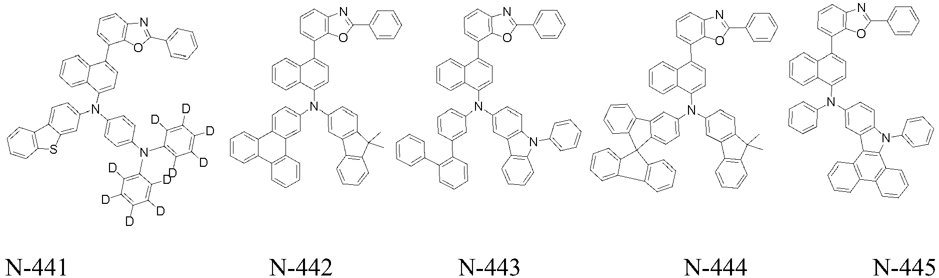 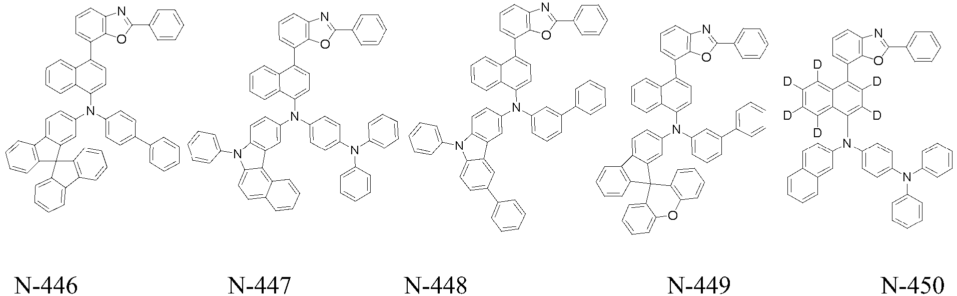 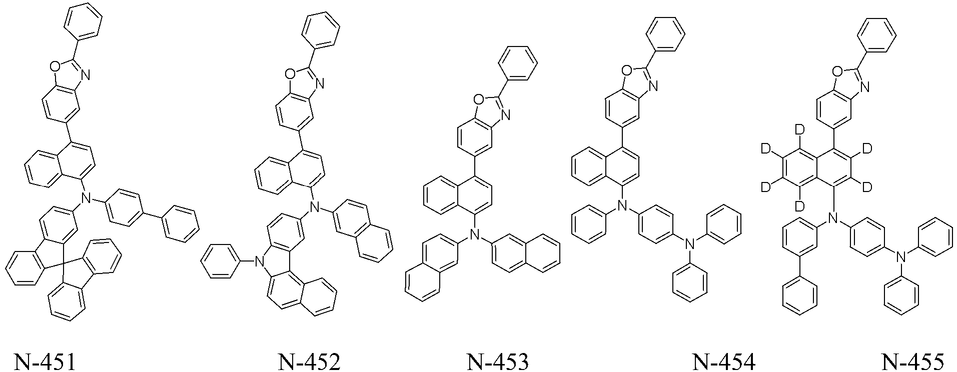 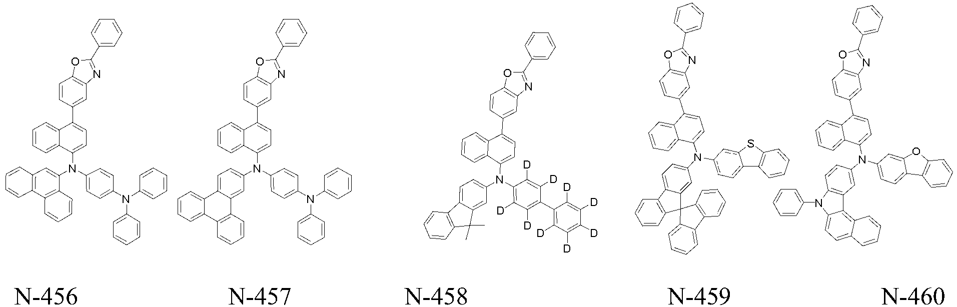 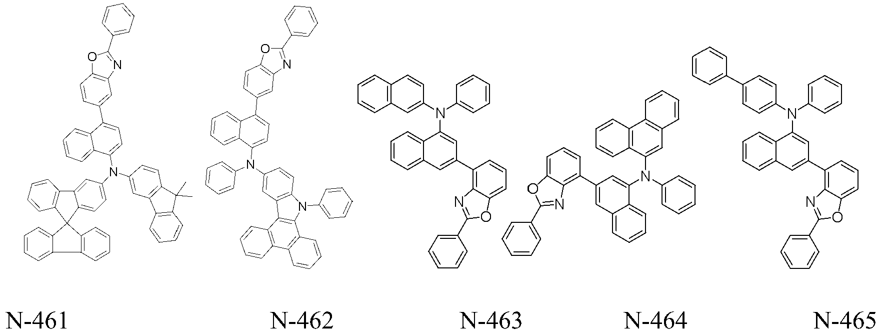 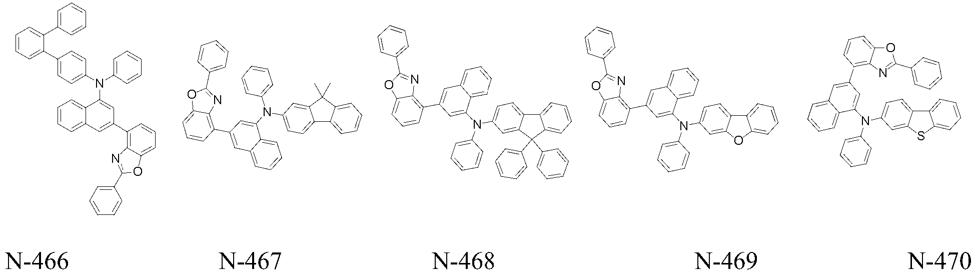 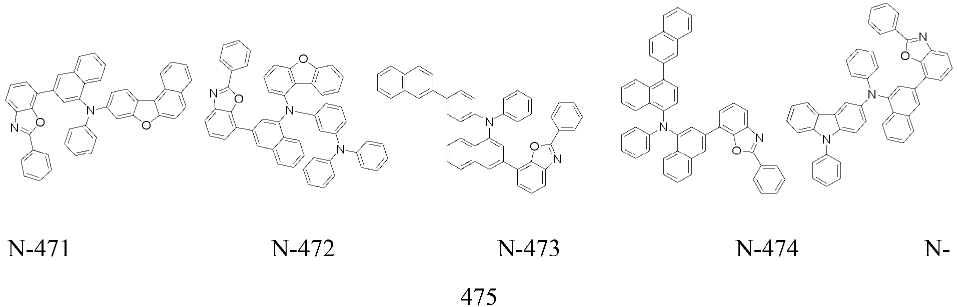 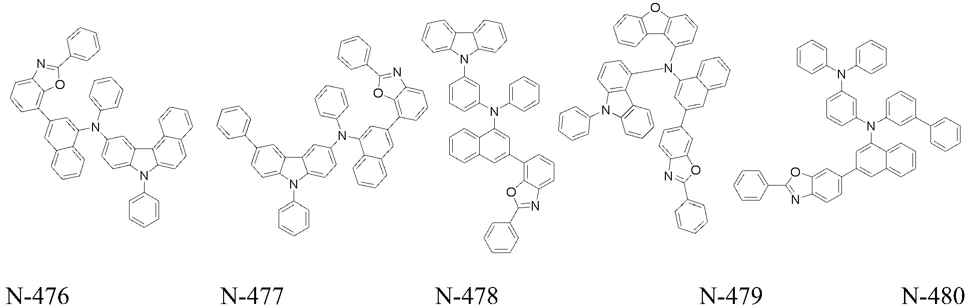 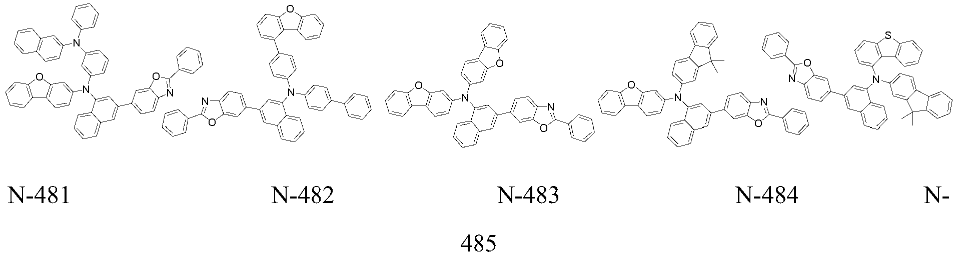 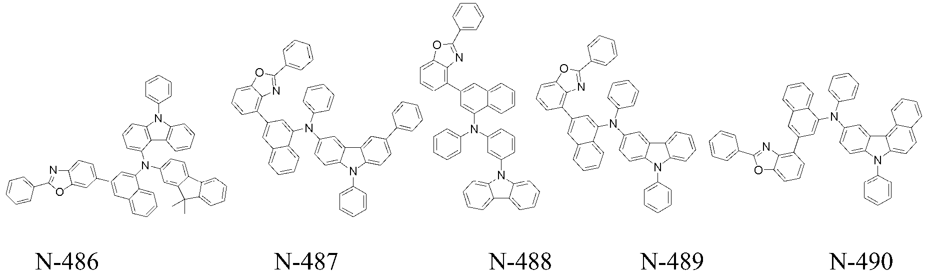 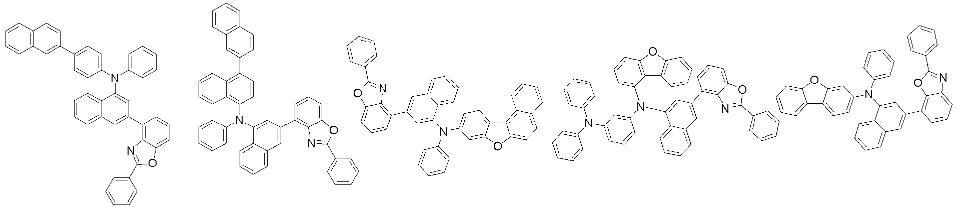 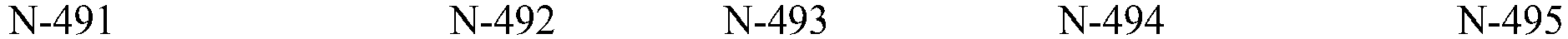 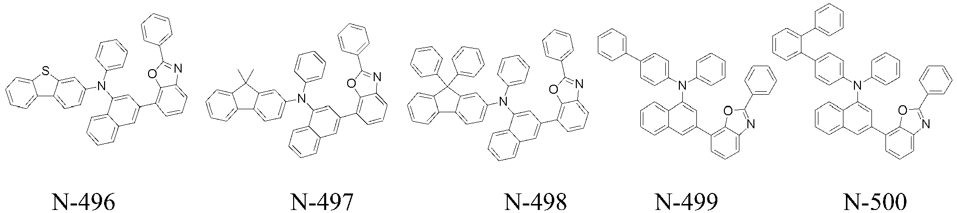 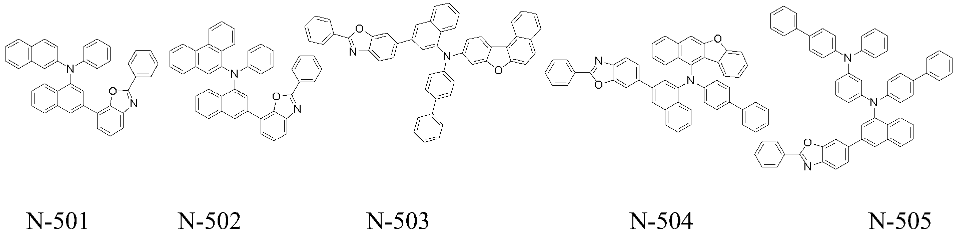 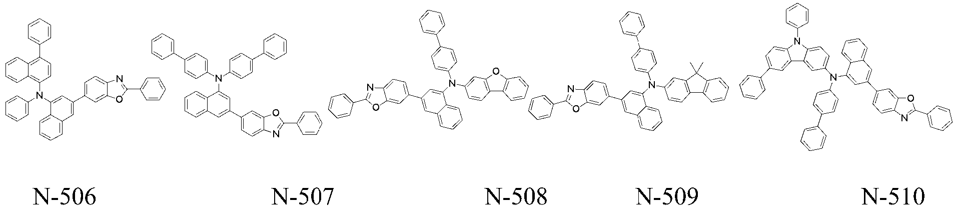 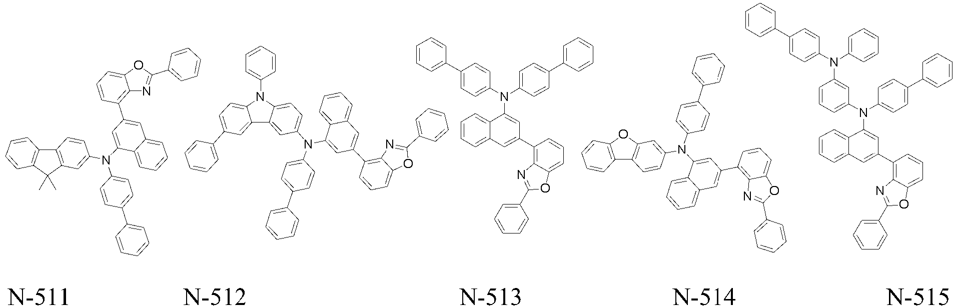 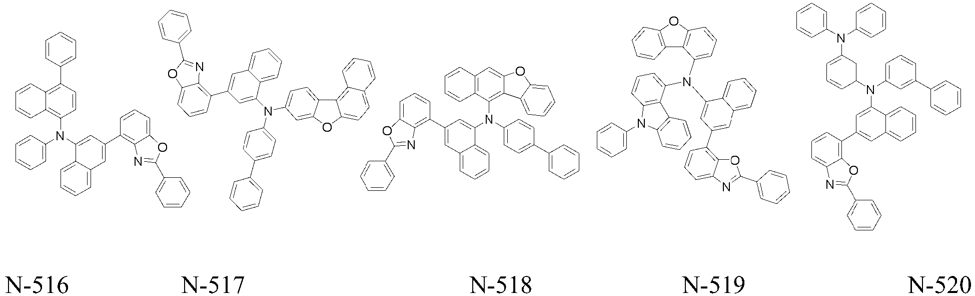 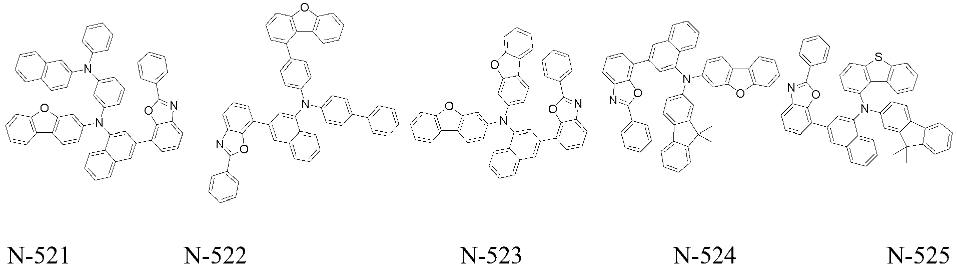 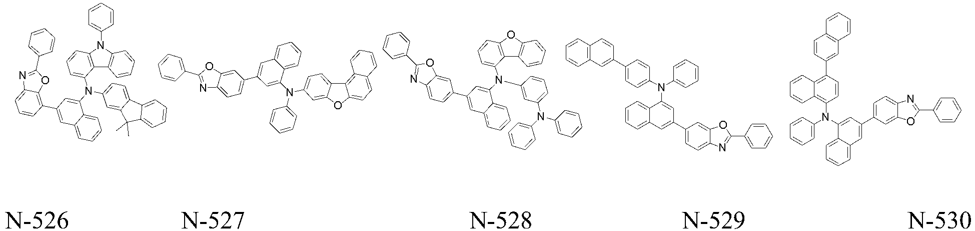 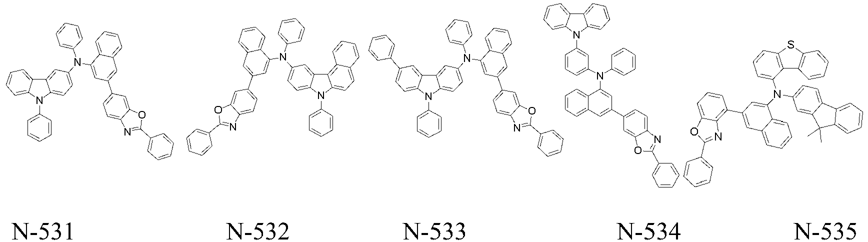 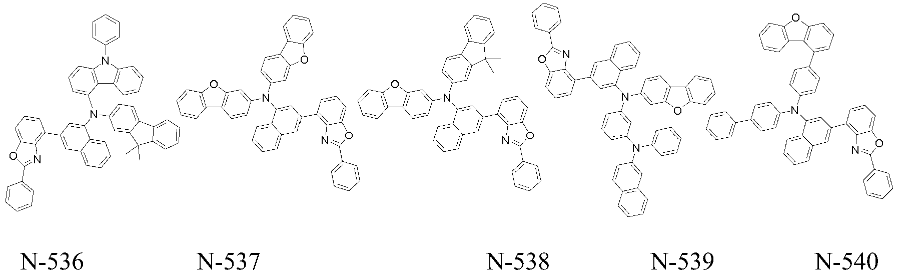 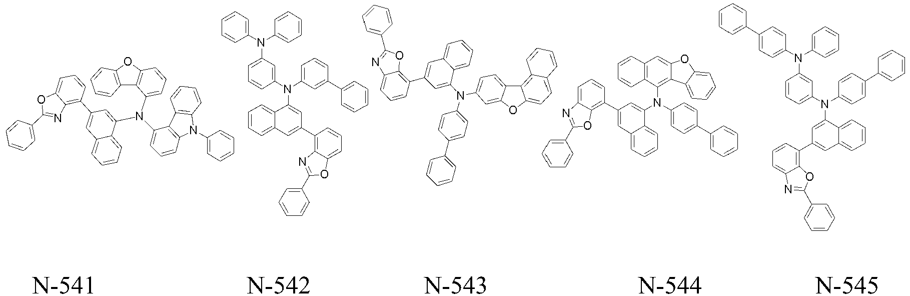 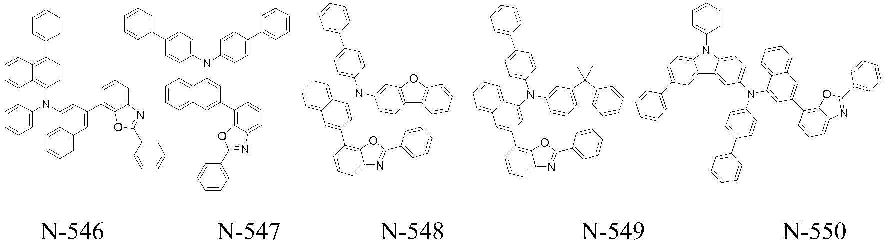 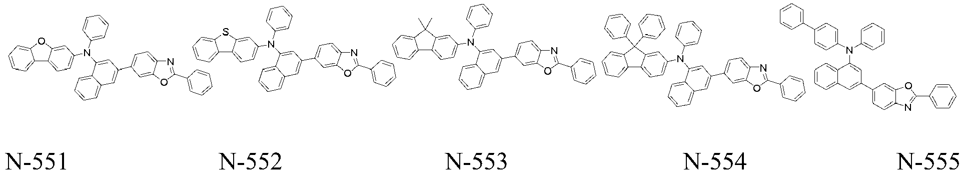 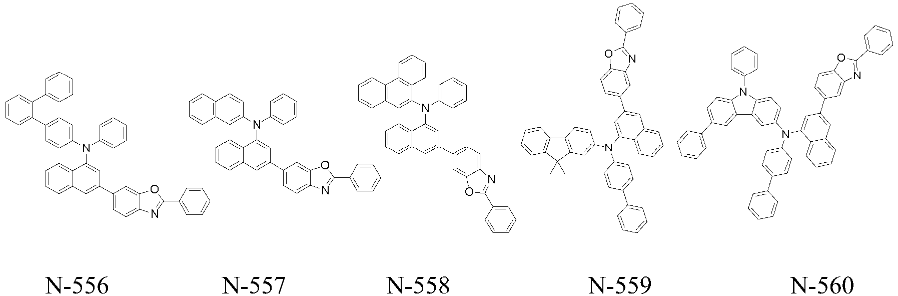 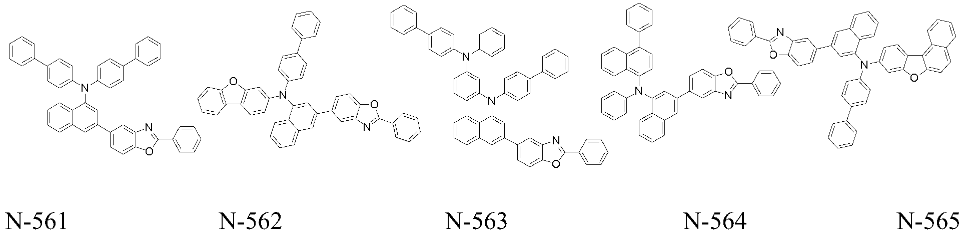 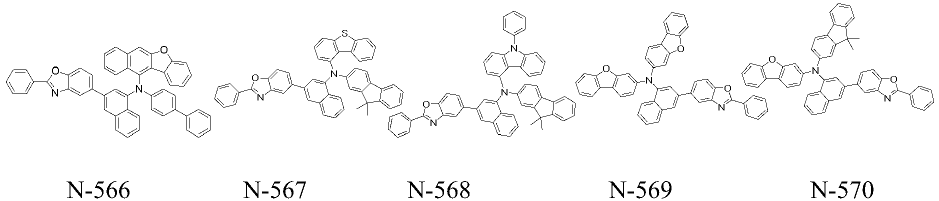 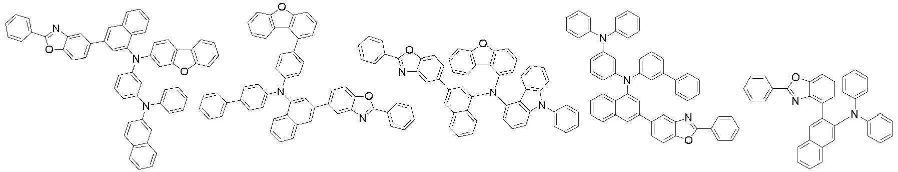 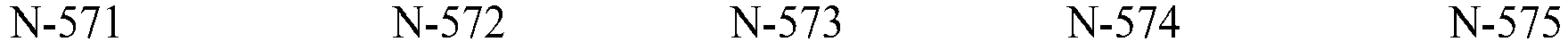 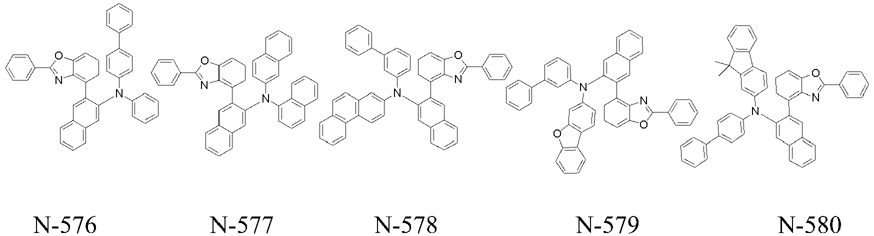 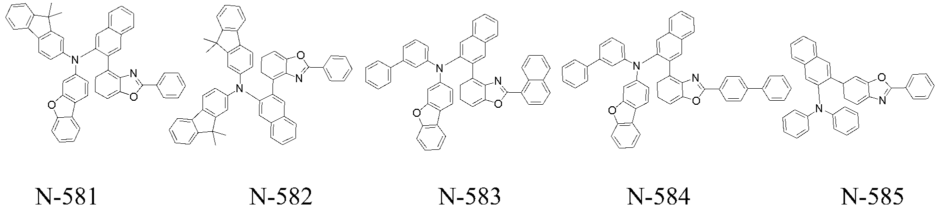 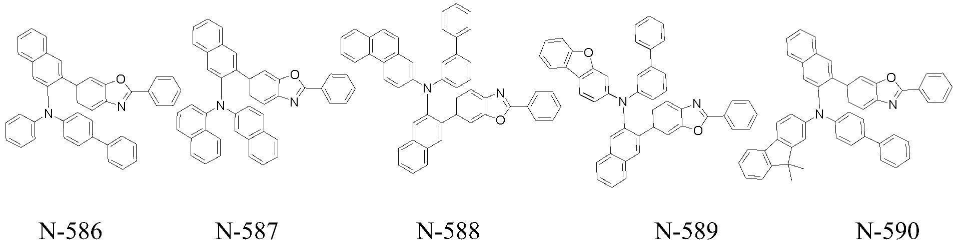 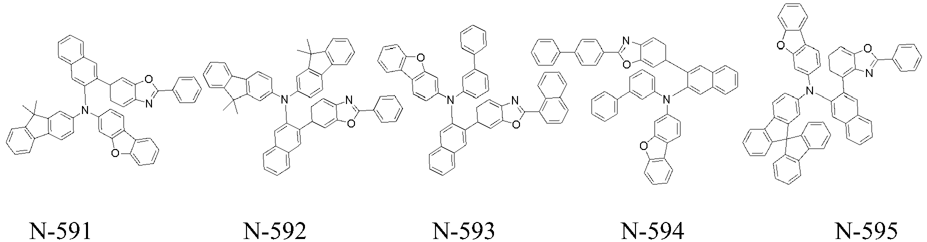 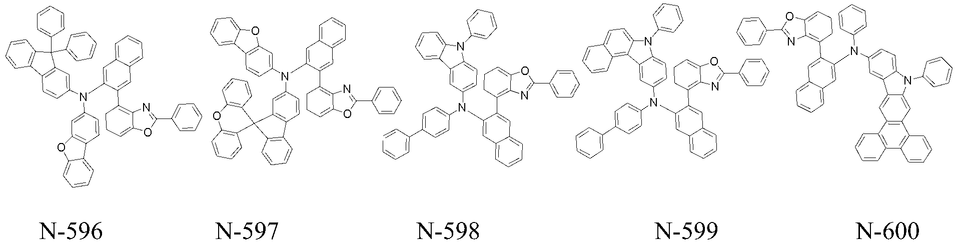 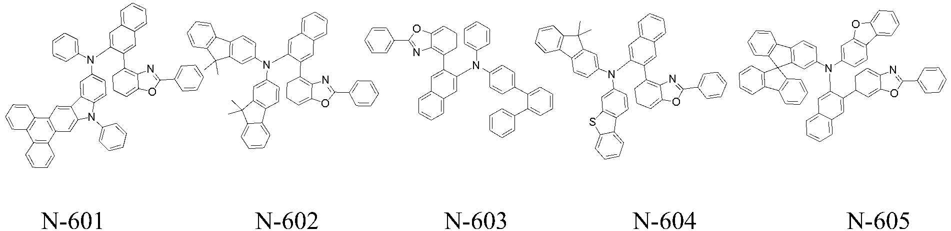 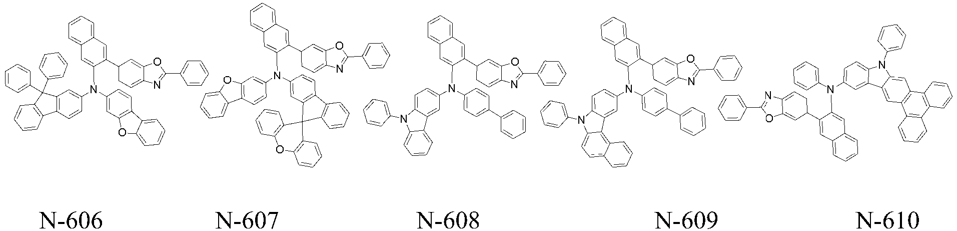 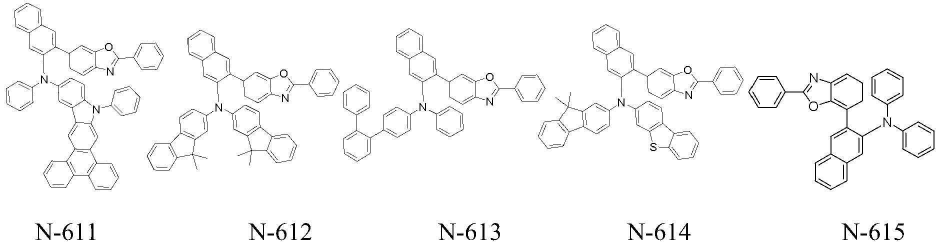 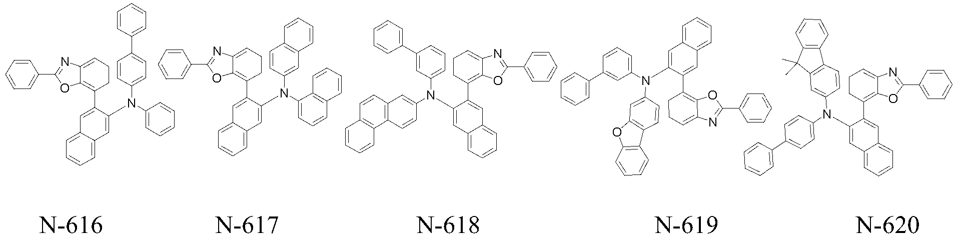 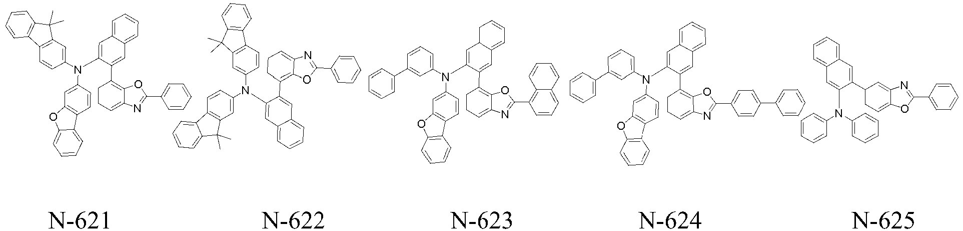 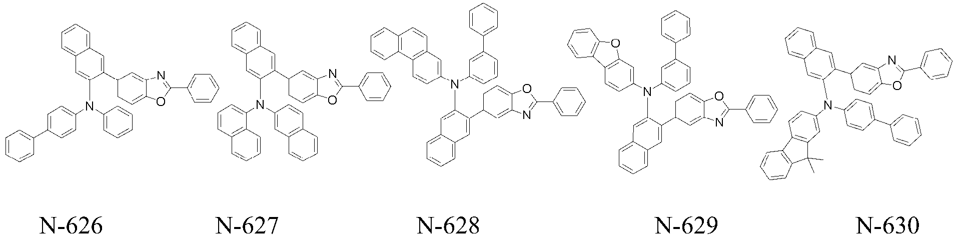 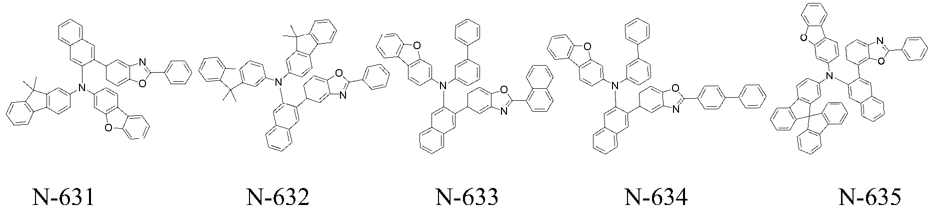 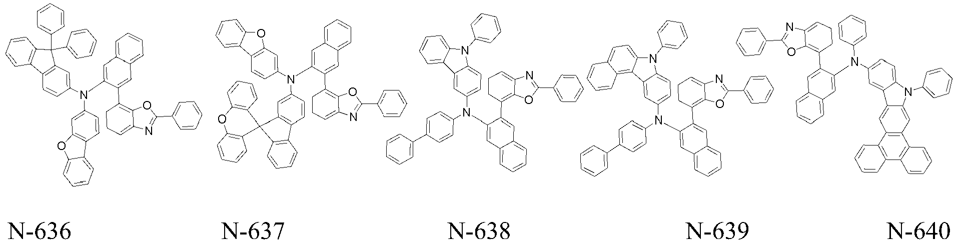 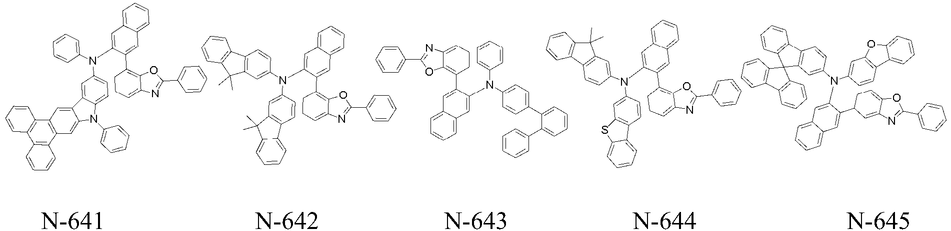 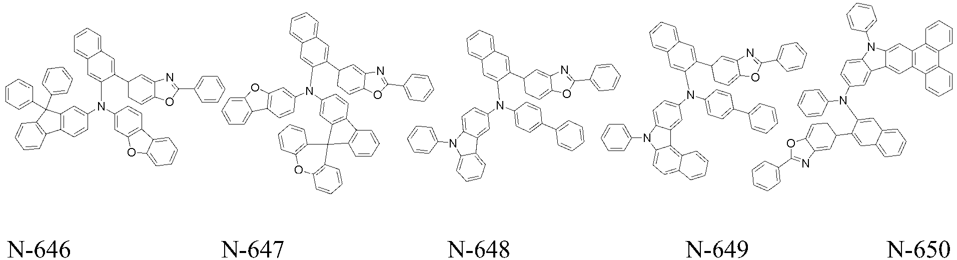 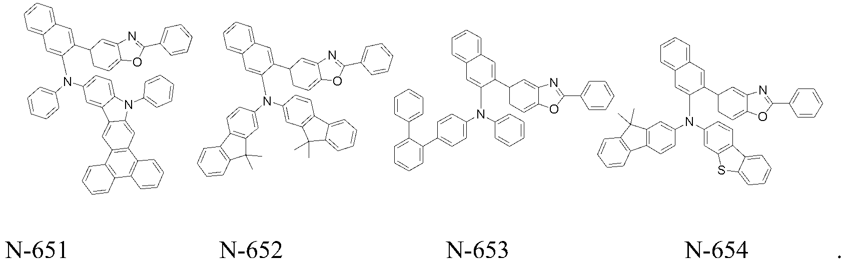
Optionally, the mass ratio of the first host material to the second host material is 9:1 to 1:9; and
Optionally, the mass ratio of the first host material to the second host material is 2:8 to 8:2; and
Optionally, the mass ratio of the first host material to the second host material is 3:7 to 7:3; and
Optionally, the mass ratio of the first host material to the second host material is further 4:6 to 6:4.

The present application also provides an organic electroluminescent material, comprising the deuterated compound as described above and the light-emitting host material composition as described above.

The present application further provides an organic electroluminescent device, comprising a cathode, an anode, and an organic layer between the cathode and the anode, wherein the organic layer comprises the deuterated compound as described above or the light-emitting host material composition as described above.

The present application also provides uses of the organic electroluminescent device as described above in fiber optic devices, illumination devices, electrophotographic photoreceptors, photoelectric converters, organic solar cells, switching devices, organic light-emitting field-effect transistors, image sensors, or dye lasers.

The beneficial effects of the present application are as follows:
The organic electroluminescent compound provided in the present application has the structure shown in Formula (1), wherein deuterium substitution at certain groups in Formula (1) enhances the quantum yield of the molecular, thereby improving the luminance efficiency of the organic light-emitting device comprising said compound. In the compound represented by Formula (1), deuterium substitution at specific positions and in specific quantities can improve the molecular stability of said compound; excessive deuteration, however, may lead to increased vibrational intensity and reduced stability. By precisely controlling the position and degree of deuteration, the present application achieves enhanced molecular stability, which contributes to an extended lifetime of the organic electroluminescent device comprising said compound. In summary, the organic electroluminescent compound provided in this application can effectively improve the efficiency and prolong the lifetime of the organic light-emitting device.

The present application provides a multi-host material composition, comprising a first host material and a second host material, wherein the first host material comprises an organic electroluminescent compound having the structure shown in Formula (1), and the second host material comprises a compound shown in Formula (2). The combination of the organic electroluminescent compound shown in Formula (1) and the compound shown in Formula (2) forms a dual-host system, which is used in the light-emitting layer of an organic electroluminescent device and can improve efficiency, reduce driving voltage, and extend the lifetime of the organic electroluminescent device.

### BRIEF DESCRIPTION OF THE DRAWINGS

To more clearly describe the technical solutions in the specific embodiments of the present application or in the prior art, a brief introduction to the drawings used in the description of the specific embodiments or the prior art is provided below. Obviously, the drawings described below are merely some embodiments of the present application. To those of ordinary skill in the art, without exercising inventive effort, additional drawings can also be derived from these.

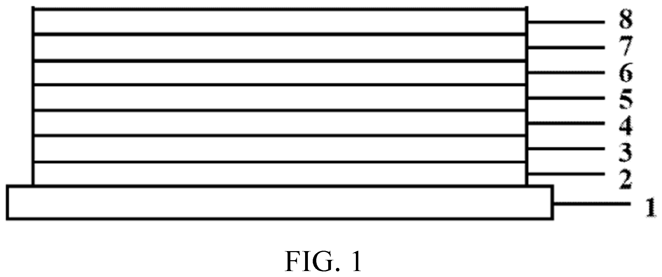

Figure 1 is a structural diagram of an organic electroluminescent device in a device embodiment of the present application;
1 - substrate; 2 - anode; 3 - hole injection layer; 4 - hole transport layer; 5 - light-emitting layer; 6 - electron transport layer; 7 - electron injection layer; and 8 - cathode.

### DETAILED DESCRIPTION

The following examples are provided to better and further understand the present application, and are not intended to limit the best modes described herein, nor restrict the content or scope of protection of the present application. Any product that is identical or similar to the present application, derived by anyone based on the teachings of this application or by combining features of this application with those of existing technologies, falls within the scope of protection of the present application.

Where specific experimental procedures or conditions are not specified in the examples, conventional procedures or conditions as described in the relevant literature in the field may be followed. Reagents or instruments that are used herein and not specified with regard to manufacturers are commercially available standard products.

### Synthesis of Intermediates:

### Synthesis of Intermediate N1-A

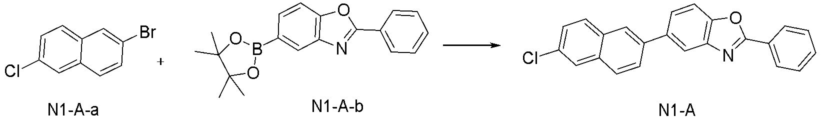

A three-necked reaction flask equipped with a mechanical stirrer, a thermometer, and a condenser tube was purged with nitrogen, then sequentially added Intermediate N1-A-a (10 mmol), Intermediate N1-A-b (10 mmol), toluene (100 mL), ethanol (20 mL), water (20 mL), potassium carbonate (20 mmol), and Pd(PPh₃)₄ (0.05 mmol), heated to 70-80 °C and reacted for 3 h. The reaction mixture was cooled to 25-30 °C, added water (100 mL) and toluene (100 mL), and then stirred and separated. The aqueous phase was extracted once with toluene (100 mL), then separated. The organic phases were combined and dried over 7 g of anhydrous sodium sulfate with stirring. After filtration, the organic phase was concentrated (-0.08 to 0.09 MPa, 55-60 °C) until no more liquid distilled. Then, 50 mL of a mixed solvent of dichloromethane and petroleum ether was added with stirring, cooled to 0-5 °C, and filtered to obtain Compound N1-A with a yield of 67%.
Elemental analysis: C₂₃H₁₄ClNO theoretical value: C, 77.64; H, 3.97; Cl, 9.96; N, 3.94; O, 4.50; measured value: C, 77.59; H, 3.98; Cl, 9.95; N, 3.98;
HRMS (ESI) m/z [M+H]⁺: theoretical value: 355.08; measured value: 356.32.

The preparation of the following Intermediates N2-A to N12-A was carried out in the same manner as that of N1-A, except that bromo- and chloro-substituted starting materials with different substitution sites from N1-A-a, and boronic ester-substituted starting materials with different substitution sites from N1-A-b were used.

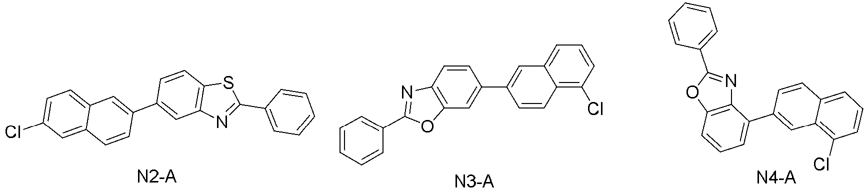

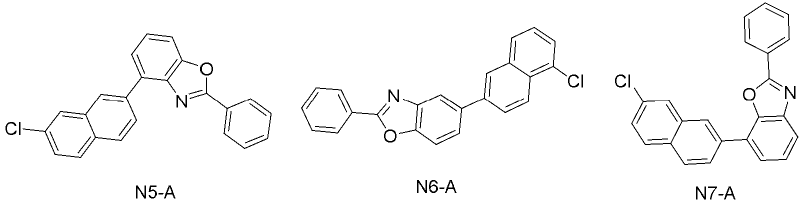

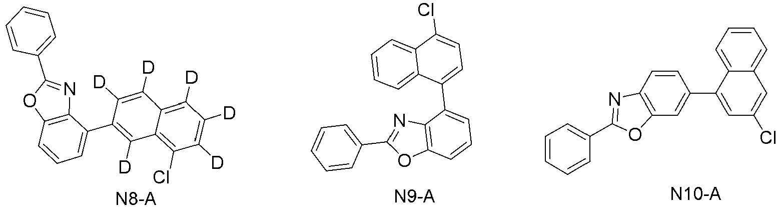

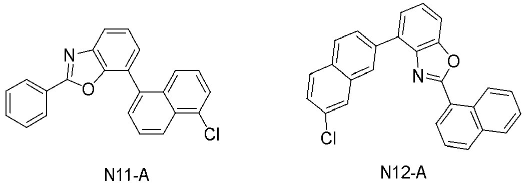

### Synthesis route of Intermediate N13-A:

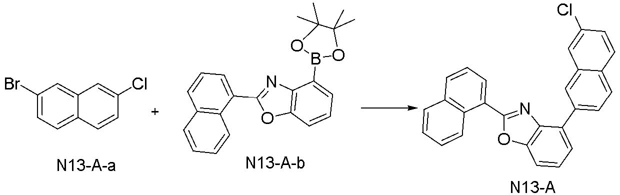

A three-necked reaction flask equipped with a mechanical stirrer, a thermometer, and a condenser tube was purged with nitrogen, then sequentially added Intermediate N13-A-a (10 mmol), Intermediate N13-A-b (10 mmol), toluene (100 mL), ethanol (20 mL), water (20 mL), potassium carbonate (20 mmol), and Pd(PPh₃)₄ (0.05 mmol), heated to 70 °C and reacted for 3 h. The reaction mixture was cooled to 25-30 °C, slowly added water (100 mL) and toluene (100 mL), and then stirred and separated. The aqueous phase was extracted once with toluene (100 mL), then separated. The organic phases were combined and washed multiple times with water until neutral, then dried over 7 g of anhydrous sodium sulfate with stirring. After filtration, the organic phase was concentrated (-0.08 to 0.09 MPa, 55-60 °C) until no more liquid distilled. Then, 50 mL of a mixed solvent of dichloromethane and petroleum ether was added with stirring, cooled to 0-5 °C, and filtered to obtain Compound N13-A with a yield of 72%.
Elemental analysis: C₂₇H₁₆ClNO theoretical value: C, 79.90; H, 3.97; Cl, 8.73; N, 3.45; O, 3.94; measured value: C, 79.90; H, 3.97; Cl, 8.73; N, 3.45; O, 3.94;
HRMS (ESI) m/z [M+H]⁺: theoretical value: 405.09; measured value: 406.12.

### Synthesis of Intermediate SubM1-B

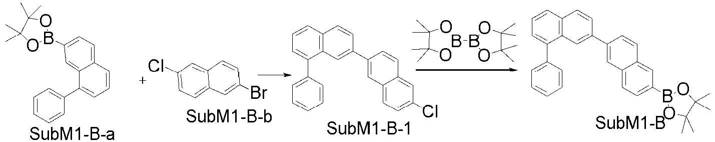

A three-necked reaction flask equipped with a mechanical stirrer, a thermometer, and a condenser tube was purged with nitrogen, then sequentially added starting material SubM1-B-b (10 mmol), Intermediate SubM1-B-a (10 mmol), toluene (100 mL), ethanol (20 mL), water (20 mL), potassium carbonate (20 mmol), and Pd(PPh₃)₄ (0.05 mmol), heated to 70-80 °C and reacted for 3 h. The reaction mixture was cooled to 25-30 °C, added water (100 mL) and toluene (100 mL), and then stirred and separated. The aqueous phase was extracted once with toluene (100 mL), then separated. The organic phases were combined and dried over 7 g of anhydrous sodium sulfate with stirring. After filtration, the organic phase was concentrated (-0.08 to 0.09 MPa, 55-60 °C) until no more liquid distilled. Then, 50 mL of a mixed solvent of dichloromethane and petroleum ether was added with stirring, cooled to 0-5 °C, and filtered to obtain Compound SubM1-B-1 with a yield of 59%.

A three-necked reaction flask equipped with a mechanical stirrer, a thermometer, and a condenser tube was purged with nitrogen, then sequentially added Intermediate SubM1-B-1 (10 mmol), starting material bis(pinacolato)diboron (10 mmol), 1,4-dioxane (100 mL), potassium acetate (20 mmol), and Pd₂(dba)₃ (0.05 mmol), heated to 100 °C and reacted for 3 h. The reaction mixture was cooled to 25-30 °C, added water (100 mL) and toluene (100 mL), and then stirred and separated. The aqueous phase was extracted once with toluene (100 mL), then separated. The organic phases were combined and dried over 7 g of anhydrous sodium sulfate with stirring. After filtration, the organic phase was concentrated (-0.08 to 0.09 MPa, 55-60 °C) until no more liquid distilled. Then, 50 mL of a mixed solvent of dichloromethane and petroleum ether was added with stirring, cooled to 0-5 °C, and filtered to obtain Intermediate SubM1-B with a yield of 45%.

### Synthesis of Intermediate SubM2-B

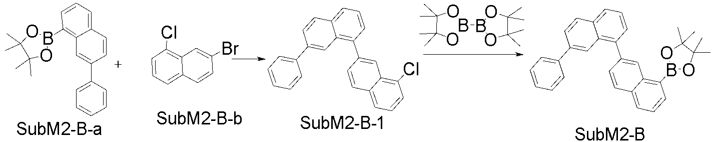

The preparation of SubM2-B was carried out in the same manner as that of SubM1-B, except that SubM2-B-a was used instead of SubM1-B-a and SubM2-B-b instead of SubM1-B-b, to obtain SubM2-B with a yield of 52%.

### Synthesis of Intermediate SubM3-B

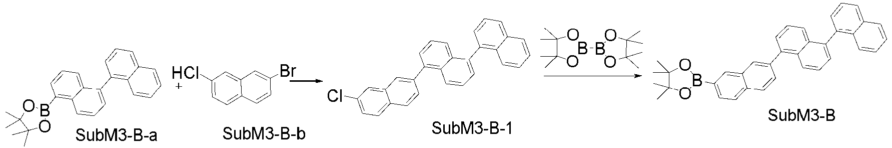

The preparation of SubM3-B was carried out in the same manner as that of SubM1-B, except that SubM3-B-a was used instead of SubM1-B-a and SubM3-B-b instead of SubM1-B-b, to obtain SubM3-B with a yield of 42%.

### Synthesis of Intermediate SubM1-A

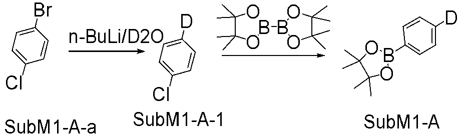

A three-necked reaction flask equipped with a mechanical stirrer, a cryogenic thermometer, and a constant pressure dropping funnel was purged with nitrogen, then sequentially added starting material SubM1-A-a (10 mmol) and tetrahydrofuran (100 ml), stirred and cooled to -70 to -80 °C. N-butyl lithium (12 mmol)was then added dropwise while maintaining the temperature. After the addition was complete, the temperature was maintained for1 h. Then, D₂O (20 mmol) was added slowly. The mixture was allowed to warm naturally to 25-30 °C, followed by the addition of 100 ml waterand100 ml toluene, and then stirred and separated. The aqueous phase was extracted once with toluene (100 ml), then separated. The organic phases were combined and dried over 7 g of anhydrous sodium sulfate with stirring. After filtration, the organic phase was concentrated (-0.08 to 0.09 MPa, 55-60 °C) until no more liquid distilled, to obtain Compound SubM1-A-1 with a yield of 76%.

A three-necked reaction flask equipped with a mechanical stirrer, a thermometer, and a condenser tube was purged with nitrogen, then sequentially added Intermediate SubM1-A-1 (10 mmol), starting material bis(pinacolato)diboron (12 mmol), 1,4-dioxane (100 ml), potassium acetate (20 mmol), and Pd2(dba)3 (0.05 mmol), heated to 100 °C and reacted for 3 h. The reaction mixture was cooled to 25-30 °C, added water (100 ml) and toluene (100 ml), and then stirred and separated. The aqueous phase was extracted once with toluene (100 ml), then separated. The organic phases were combined and dried over 7 g of anhydrous sodium sulfate with stirring, filtered, and concentrated (-0.08 to 0.09 MPa, 55-60 °C) until no more liquid distilled. Then, 50 ml of a mixed solvent of dichloromethane and petroleum ether was added with stirring, cooled to 0-5 °C, and filtered to obtain Compound SubM1-A with a yield of 62%.

### Synthesis of Intermediate SubM2-A

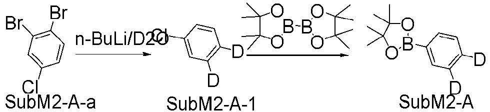

The preparation of SubM2-A was carried out in the same manner as that of SubM1-A, except that SubM2-A-a was used instead of SubM1-A-a, to obtain SubM2-A with a yield of 75%.

### Synthesis of Intermediate SubM3-A

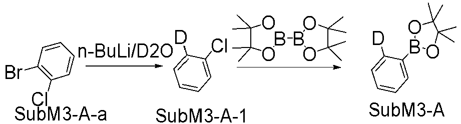

The preparation of SubM3-A was carried out in the same manner as that of SubM1-A, except that SubM3-A-a was used instead of SubM1-A-a, to obtain SubM3-A with a yield of 68%.

### Synthesis of Intermediate SubM4-A

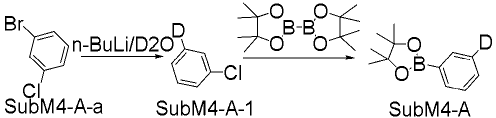

The preparation of SubM4-A was carried out in the same manner as that of SubM1-A, except that SubM4-A-a was used instead of SubM1-A-a, to obtain SubM4-A with a yield of 72%.

### Synthesis of Intermediate SubMS-A

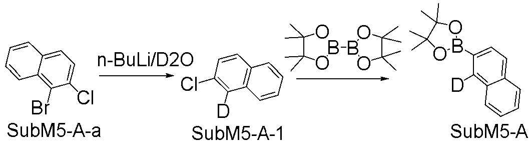

The preparation of SubM5-A was carried out in the same manner as that of SubM1-A, except that SubM5-A-a was used instead of SubM1-A-a, to obtain SubM5-A with a yield of 62%.

### Synthesis of Intermediate SubM6-A

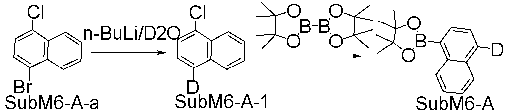

The preparation of SubM6-A was carried out in the same manner as that of SubM1-A, except that SubM6-A-a was used instead of SubM1-A-a, to obtain SubM6-A with a yield of 68%.

### Synthesis of Intermediate SubM7-A

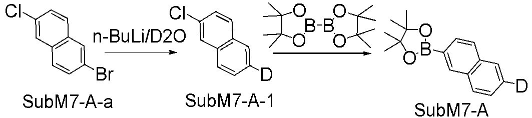

The preparation of SubM7-A was carried out in the same manner as that of SubM1-A, except that SubM7-A-a was used instead of SubM1-A-a, to obtain SubM7-A with a yield of 70%.

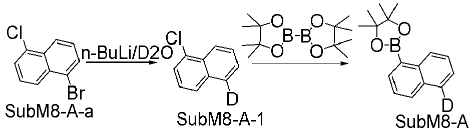

The preparation of SubM8-A was carried out in the same manner as that of SubM1-A, except that SubM8-A-a was used instead of SubM1-A-a, to obtain SubM8-A with a yield of 65%.

### Synthesis of Intermediate SubM1-B

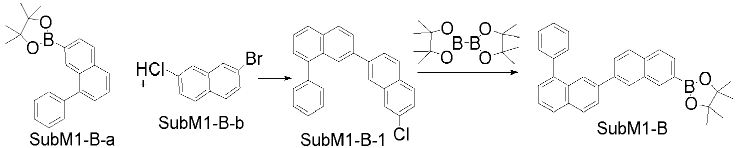

A three-necked reaction flask equipped with a mechanical stirrer, a thermometer, and a condenser tube was purged with nitrogen, then sequentially added starting material SubM1-B-b (10 mmol), Intermediate SubM1-B-a (10 mmol), toluene (100 mL), ethanol (20 mL), water (20 mL), potassium carbonate (20 mmol), and Pd(PPh₃)₄ (0.05 mmol), heated to 70-80 °C and reacted for 3 h. The reaction mixture was cooled to 25-30 °C, added water (100 mL) and toluene (100 mL), and then stirred and separated. The aqueous phase was extracted once with toluene (100 mL), then separated. The organic phases were combined and dried over 7 g of anhydrous sodium sulfate with stirring. After filtration, the organic phase was concentrated (-0.08 to 0.09 MPa, 55-60 °C) until no more liquid distilled. Then, 50 mL of a mixed solvent of dichloromethane and petroleum ether was added with stirring, cooled to 0-5 °C, and filtered to obtain Compound SubM1-B-1 with a yield of 59%.

A three-necked reaction flask equipped with a mechanical stirrer, a thermometer, and a condenser tube was purged with nitrogen, then sequentially added Intermediate SubM1-B-1 (10 mmol), starting material bis(pinacolato)diboron (12 mmol), 1,4-dioxane (100 mL), potassium acetate (20 mmol), and Pd₂(dba)₃ (0.05 mmol), heated to 100 °C and reacted for 3 h. The reaction mixture was cooled to 25-30 °C, added water (100 mL) and toluene (100 mL), and then stirred and separated. The aqueous phase was extracted once with toluene (100 mL), then separated. The organic phases were combined and dried over 7 g of anhydrous sodium sulfate with stirring. After filtration, the organic phase was concentrated (-0.08 to 0.09 MPa, 55-60 °C) until no more liquid distilled. Then, 50 mL of a mixed solvent of dichloromethane and petroleum ether was added with stirring, cooled to 0-5 °C, and filtered to obtain Intermediate SubM1-B with a yield of 45%.

### Synthesis of Intermediate SubM2-B

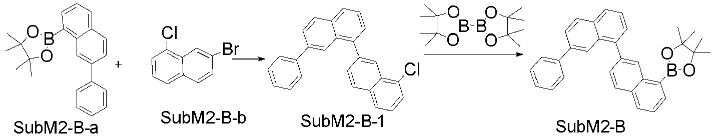

The preparation of SubM2-B was carried out in the same manner as that of SubM1-B, except that SubM2-B-a was used instead of SubM1-B-a and SubM2-B-b instead of SubM1-B-b, to obtainSubM2-B with a yield of 52%.

### Synthesis of Intermediate SubM3-B

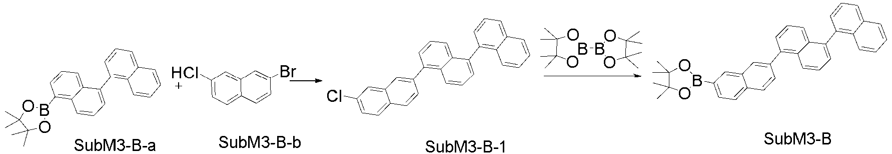

The preparation of SubM3-B was carried out in the same manner as that of SubM1-B, except that SubM3-B-a was used instead of SubM1-B-a and SubM3-B-b instead of SubM1-B-b, to obtain SubM3-B with a yield of 42%.

### Synthesis of Intermediate SubM1-C

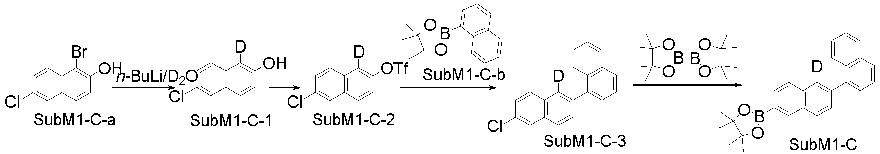

### Step 1:

A three-necked reaction flask equipped with a mechanical stirrer, a cryogenic thermometer, and a constant pressure dropping funnel was purged with nitrogen, then sequentially added starting material SubM1-C-a (14 mmol) and tetrahydrofuran (140 mL), stirred and cooled to -70 to -80 °C. N-butyl lithium (16 mmol)was then added dropwise while maintaining the temperature. After the addition was complete, the temperature was maintained for1 h. Then, D₂O (10 mmol)was added slowly. The mixture was allowed to warm naturally to 25-30 °C, followed by the addition of 100 mL waterand100 mL toluene, and then stirred and separated. The aqueous phase was extracted once with toluene (100 mL), then separated. The organic phases were combined and dried over 7 g of anhydrous sodium sulfate with stirring. After filtration, the organic phase was concentrated (-0.08 to 0.09 MPa, 55-60 °C) until no more liquid distilled, to obtain Compound SubM1-C-1 with a yield of 76%.

### Step 2:

A three-necked reaction flask equipped with a mechanical stirrer, a cryogenic thermometer, and a constant pressure dropping funnel was purged with nitrogen, then sequentially added Intermediate SubM1-C-1 (10 mmol) and dichloromethane (100 mL), stirred and cooled to -10°C. Triethylamine (12 mmol)was then added dropwise. After the addition was complete, the temperature was maintained for1 h. Then, trifluoromethanesulfonic anhydride (10 mmol)was added slowly. The mixture was allowed to warm naturally to 25-30 °C, followed by the addition of 100 mL water and 100 mL dichloromethane, and then stirred and separated. The aqueous phase was extracted once with dichloromethane (100 mL), then separated. The organic phases were combined and dried over 7 g of anhydrous sodium sulfate with stirring. After filtration, the organic phase was concentrated (-0.08 to 0.09 MPa, 55-60 °C) until no more liquid distilled, to obtain Compound SubM1-C-2 with a yield of 86%.

### Step 3:

A three-necked reaction flask equipped with a mechanical stirrer, a thermometer, and a condenser tube was purged with nitrogen, then sequentially added Intermediate SubM1-C-2 (8 mmol), starting material SubM1-C-a (8 mmol), potassium carbonate (16 mmol), Pd(PPh₃)₄ (0.04 mmol), 1,4-dioxane (100 mL), and water (30 mL), stirred, heated to 70-75 °C and reacted for 3 h. The reaction mixture was cooled to 25-30 °C, added water (100 mL) and dichloromethane (100 mL), and then stirred and separated. The aqueous phase was extracted once with dichloromethane (100 mL), then separated. The organic phases were combined and dried over 7 g of anhydrous sodium sulfate with stirring. After filtration, the organic phase was concentrated (-0.08 to 0.09 MPa, 55-60 °C) until no more liquid distilled. Then, 20 mL petroleum ether was added with stirring, cooled to 0-5 °C, and filtered to obtain a crude product, which was recrystallized from toluene to obtain the product SubM1-C-3 with a yield of 77%.

### Step 4:

A three-necked reaction flask equipped with a mechanical stirrer, a thermometer, and a condenser tube was purged with nitrogen, then sequentially added Intermediate SubM1-C-3 (10 mmol), starting material bis(pinacolato)diboron (6 mmol), 1,4-dioxane (100 mL), potassium acetate (12 mmol), and Pd₂(dba)₃ (0.05 mmol), heated to 100 °C and reacted for 3 h. The reaction mixture was cooled to 25-30 °C, added water (100 mL) and toluene (100 mL), and then stirred and separated. The aqueous phase was extracted once with toluene (100 mL), then separated. The organic phases were combined and dried over 7 g of anhydrous sodium sulfate with stirring. After filtration, the organic phase was concentrated (-0.08 to 0.09 MPa, 55-60 °C) until no more liquid distilled. Then, 50 mL of a mixed solvent of dichloromethane and petroleum ether was added with stirring, cooled to 0-5 °C, and filtered to obtain Compound SubM1-C with a yield of 63%.

### Synthesis of Intermediate SubM2-C

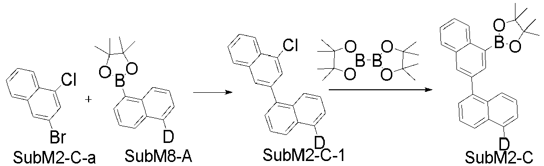

A three-necked reaction flask equipped with a mechanical stirrer, a thermometer, and a condenser tube was purged with nitrogen, then sequentially added starting material SubM2-C-a (10 mmol), Intermediate SubM8-A (10 mmol), potassium carbonate (20 mmol), Pd(PPh₃)₄ (0.05 mmol), 1,4-dioxane (100 mL), and water (30 mL), stirred, heated to 70-75 °C and reacted for 3 h. The reaction mixture was cooled to 25-30 °C, added water (100 mL) and dichloromethane (100 mL), and then stirred and separated. The aqueous phase was extracted once with dichloromethane (100 mL), then separated. The organic phases were combined and dried over 7 g of anhydrous sodium sulfate with stirring. After filtration, the organic phase was concentrated (-0.08 to 0.09 MPa, 55-60 °C) until no more liquid distilled. Then, 20 mL petroleum ether was added with stirring, cooled to 0-5 °C, and filtered to obtain a crude product, which was recrystallized from toluene to obtain Intermediate SubM2-C-1 with a yield of 72%.

A three-necked reaction flask equipped with a mechanical stirrer, a thermometer, and a condenser tube was purged with nitrogen, then sequentially added Intermediate SubM4-C-1 (10 mmol), starting material bis(pinacolato)diboron (12 mmol), 1,4-dioxane (100 mL), potassium acetate (20 mmol), and Pd₂(dba)₃ (0.05 mmol), heated to 100 °C and reacted for 3 h. The reaction mixture was cooled to 25-30 °C, added water (100 mL) and toluene (100 mL), and then stirred and separated. The aqueous phase was extracted once with toluene (100 mL), then separated. The organic phases were combined and dried over 7 g of anhydrous sodium sulfate with stirring. After filtration, the organic phase was concentrated (-0.08 to 0.09 MPa, 55-60 °C) until no more liquid distilled. Then, 50 mL of a mixed solvent of dichloromethane and petroleum ether was added with stirring, cooled to 0-5 °C, and filtered to obtain Intermediate SubM2-C with a yield of 70%.

### Synthesis of Intermediate SubM3-C

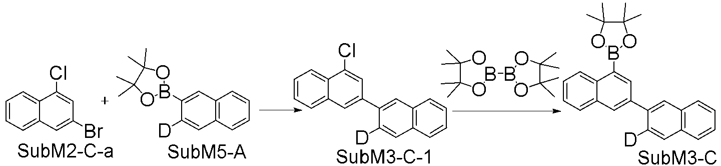

The preparation of SubM3-C was carried out in the same manner as that of SubM2-C, except that SubM5-A was used instead of SubM8-A, to obtain Intermediate SubM3-C with a yield of 70%.

### Synthesis of Intermediate SubM4-C

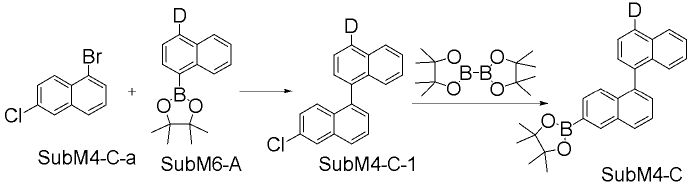

The preparation of SubM4-C was carried out in the same manner as that of SubM2-C, except that SubM4-C-a was used instead of SubM2-C-a and SubM6-A instead of SubM8-A, to obtain Intermediate SubM4-C with a yield of 69%.

### Example 1

This example provides the deuterated compound N-1. The synthesis of deuterated compound N-1 specifically involves the following steps:

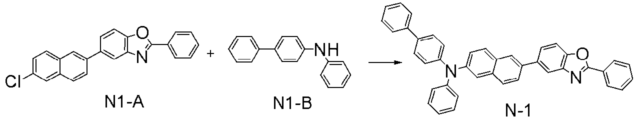

A three-necked reaction flask equipped with a mechanical stirrer, a thermometer, and a condenser tube was purged with nitrogen, then sequentially added Intermediate N1-B (10 mmol), Intermediate N1-A (10 mmol), and toluene (100 mL), heated to reflux for 0.5 h with removal of water, and cooled to 70-80 °C. Sodium tert-butoxide (15 mmol), Pd₂(dba)₃ (0.05 mmol), and s-phos (0.1 mmol) were added slowly. After the system stabilized, the reaction was heated to 100-110 °C and reacted for 3 h. The reaction mixture was cooled to 25-30 °C, added water (100 mL) and toluene (100 mL), and then stirred and separated. The aqueous phase was extracted once with toluene (100 mL), then separated. The organic phases were combined and dried over 7 g of anhydrous sodium sulfate with stirring. After filtration, the organic phase was concentrated (-0.08 to 0.09 MPa, 55-60 °C) until no more liquid distilled. Then, 20 mL petroleum ether was added with stirring, cooled to 0-5 °C, and filtered to obtain Compound N-1 with a yield of 68%.
Elemental analysis: C₄₁H₂₈N₂O theoretical value: C, 87.21; H, 5.00; N, 4.96; O, 2.83; measured value: C, 87.15; H, 5.01; N, 4.99;
HRMS (ESI) m/z [M+H]⁺: theoretical value: 564.22; measured value: 565.31.

### Example 2

This example provides the deuterated compound N-5. The synthesis of deuterated compound N-5 specifically involves the following steps:

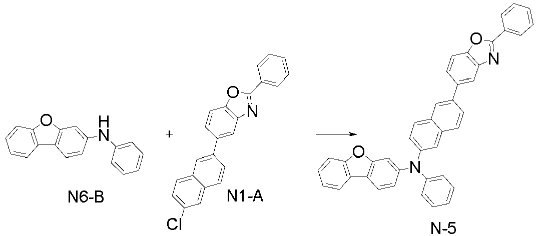

A three-necked reaction flask equipped with a mechanical stirrer, a thermometer, and a condenser tube was purged with nitrogen, then sequentially added Intermediate N6-B (10 mmol), Intermediate N1-A (10 mmol), and toluene (100 mL), heated to reflux for 0.5 h with removal of water, and cooled to 70-80 °C. Sodium tert-butoxide (15 mmol), Pd₂(dba)₃ (0.05 mmol), and s-phos (0.1 mmol) were added slowly. After the system stabilized, the reaction was heated to 100-110 °C and reacted for 3 h. The reaction mixture was cooled to 25-30 °C, added water (100 mL) and toluene (100 mL), then stirred and separated. The aqueous phase was extracted once with toluene (100 mL), then separated. The organic phases were combined and dried over 7 g of anhydrous sodium sulfate with stirring. After filtration, the organic phase was concentrated (-0.08 to 0.09 MPa, 55-60 °C) until no more liquid distilled. Then, 20 mL petroleum ether was added with stirring, cooled to 0-5 °C, and filtered to obtain Compound N-5 with a yield of 66%.
Elemental analysis: C₄₁H₂₆N₂O₂ theoretical value: C, 85.10; H, 4.53; N, 4.84; O, 5.53; measured value: C, 85.10; H, 4.53; N, 4.84; O, 5.53;
HRMS (ESI) m/z [M+H]⁺: theoretical value: 578.20; measured value: 579.25.

### Example 3

This example provides the deuterated compound N-16. The synthesis of deuterated compound N-16 specifically involves the following steps:

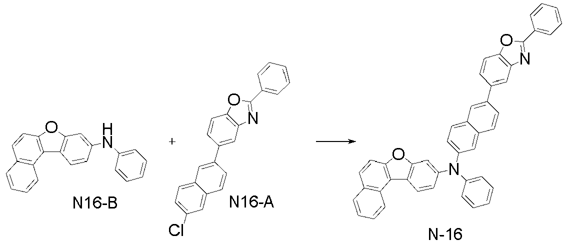

A three-necked reaction flask equipped with a mechanical stirrer, a thermometer, and a condenser tube was purged with nitrogen, then sequentially added Intermediate N16-B (10 mmol), Intermediate N16-A (10 mmol), and toluene (100 mL), heated to reflux for 0.5 h with removal of water, and cooled to 70-80 °C. Sodium tert-butoxide (15 mmol), Pd₂(dba)₃ (0.05 mmol), and s-phos (0.1 mmol) were added slowly. After the system stabilized, the reaction was heated to 100-110 °C and reacted for 3 h. The reaction mixture was cooled to 25-30 °C, added water (100 mL) and toluene (100 mL), and then stirred and separated. The aqueous phase was extracted once with toluene (100 mL), then separated. The organic phases were combined and dried over 7 g of anhydrous sodium sulfate with stirring. After filtration, the organic phase was concentrated (-0.08 to 0.09 MPa, 55-60 °C) until no more liquid distilled. Then, 20 mL petroleum ether was added with stirring, cooled to 0-5 °C, and filtered to obtain Compound N-16 with a yield of 61%.

Elemental analysis: C₅₁H₃₂N₂O₂ theoretical value: C, 85.97; H, 4.49; N, 4.46; O, 5.09; measured value: C, 85.94; H, 4.50; N, 4.48; HRMS (ESI) m/z [M+H]⁺: theoretical value: 628.22; measured value: 629.25.

### Example 4

This example provides the deuterated compound N-18. The synthesis of deuterated compound N-18 specifically involves the following steps:

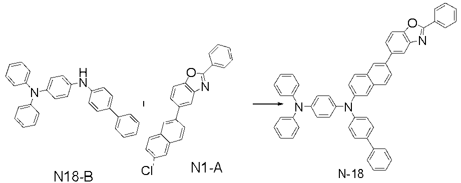

A three-necked reaction flask equipped with a mechanical stirrer, a thermometer, and a condenser tube was purged with nitrogen, then sequentially added Intermediate N18-B (10 mmol), Intermediate N1-A (10 mmol), and toluene (100 mL), heated to reflux for 0.5 h with removal of water, and cooled to 70-80 °C. Sodium tert-butoxide (15 mmol), Pd₂(dba)₃ (0.05 mmol), and s-phos (0.1 mmol) were added slowly. After the system stabilized, the reaction was heated to 100-110 °C and reacted for 3 h. The reaction mixture was cooled to 25-30 °C, added water (100 mL) and toluene (100 mL), and then stirred and separated. The aqueous phase was extracted once with toluene (100 mL), then separated. The organic phases were combined and dried over 7 g of anhydrous sodium sulfate with stirring. After filtration, the organic phase was concentrated (-0.08 to 0.09 MPa, 55-60 °C) until no more liquid distilled. Then, 20 mL petroleum ether was added with stirring, cooled to 0-5 °C, and filtered to obtain Compound N-18 with a yield of 61%.
Elemental analysis: C₅₃H₃₇N₃O theoretical value: C, 86.98; H, 5.10; N, 5.74; O, 2.19; measured value: C, 87.00; H, 5.11; N, 5.70;
HRMS (ESI) m/z [M+H]⁺: theoretical value: 731.29; measured value: 732.15.

### Example 5

This example provides the deuterated compound N-45. The synthesis of deuterated compound N-45 specifically involves the following steps:

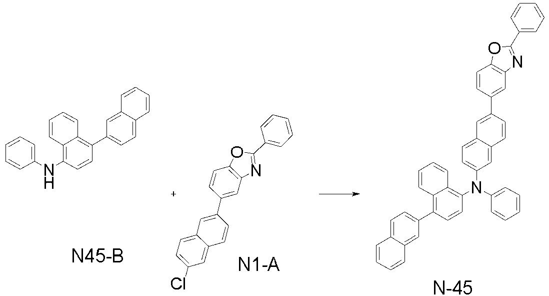

A three-necked reaction flask equipped with a mechanical stirrer, a thermometer, and a condenser tube was purged with nitrogen, then sequentially added Intermediate N45-B (10 mmol), Intermediate N1-A (10 mmol), and toluene (100 mL), heated to reflux for 0.5 h with removal of water, and cooled to 70-80 °C. Sodium tert-butoxide (15 mmol), Pd₂(dba)₃ (0.05 mmol), and s-phos (0.1 mmol) were added slowly. After the system stabilized, the reaction was heated to 100-110 °C and reacted for 3 h. The reaction mixture was cooled to 25-30 °C, added water (100 mL) and toluene (100 mL), and then stirred and separated. The aqueous phase was extracted once with toluene (100 mL), then separated. The organic phases were combined and dried over 7 g of anhydrous sodium sulfate with stirring. After filtration, the organic phase was concentrated (-0.08 to 0.09 MPa, 55-60 °C) until no more liquid distilled. Then, 20 mL petroleum ether was added with stirring, cooled to 0-5 °C, and filtered to obtain Compound N-45 with a yield of 65%.

Elemental analysis: C₄₉H₃₂N₂O theoretical value: C, 88.53; H, 4.85; N, 4.21; O, 2.41; measured value: C, 88.49; H, 4.86; N, 4.24; HRMS (ESI) m/z [M+H]⁺: theoretical value: 664.25; measured value: 665.19.

### Example 6

This example provides the deuterated compound N-130. The synthesis of deuterated compound N-130 specifically involves the following steps:

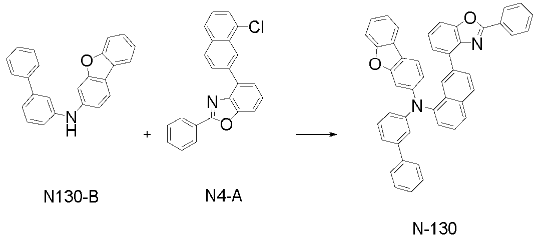

A three-necked reaction flask equipped with a mechanical stirrer, a thermometer, and a condenser tube was purged with nitrogen, then sequentially added Intermediate N130-B (10 mmol), Intermediate N4-A (10 mmol), and toluene (100 mL), heated to reflux for 0.5 h with removal of water, and cooled to 70-80 °C. Sodium tert-butoxide (15 mmol), Pd₂(dba)₃ (0.05 mmol), and s-phos (0.1 mmol) were added slowly. After the system stabilized, the reaction was heated to 100-110 °C and reacted for 3 h. The reaction mixture was cooled to 25-30 °C, added water (100 mL) and toluene (100 mL), and then stirred and separated. The aqueous phase was extracted once with toluene (100 mL), then separated. The organic phases were combined and dried over 7 g of anhydrous sodium sulfate with stirring. After filtration, the organic phase was concentrated (-0.08 to 0.09 MPa, 55-60 °C) until no more liquid distilled. Then, 20 mL petroleum ether was added with stirring, cooled to 0-5 °C, and filtered to obtain Compound N-130 with a yield of 65%.

Elemental analysis: C₄₇H₃₀N₂O₂ theoretical value: C, 86.22; H, 4.62; N, 4.28; O, 4.89; measured value: C, 86.20; H, 4.61; N, 4.31; HRMS (ESI) m/z [M+H]⁺: theoretical value: 654.23; measured value: 655.28

### Example 7

This example provides the deuterated compound M-1. The synthesis of deuterated compound M-1 specifically involves the following steps:

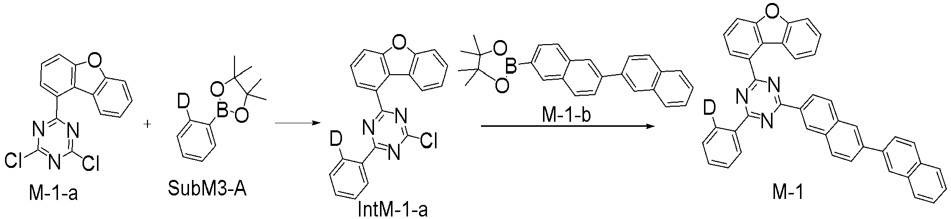

A three-necked reaction flask equipped with a mechanical stirrer, a thermometer, and a condenser tube was purged with nitrogen, then sequentially added SubM3-A (10 mmol), M-1-a (1.05 mmol), 1,4-dioxane (100 mL), water (30 mL), sodium carbonate (20 mmol), and Pd(PPh₃)₄ (0.05 mmol), heated to 70-80 °C and reacted for 3 h. The reaction mixture was cooled to 25-30 °C, added water (100 mL) and toluene (100 mL), and then stirred and separated. The aqueous phase was extracted once with toluene (100 mL), then separated. The organic phases were combined and dried over 7 g of anhydrous sodium sulfate with stirring. After filtration, the organic phase was concentrated (-0.08 to 0.09 MPa, 55-60 °C) until no more liquid distilled. Then, 50 mL of a mixed solvent of dichloromethane and petroleum ether was added with stirring, cooled to 0-5 °C, and filtered to obtain Intermediate IntM-1-a with a yield of 43%.

A three-necked reaction flask equipped with a mechanical stirrer, a thermometer, and a condenser tube was purged with nitrogen, then sequentially added Intermediate IntM-1-a (10 mmol), Intermediate 1-A (10 mmol), potassium carbonate (20 mmol), Pd(PPh₃)₄ (0.05 mmol), 1,4-dioxane (100 mL), and water (30 mL), stirred, heated to 70-75 °C and reacted for 3 h. The reaction mixture was cooled to 25-30 °C, added water (100 mL) and dichloromethane (100 mL), and then stirred and separated. The aqueous phase was extracted once with dichloromethane (100 mL), then separated. The organic phases were combined and dried over 7 g of anhydrous sodium sulfate with stirring. After filtration, the organic phase was concentrated (-0.08 to 0.09 MPa, 55-60 °C) until no more liquid distilled. Then, 20 mL petroleum ether was added with stirring, cooled to 0-5 °C, and filtered to obtain a crude product, which was recrystallized from toluene to obtain the product M-1 with a yield of 54%.

Elemental analysis: C₄₁H₂₄DN₃O theoretical value: C, 85.39; H, 4.54; N, 7.29; O, 2.77; measured value: C, 85.42; H, 4.55; N, 7.24; HRMS(ESI)m/z [M+H]+: theoretical value: 576.21; measured value: 577.32.

### Example 8

This example provides the deuterated compound M-8. The synthesis of deuterated compound M-8 specifically involves the following steps:

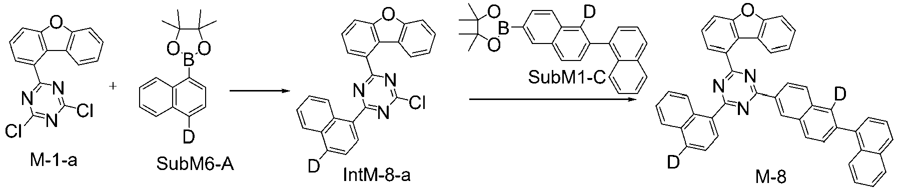

The preparation of M-8 was carried out in the same manner as that of M-1, except that SubM6-A was used instead of SubM3-A and SubM1-C instead of M-1-b, to obtain the product M-8 with a yield of 56%.

Elemental analysis: C₄₅H₂₅D₂N₃O theoretical value: C, 86.10; H, 4.66; N, 6.69; O, 2.55; measured value: C, 86.15; H, 4.67; N, 6.62; HRMS(ESI)m/z [M+H]+: theoretical value: 627.23; measured value: 628.43.

### Example 9

This example provides the deuterated compound M-15. The synthesis of deuterated compound M-15 specifically involves the following steps:

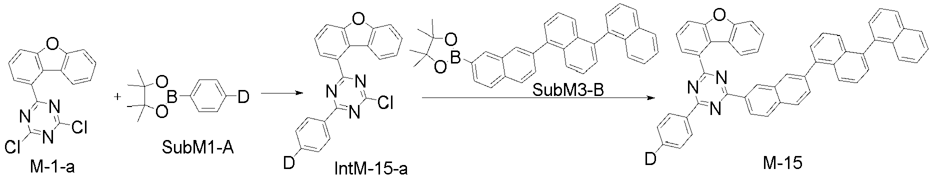

The preparation of M-15 was carried out in the same manner as that of M-1, except that SubM1-A was used instead of SubM3-A and SubM3-B instead of M-1-b, to obtain the product M-15 with a yield of 56%.

Elemental analysis: C₅₁H₃₀DN₃O theoretical value: C, 87.16; H, 4.59; N, 5.98; O, 2.28; measured value: C, 87.18; H, 4.61; N, 5.94; HRMS(ESI)m/z [M+H]+: theoretical value: 702.25; measured value: 703.25.

### Example 10

This example provides the deuterated compound M-16. The synthesis of deuterated compound M-16 specifically involves the following steps:

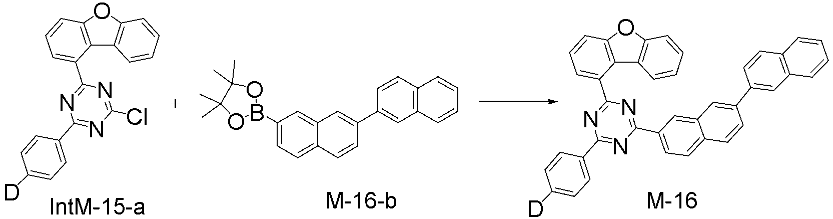

A three-necked reaction flask equipped with a mechanical stirrer, a thermometer, and a condenser tube was purged with nitrogen, then sequentially added Intermediate IntM-15-a (10 mmol), starting material M-16-b (10 mmol), potassium carbonate (20 mmol), Pd(PPh₃)₄ (0.05 mmol), 1,4-dioxane (100 mL), and water (30 mL), stirred, heated to 70-75 °C and reacted for 3 h. The reaction mixture was cooled to 25-30 °C, added water (100 mL) and dichloromethane (100 mL), and then stirred and separated. The aqueous phase was extracted once with dichloromethane (100 mL), then separated. The organic phases were combined and dried over 7 g of anhydrous sodium sulfate with stirring. After filtration, the organic phase was concentrated (-0.08 to 0.09 MPa, 55-60 °C) until no more liquid distilled. Then, 20 mL petroleum ether was added with stirring, cooled to 0-5 °C, and filtered to obtain a crude product, which was recrystallized from toluene to obtain the product M-16 with a yield of 62%.

Elemental analysis: C₄₁H₂₄DN₃O theoretical value: C, 85.39; H, 4.54; N, 7.29; O, 2.77; measured value: C, 85.43; H, 4.55; N, 7.23; HRMS(ESI)m/z [M+H]+: theoretical value: 576.21; measured value: 576.46.

### Example 11

This example provides the deuterated compound M-19. The synthesis of deuterated compound M-19 specifically involves the following steps:

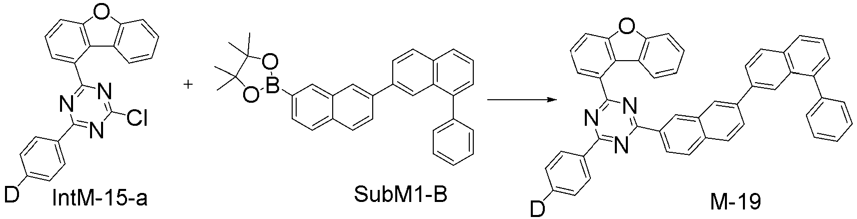

The preparation of M-19 was carried out in the same manner as that of M-16, except that SubM1-B was used instead of M-16-b, to obtain the product M-19 with a yield of 57%.

Elemental analysis: C₄₇H₂₈DN₃O theoretical value: C, 86.48; H, 4.63; N, 6.44; O, 2.45; measured value: C, 86.49; H, 4.62; N, 6.45; HRMS(ESI)m/z [M+H]+: theoretical value: 652.24; measured value: 653.23,

### Example 12

This example provides the deuterated compound M-24. The synthesis of deuterated compound M-24 specifically involves the following steps:

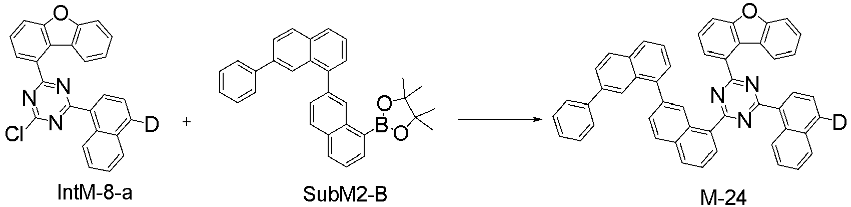

The preparation of M-24 was carried out in the same manner as that of M-16, except that IntM-8-a was used instead of IntM-15-a and SubM2-B instead of M-16-b, to obtain the product M-24 with a yield of 52%.

Elemental analysis: C₅₁H₃₀DN₃O theoretical value: C, 87.16; H, 4.59; N, 5.98; O, 2.28, measured value: HRMS(ESI)m/z [M+H]+: C, 87.21; H, 4.60; N, 5.92; theoretical value: 702.25; measured value: 703.19.

### Example 13

This example provides the deuterated compound M-27. The synthesis of deuterated compound M-27 specifically involves the following steps:

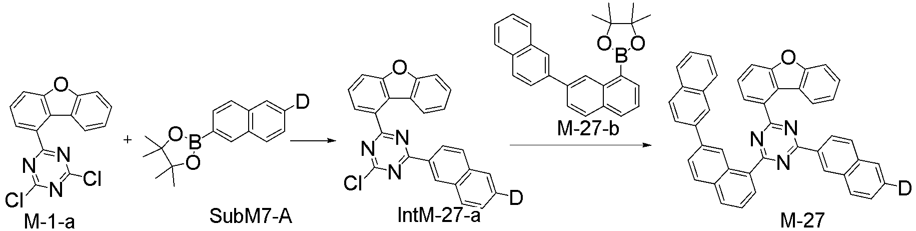

The preparation of M-27 was carried out in the same manner as that of M-1, except that SubM7-A was used instead of SubM3-A and M-27-b instead of M-1-b, to obtain the product M-27 with a yield of 61%.

Elemental analysis: C₄₅H₂₆DN₃O theoretical value: C, 86.24; H, 4.50; N, 6.70; O, 2.55; measured value: C, 86.28; H, 4.52; N, 6.64; HRMS(ESI)m/z [M+H]+: theoretical value: 626.22; measured value: 627.25.

### Example 14

This example provides the deuterated compound M-31. The synthesis of deuterated compound M-31 specifically involves the following steps:

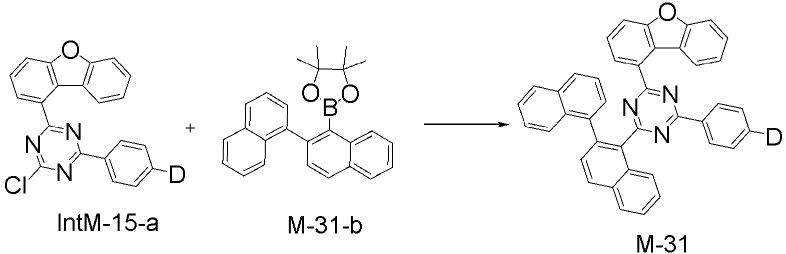

The preparation of M-31 was carried out in the same manner as that of M-16, except that M-31-b was used instead of M-16-b, to obtain the product M-31 with a yield of 55%.

Elemental analysis: C₄₁H₂₄DN₃O theoretical value: C, 85.39; H, 4.54; N, 7.29; O, 2.77; measured value: C, 85.45; H, 4.56; N, 7.21;

HRMS(ESI)m/z [M+H]+: theoretical value: 576.21; measured value: 577.36.

### Example 15

This example provides the deuterated compound M-53. The synthesis of deuterated compound M-53 specifically involves the following steps:

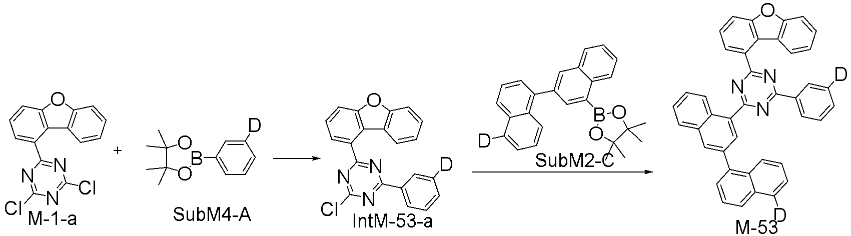

The preparation of M-53 was carried out in the same manner as that of M-1, except that SubM4-A was used instead of SubM3-A and SubM2-C instead of M-1-b, to obtain the product M-53 with a yield of 58%.
Elemental analysis: C₄₁H₂₃D₂N₃O theoretical value: C, 85.25; H, 4.71; N, 7.27; O, 2.77, measured value: C, 85.28; H, 4.72; N, 7.22;
HRMS(ESI)m/z [M+H]+: theoretical value: 577.21; measured value: 578.40.

### Example 16

This example provides the deuterated compound M-57. The synthesis of deuterated compound M-57 specifically involves the following steps:

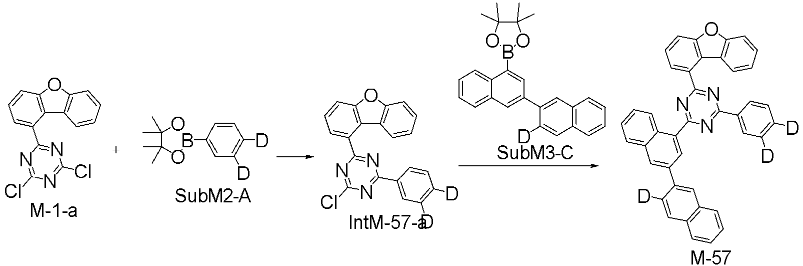

The preparation of M-57 was carried out in the same manner as that of M-1, except that SubM2-A was used instead of SubM3-A and SubM3-C instead of M-1-b, to obtain the product M-57 with a yield of 54%.
Elemental analysis: C₄₁H₂₂D₃N₃O theoretical value: C, 85.10; H, 4.88; N, 7.26; O, 2.76, measured value: C, 85.14; H, 4.90; N, 7.22;
HRMS(ESI)m/z [M+H]+: theoretical value: 578.22; measured value: 579.40.

### Example 17

This example provides the deuterated compound M-68. The synthesis of deuterated compound M-68 specifically involves the following steps:

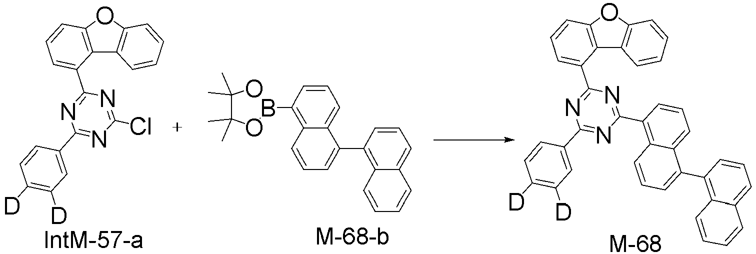

The preparation of M-68 was carried out in the same manner as that of M-16, except that IntM-57-a was used instead of IntM-15-a and M-68-b instead of M-16-b, to obtain the product M-68 with a yield of 60%.

Elemental analysis: C₄₁H₂₃D₂N₃O theoretical value: C, 85.25; H, 4.71; N, 7.27; O, 2.77, measured value: C, 85.28; H, 4.72; N, 7.22; HRMS(ESI)m/z [M+H]+: theoretical value: 577.21; measured value: 578.56.

### Example 18

This example provides the deuterated compound M-72. The synthesis of deuterated compound M-72 specifically involves the following steps:

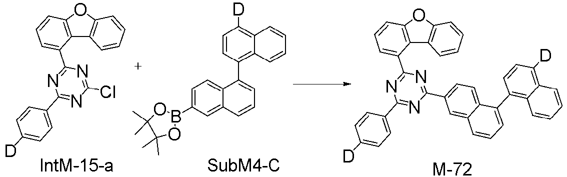

The preparation of M-72 was carried out in the same manner as that of M-16, except that SubM4-C was used instead of M-16-b, to obtain the product M-72 with a yield of 57%.
Elemental analysis: C₄₁H₂₃D₂N₃O theoretical value: C, 85.25; H, 4.71; N, 7.27; O, 2.77, measured value: C, 85.30; H, 4.72; N, 7.21;
HRMS(ESI)m/z [M+H]+: theoretical value: 577.21; measured value: 578.54.

### Example 19

This example provides the deuterated compound M-81. The synthesis of deuterated compound M-81 specifically involves the following steps:

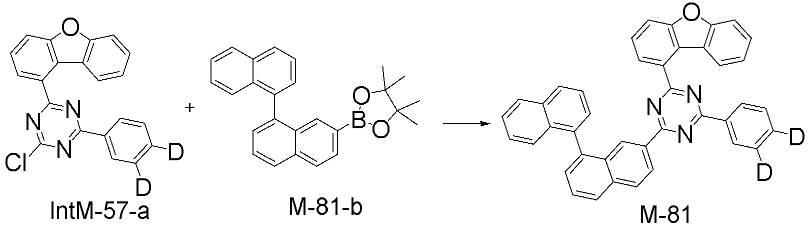

The preparation of M-81 was carried out in the same manner as that of M-16, except that IntM-57-a was used instead of IntM-15-a and M-81-b instead of M-16-b, to obtain the product M-81 with a yield of 61%.

Elemental analysis: C₄₁H₂₃D₂N₃O theoretical value: C, 85.25; H, 4.71; N, 7.27; O, 2.77; measured value: C, 85.28; H, 4.72; N, 7.21; HRMS(ESI)m/z [M+H]+: theoretical value: 577.21; measured value: 578.18.

### Example 20

This example provides the deuterated compound M-86. The synthesis of deuterated compound M-86 specifically involves the following steps:

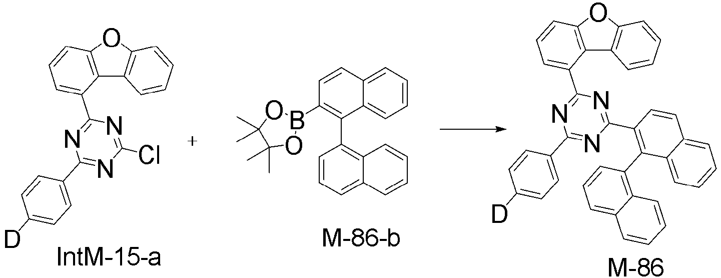

The preparation of M-86 was carried out in the same manner as that of M-16, except that M-86-b was used instead of M-16-b, to obtain the product M-86, with a yield of 58%.
Elemental analysis: C₄₁H₂₄DN₃O theoretical value: C, 85.39; H, 4.54; N, 7.29; O, 2.77, measured value: C, 85.45; H, 4.55; N, 7.21;
HRMS(ESI)m/z [M+H]+: theoretical value: 576.21; measured value: 577.32.

### Example 21

This example provides the deuterated compound M-98. The synthesis of deuterated compound M-98 specifically involves the following steps:

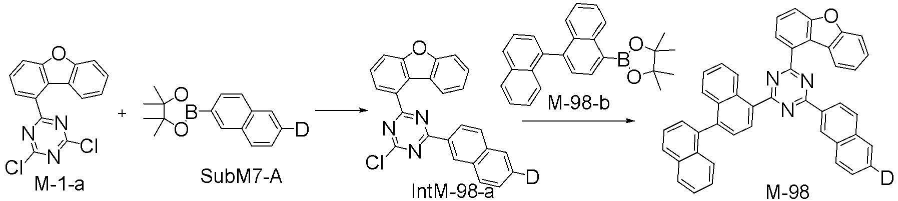

The preparation of M-98 was carried out in the same manner as that of M-1, except that SubM7-A was used instead of SubM3-A and M-98-b instead of M-1-b, to obtain the product M-98, with a yield of 64%.

Elemental analysis: C₄₅H₂₆DN₃O theoretical value: C, 86.24; H, 4.50; N, 6.70; O, 2.55; measured value: C, 86.27; H, 4.52; N, 6.64; HRMS(ESI)m/z [M+H]+: theoretical value: 626.22; measured value: 627.35.

### Example 22

This example provides the deuterated compound M-99. The synthesis of deuterated compound M-99 specifically involves the following steps:

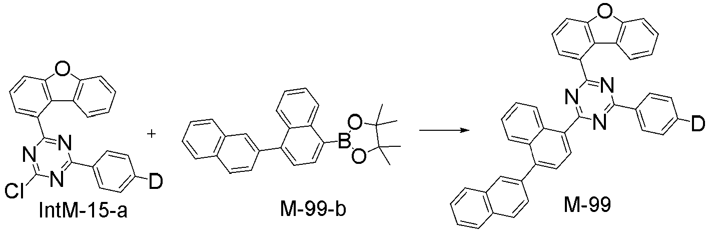

The preparation of M-99 was carried out in the same manner as that of M-16, except that M-99-b was used instead of M-16-b, to obtain the product M-99, with a yield of 52%.
Elemental analysis: C₄₁H₂₄DN₃O theoretical value: C, 85.39; H, 4.54; N, 7.29; O, 2.77; measured value: C, 85.43; H, 4.55; N, 7.24;
HRMS(ESI)m/z [M+H]+: theoretical value: 576.21; measured value: 577.64.

### Device Examples And Comparative Examples

Some of materials used in the fabrication of organic electroluminescent devices in Device Examples and Comparative Examples are as follows:

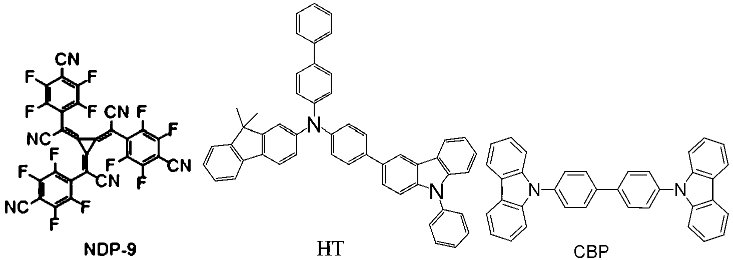

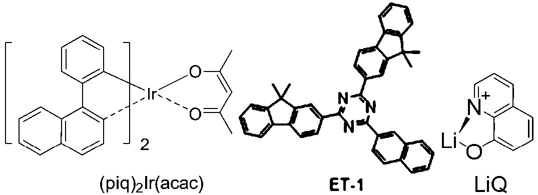

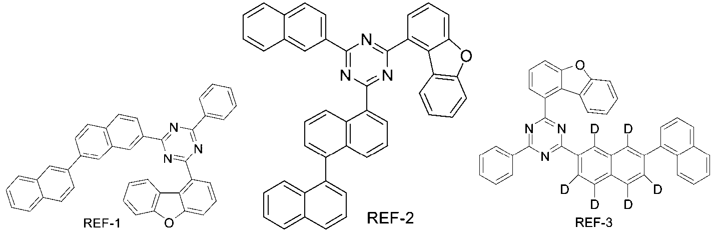

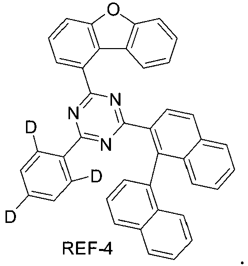

The organic electroluminescent devices in Device Examples and Comparative Examples have a similar structure (as shown in Fig. 1), comprising an anode 2, a hole injection layer 3, a hole transport layer 4, a light-emitting layer 5, an electron transport layer 6, an electron injection layer 7, and a cathode 8 which are sequentially stacked on a substrate 1. The device structure is: substrate + anode (indium tin oxide (ITO)-coated glass substrate) / hole injection layer (HIL) / hole transport layer (HTL) / light-emitting layer (EML) / electron transport layer (ETL) / electron injection layer (EIL) / cathode (Al).

The preparation of organic electroluminescent devices in Device Examples and Comparative Examples includes the following steps:

### 1)Substrate cleaning:

A glass substrate coated with transparent ITO was ultrasonically treated in an aqueous cleaning solution (composition and concentration of the aqueous cleaning solution: glycol-based solvent ≤ 10 wt%, and triethanolamine ≤ 1 wt%), then rinsed with deionized water, ultrasonically degreased in a mixed solvent of acetone and ethanol (volume ratio: 1:1), baked under clean conditions until completely dry, and cleaned with ultraviolet light and ozone.

### 2) Organic layer preparation:

The ITO transparent substrate was transferred to a vacuum evaporation apparatus and evacuated to 1×10⁻⁶ to 2×10⁻⁴ Pa. The hole injection layer (HIL) / hole transport layer (HTL)/ light-emitting layer (EML)/electron transport layer (ETL)/electron injection layer (EIL)/cathode (Al) were sequentially deposited on the anode by evaporation.

In which:
the hole injection layer (HIL) is composed of a mixture of NDP-9 and HT with a mass ratio of 3:97, which is deposited by co-evaporation; and the material and thickness are as listed in Table 1;
the material and thickness of the hole transport layer (HTL) are as listed in Table 1;
the light-emitting layer (EML) is deposited by co-evaporation; and the material and thickness are specifically as listed in Table 1;
the material and thickness of the electron transport layer (ETL) are specifically as listed in Table 1, and this layer is deposited by co-evaporation;
the material of the electron injection layer (EIL) is LiQ. The material and thickness are as listed in Table 1; and
the thicknesses and materials for those layers are as listed in Table 1.

**The materials and thicknesses used in Device Examples and Device Comparative Examples are specifically shown in Table 1 below:**

**Table 1**

| No. | HIL Thickness | HTL Thickness | EML Thickness | ETL Thickness | EIL Thickness | Cathode Thickness |
|---|---|---|---|---|---|---|
| Example 1 | NDP-9: HT (mass ratio: 3:97) 10 nm | HT 80 nm | N-1:M-1: (piq)₂Ir(aca c) (mass ratio = 47.5:47.5:5) 38 nm | ET-1:LiQ (mass ratio 1:1) 30 nm | LiQ 1 nm | Al 80 nm |
| Example 2 | NDP-9: HT (mass ratio: 3:97) 10 nm | HT 80 nm | N-1:M-8: (piq)₂Ir(aca c) (mass ratio = 47.5:47.5:5) 38 nm | ET-1:LiQ (mass ratio 1:1) 30 nm | LiQ 1 nm | Al 80 nm |
| Example 3 | NDP-9: HT (mass ratio: 3:97) 10 nm | HT 80 nm | N-1:M-15:(piq)₂Ir(a cac) (mass ratio = 47.5:47.5:5) 38 nm | ET-1:LiQ (mass ratio 1:1) 30 nm | LiQ 1 nm | Al 80 nm |
| Example 4 | NDP-9: HT (mass ratio: 3:97) 10 nm | HT 80 nm | N-1:M-16:(piq)₂Ir(a cac)(mass ratio = 47.5:47.5:5) 38 nm | ET-1:LiQ (mass ratio 1:1) 30 nm | LiQ 1 nm | Al 80 nm |
| Example 5 | NDP-9: HT (mass ratio: 3:97) 10 nm | HT 80 nm | N-1:M-19:(piq)₂Ir(a cac)(mass ratio = 47.5:47.5:5) 38 nm | ET-1:LiQ (mass ratio 1:1) 30 nm | LiQ 1 nm | Al 80 nm |
| Example 6 | NDP-9: HT (mass ratio: 3:97) 10 nm | HT 80 nm | N-5:M-24:(piq)₂Ir(a cac)(mass ratio = 47.5:47.5:5) 38 nm | ET-1:LiQ (mass ratio 1:1) 30 nm | LiQ 1 nm | Al 80 nm |
| Example 7 | NDP-9: HT (mass | HT 80 nm | N-5:M-27:(piq)₂Ir(a | ET-1:LiQ (mass | LiQ 1 nm | Al 80 nm |
| | ratio: 3:97) 10 nm | | cac)(mass ratio = 47.5:47.5:5) 38 nm | ratio 1:1) 30 nm | | |
| Example 8 | NDP-9: HT (mass ratio: 3:97) 10 nm | HT 80 nm | N-16: M-31:(piq)₂Ir(a cac) (mass ratio = 47.5:47.5:5) 38 nm | ET-1:LiQ (mass ratio 1:1) 30 nm | LiQ 1 nm | Al 80 nm |
| Example 9 | NDP-9: HT (mass ratio: 3:97) 10 nm | HT 80 nm | N-16: M-53:(piq)₂Ir(a cac) (mass ratio = 47.5:47.5:5) 38 nm | ET-1:LiQ (mass ratio 1:1) 30 nm | LiQ 1 nm | Al 80 nm |
| Example 10 | NDP-9: HT (mass ratio: 3:97) 10 nm | HT 80 nm | N-18:M-57:(piq)₂Ir(a cac) (mass ratio = 47.5:47.5:5) 38 nm | ET-1:LiQ (mass ratio 1:1) 30 nm | LiQ 1 nm | Al 80 nm |
| Example 11 | NDP-9: HT (mass ratio: 3:97) 10 nm | HT 80 nm | N-18:M-68:(piq)₂Ir(a cac) (mass ratio = 47.5:47.5:5) 38 nm | ET-1:LiQ (mass ratio 1:1) 30 nm | LiQ 1 nm | Al 80 nm |
| Example 12 | NDP-9: HT (mass ratio: 3:97) 10 nm | HT 80 nm | N-45:M-72:(piq)₂Ir(a cac) (mass ratio = 47.5:47.5:5) 38 nm | ET:LiQ (mass ratio 1:1) 30 nm | LiQ 1 nm | Al 80 nm |
| Example 13 | NDP-9: HT (mass ratio: 3:97) 10 nm | HT 80 nm | N-45:M-81:(piq)₂Ir(a cac) (mass ratio = 47.5:47.5:5) 38 nm | ET-1:LiQ (mass ratio 1:1) 30 nm | LiQ 1 nm | Al 80 nm |
| Example 14 | NDP-9: HT (mass ratio: 3:97) 10 nm | HT 80 nm | N-130:M-86:(piq)₂Ir(a cac) (mass ratio = 47.5:47.5:5) 38 nm | ET-1:LiQ (mass ratio 1:1) 30 nm | LiQ 1 nm | Al 80 nm |
| Example 15 | NDP-9: HT (mass ratio: 3:97) 10 nm | HT 80 nm | N-130:M-98:(piq)₂Ir(a cac) (mass ratio = 47.5:47.5:5) 38 nm | ET-1:LiQ (mass ratio 1:1) 30 nm | LiQ 1 nm | Al 80 nm |
| Example 16 | NDP-9: HT (mass ratio: 3:97) 10 nm | HT 80 nm | N-130:M-99:(piq)₂Ir(a cac) (mass ratio = 47.5:47.5:5) 38 nm | ET-1:LiQ (mass ratio 1:1) 30 nm | LiQ 1 nm | Al 80 nm |
| Example 17 | NDP-9: HT (mass ratio: 3:97) 10 nm | HT 80 nm | M-1: (piq)₂Ir(aca c) (mass ratio = 95:5) 38 nm | ET-1:LiQ (mass ratio 1:1) 30 nm | LiQ 1 nm | Al 80 nm |
| Example 18 | NDP-9: HT (mass ratio: 3:97) 10 nm | HT 80 nm | M-8: (piq)₂Ir(aca c) (mass ratio = 95:5) 38 nm | ET-1:LiQ (mass ratio 1:1) 30 nm | LiQ 1 nm | Al 80 nm |
| Example 19 | NDP-9: HT (mass ratio: 3:97) 10 nm | HT 80 nm | M-15: (piq)₂Ir(aca c) (mass ratio = 95:5) 38 nm | ET-1:LiQ (mass ratio 1:1) 30 nm | LiQ 1 nm | Al 80 nm |
| Example 20 | NDP-9: HT (mass ratio: 3:97) 10 nm | HT 80 nm | M-16: (piq)₂Ir(aca c) (mass ratio = 95:5) 38 nm | ET-1:LiQ (mass ratio 1:1) 30 nm | LiQ 1 nm | Al 80 nm |
| Example 21 | NDP-9: HT (mass ratio: 3:97) 10 nm | HT 80 nm | M-24: (piq)₂Ir(aca c) (mass ratio = 95:5) 38 nm | ET-1:LiQ (mass ratio 1:1) 30 nm | LiQ 1 nm | Al 80 nm |
| Example 22 | NDP-9: HT (mass ratio: 3:97) 10 nm | HT 80 nm | M-27: (piq)₂Ir(aca c) (mass ratio = 95:5) 38 nm | ET-1:LiQ (mass ratio 1:1) 30 nm | LiQ 1 nm | Al 80 nm |
| Example 23 | NDP-9: HT (mass ratio: 3:97) | HT 80 nm | M-31: (piq)₂Ir(aca c) (mass ratio = 95:5) | ET-1:LiQ (mass ratio 1:1) 30 nm | LiQ 1 nm | Al 80 nm |
| | 10 nm | | 38 nm | | | |
| Example 24 | NDP-9: HT (mass ratio: 3:97) 10 nm | HT 80 nm | M-53: (piq)₂Ir(aca c) (mass ratio = 95:5) 38 nm | ET-1:LiQ (mass ratio 1:1) 30 nm | LiQ 1 nm | Al 80 nm |
| Example 25 | NDP-9: HT (mass ratio: 3:97) 10 nm | HT 80 nm | M-68: (piq)₂Ir(aca c) (mass ratio = 95:5) 38 nm | ET-1:LiQ (mass ratio 1:1) 30 nm | LiQ 1 nm | Al 80 nm |
| Example 26 | NDP-9: HT (mass ratio: 3:97) 10 nm | HT 80 nm | M-1:CBP:(piq) ₂Ir(acac) (mass ratio 47.5:47.5:5) 38 nm | ET-1:LiQ (mass ratio 1:1) 30 nm | LiQ 1 nm | Al 80 nm |
| Comparative Example 1 | NDP-9: HT (mass ratio: 3:97) 10 nm | HT 80 nm | CBP:(piq)2I r(acac) (mass ratio = 95:5) 38 nm | ET-1:LiQ (mass ratio 1:1) 30 nm | LiQ 1 nm | Al 80 nm |
| Comparative Example 2 | NDP-9: HT (mass ratio: 3:97) 10 nm | HT 80 nm | REF-1: (piq)₂Ir(aca c) (mass ratio = 95:5) 38 nm | ET-1:LiQ (mass ratio 1:1) 30 nm | LiQ 1 nm | Al 80 nm |
| Comparative Example 3 | NDP-9: HT (mass ratio: 3:97) 10 nm | HT 80 nm | REF-2: (piq)₂Ir(aca c) (mass ratio = 95:5) 38 nm | ET-1:LiQ (mass ratio 1:1) 30 nm | LiQ 1 nm | Al 80 nm |
| Comparative Example 4 | NDP-9: HT (mass ratio: 3:97) 10 nm | HT 80 nm | REF-3: (piq)₂Ir(aca c) (mass ratio = 95:5) 38 nm | ET-1:LiQ (mass ratio 1:1) 30 nm | LiQ 1 nm | Al 80 nm |
| Comparative Example 5 | NDP-9: HT (mass ratio: 3:97) 10 nm | HT 80 nm | REF-4: (piq)₂Ir(aca c) (mass ratio = 95:5) 38 nm | ET-1:LiQ (mass ratio 1:1) 30 nm | LiQ 1 nm | Al 80 nm |
| Comparative Example 6 | NDP-9: HT (mass ratio: | HT 80 nm | N-1:REF-1:(piq)₂Ir(ac ac) (mass | ET-1:LiQ (mass ratio 1:1) | LiQ 1 nm | Al 80 nm |
| | 3:97) 10 nm | | ratio = 47.5:47.5:5) 38 nm | 30 nm | | |
| Comparative Example 7 | NDP-9: HT (mass ratio: 3:97) 10 nm | HT 80 nm | N-130:REF-2:(piq)₂Ir(ac ac) (mass ratio = 47.5:47.5:5) 38 nm | ET-1:LiQ (mass ratio 1:1) 30 nm | LiQ 1 nm | Al 80 nm |
| Comparative Example 8 | NDP-9: HT (mass ratio: 3:97) 10 nm | HT 80 nm | N-1:REF-3:(piq)₂Ir(ac ac) (mass ratio = 47.5:47.5:5) 38 nm | ET-1:LiQ (mass ratio 1:1) 30 nm | LiQ 1 nm | Al 80 nm |
| Comparative Example 9 | NDP-9: HT (mass ratio: 3:97) 10 nm | HT 80 nm | N-130:REF-4:(piq)₂Ir(ac ac) (mass ratio = 47.5:47.5:5) 38 nm | ET:LiQ (mass ratio 1:1) 30 nm | LiQ 1 nm | Al 80 nm |

In Table 1, the examples are Device Examples, and the comparative examples are Device Comparative Examples.

### Test Examples

The organic electroluminescent devices obtained from Device Examples 1 to 26 and Comparative Examples 1 to 9 are tested.

Instruments: The characteristics of the devices, such as current, voltage, luminance, and emission spectra, were measured simultaneously using a PR 650 spectroradiometer and a Keithley 2400 Source Meter system.

Test conditions: Photoelectric properties: measured at a current density of 10 mA/cm².

Lifetime testing: conducted at a current density of 50 mA/cm², with the time recorded (in hours) when the luminance decreased to95% of the initial one.

The test results for the performance of devices are shown in Table 2:

**Table 2**

| Item | Driving Voltage (V) | Current efficiency (Cd/A) | Lifetime T95 (hrs) |
|---|---|---|---|
| Example 1 | 3.20 | 34.25 | 345.4 |
| Example 2 | 3.19 | 35.11 | 352.6 |
| Example 3 | 3.24 | 32.58 | 336.7 |
| Example 4 | 3.21 | 34.29 | 346.4 |
| Example 5 | 3.25 | 32.96 | 339.5 |
| Example 6 | 3.27 | 32.22 | 335.2 |
| Example 7 | 3.21 | 33.59 | 340.4 |
| Example 8 | 3.20 | 34.23 | 342.8 |
| Example 9 | 3.19 | 34.15 | 334.7 |
| Example 10 | 3.21 | 35.32 | 311.4 |
| Example 11 | 3.22 | 33.57 | 343.8 |
| Example 12 | 3.21 | 35.21 | 341.4 |
| Example 13 | 3.22 | 31.57 | 338.3 |
| Example 14 | 3.21 | 32.65 | 354.5 |
| Example 15 | 3.22 | 32.41 | 340.4 |
| Example 16 | 3.19 | 34.22 | 355.1 |
| Example 17 | 3.74 | 26.15 | 178.6 |
| Example 18 | 3.74 | 27.55 | 162.1 |
| Example 19 | 3.78 | 24.36 | 142.4 |
| Example 20 | 3.74 | 26.52 | 183.2 |
| Example 21 | 3.80 | 23.15 | 150.7 |
| Example 22 | 3.76 | 24.16 | 162.5 |
| Example 23 | 3.72 | 25.34 | 176.7 |
| Example 24 | 3.74 | 26.58 | 159.8 |
| Example 25 | 3.72 | 26.35 | 166.2 |
| Example 26 | 3.84 | 24.15 | 185.7 |
| Comparative Example 1 | 4.8 | 5 | 5 |
| Comparative Example 2 | 3.72 | 25.11 | 140.2 |
| Comparative Example 3 | 3.75 | 23.54 | 132.4 |
| Comparative Example 4 | 3.76 | 26.32 | 102.5 |
| Comparative Example 5 | 3.74 | 25.71 | 110.7 |
| Comparative Example 6 | 3.32 | 30.59 | 271.4 |
| Comparative Example 7 | 3.29 | 28.14 | 276.7 |
| Comparative Example 8 | 3.25 | 33.56 | 286.8 |
| Comparative Example 9 | 3.25 | 32.25 | 291.3 |

In Table 2, the examples are Device Examples and the comparative examples are Device Comparative Examples.

As seen in Table 2:
the comparison of the data corresponding to the examples and comparative examples in Table 2 reveals that the deuterated compound provided in this application exhibits significantly superior performance compared to those disclosed in the prior art, such as CBP and REF-1 to REF-4, enabling devices which are made therefrom to achieve lower driving voltages.

From those above, it is evident that the deuterated compound having the structure of Formula (1) and the compound having the structure of Formula (2) as provided herein can work synergistically to significantly enhance carrier injection efficiency, reduce interlayer energy level differences, and balance electron and hole transport rates, thereby effectively improving the efficiency and extending the lifetime of organic electroluminescent diodes. These materials are suitable not only as light-emitting host materials (particularly red-emitting host materials), hole transport materials, and electron-blocking materials, but also as electron transport materials and hole-blocking materials, resulting in substantially improved efficiency and prolonged lifetime of devices. The combination of such compounds can also be applied in the field of organic electroluminescent displays. Specifically, in organic electroluminescent displays, this combination may serve as hole injection or hole transport materials, or as light-emitting host material or light-emitting materials in fluorescent devices. In summary, the present application provides a novel organic electroluminescent material with closely matched HOMO, LUMO, and ET1 numeric values, which can lower driving voltage, improve luminance efficiency, and extend service life for devices.

It should be noted that the above examples are merely illustrative and are not intended to limit the scope of the embodiments. Based on the teachings herein, various modifications or alterations may be made by those of ordinary skill in the art. While it is not necessary and also unable to illustrate exhaustively, all such obvious variations or modifications derived therefrom remain within the protective scope of the present invention.

## Claims

1. A deuterated compound having a structure shown in Formula (1) below: 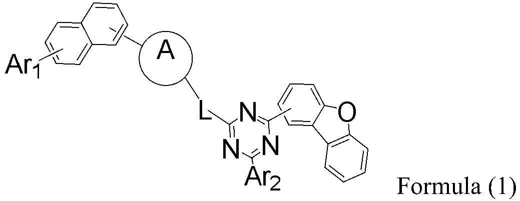
in the Formula (1), Ar₁ is selected from hydrogen, deuterium, halogen, cyano, a substituted or unsubstituted C6-C30 aryl, and a substituted or unsubstituted C3-C30 heteroaryl;
Ar₂ is selected from a substituted or unsubstituted C6-C30 aryl, and a substituted or unsubstituted C3-C30 heteroaryl;
Ring A is naphthylene, wherein at least one hydrogen on Ring A or Ar₂ is substituted with deuterium;
L is selected from a linking bond, a substituted or unsubstituted C6-C30 arylene, and a substituted or unsubstituted C3-C30 heteroarylene; and
the substituents on the substituted C6-C30 arylene, substituted C3-C30 heteroarylene, substituted C6-C30 aryl, and substituted C3-C30 heteroaryl are selected from one or two of a combination of deuterium, halogen, cyano, C1-C6 alkyl, C3-C30 cycloalkyl, C6-C30 aryl, and C3-C30 heteroaryl.

2. The deuterated compound according to claim 1, wherein Ar₁ is selected from hydrogen, deuterium, a substituted or unsubstituted C6-C20 aryl, and a substituted or unsubstituted C3-C20 heteroaryl; and
Ar₂ is selected from a substituted or unsubstituted C6-C20 aryl, and a substituted or unsubstituted C3-C20 heteroaryl;
the substituents on the substituted C6-C20 aryl, and substituted C3-C20 heteroaryl are selected from one or two of a combination of deuterium, halogen, cyano, C1-C6 alkyl, C3-C30 cycloalkyl, C6-C30 aryl, and C3-C30 heteroaryl; and
optionally, at least one hydrogen on Ring A is substituted with deuterium, and at most 5 hydrogens are substituted with deuterium.

3. The deuterated compound according to claim 1 or 2, wherein the Formula (1) is selected from one of structures below: 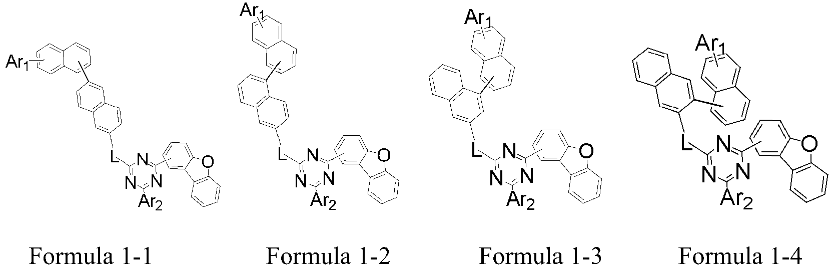 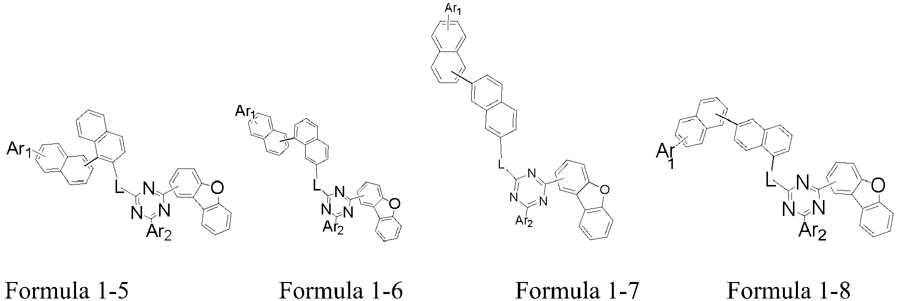 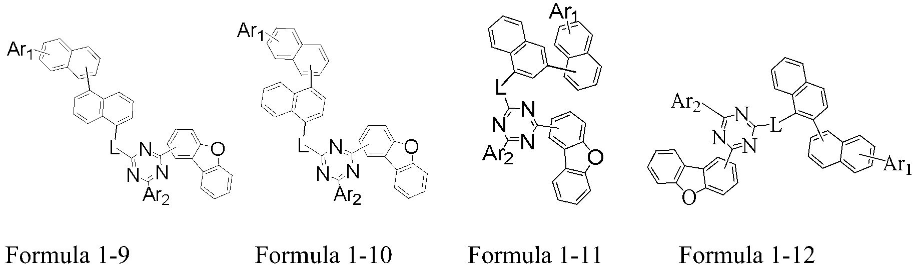 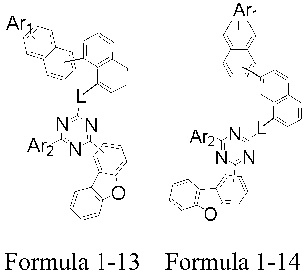 wherein Ar₁, Ar₂, and L are as defined in claim 1.

4. The deuterated compound according to any one of claims 1 to 3, wherein Ar₁ is selected from hydrogen, deuterium, and a substituted or unsubstituted B group,
Ar₂ is selected from a substituted or unsubstituted C group,
wherein the B group is selected from phenyl, naphthyl, biphenyl, terphenyl, carbazolyl, and dimethylfluorenyl;
the C group is selected from phenyl, naphthyl, biphenyl, terphenyl, phenanthrenyl, fluoranthenyl, triphenylene, dimethylfluorenyl, diphenylfluorenyl, spiro-bifluorenyl, benzodimethylfluorenyl, benzodiphenylfluorenyl, benzospirobifluorenyl, benzofuranyl, dibenzofuranyl, benzothienyl, dibenzothienyl, carbazolyl, benzocarbazolyl, and dibenzocarbazolyl; and
the substituents on the substituted B group and substituted C group are selected from one or two of a combination of deuterium, halogen, cyano, C1-C6 alkyl, C3-C30 cycloalkyl, C6-C30 aryl, and C3-C30 heteroaryl.

5. The deuterated compound according to any one of claims 1 to 4, wherein Ar₂ is selected from a substituted or unsubstituted C group;
wherein the C group is selected from phenyl, naphthyl, biphenyl, terphenyl, phenanthrenyl, fluoranthenyl, triphenylene, dimethylfluorenyl, diphenylfluorenyl, spiro-bifluorenyl, benzodimethylfluorenyl, benzodiphenylfluorenyl, benzospirobifluorenyl, benzofuranyl, dibenzofuranyl, benzothienyl, dibenzothienyl, carbazolyl, benzocarbazolyl, and dibenzocarbazolyl; and
wherein the substituent on the substituted C group is selected from deuterium;
optionally, Ar₂ is selected from phenyl, phenyl substituted with at least one deuterium and naphthyl substituted with at least one deuterium;
optionally, Ar₂ is selected from phenyl, phenyl substituted with at least one deuterium and naphthyl substituted with at least one deuterium, wherein the number of deuteriums on the phenyl substituted with at least one deuterium is 1 to 2, and the number of deuteriums on the naphthyl substituted with at least one deuterium is 1to 6;
optionally, Ar₁ is selected from hydrogen, deuterium, phenyl, naphthyl, and phenanthrenyl;
and
optionally, Ar₁ is selected from hydrogen, deuterium, and phenyl.

6. The deuterated compound according to any one of claims 1 to 5, wherein L is selected from a linking bond and C6-C12 arylene;
optionally, L is selected from a linking bond, phenylene, and naphthylene; and
optionally, Ar₂ is selected from phenyl substituted with 1 or 2 deuteriums, and naphthyl substituted with 1 to 6 deuteriums.

7. The deuterated compound according to any one of claims 1 to 6, wherein the deuterated compound is selected from one of M-1 to M-100:

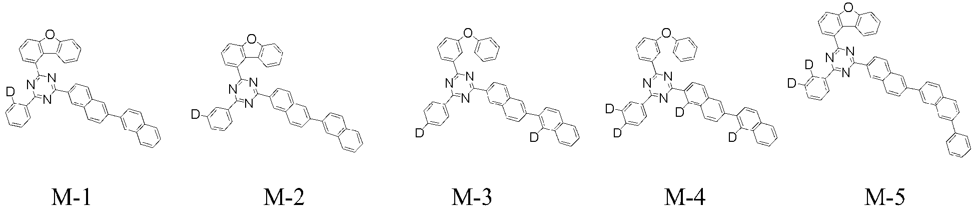

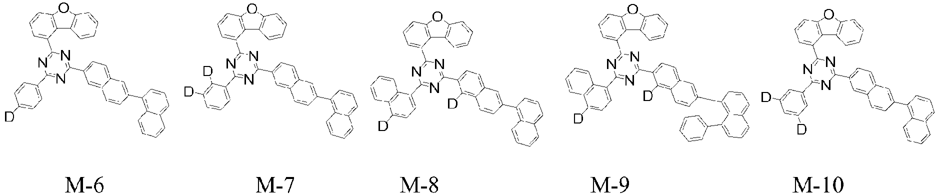

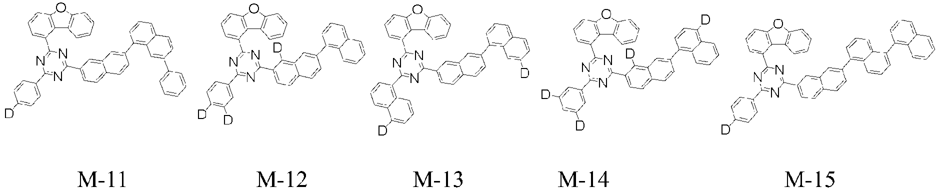

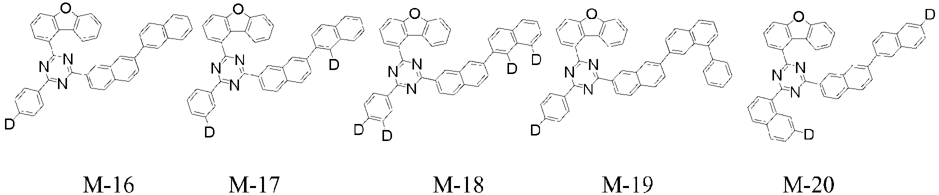

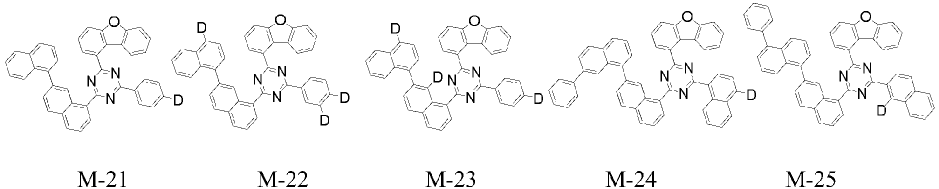

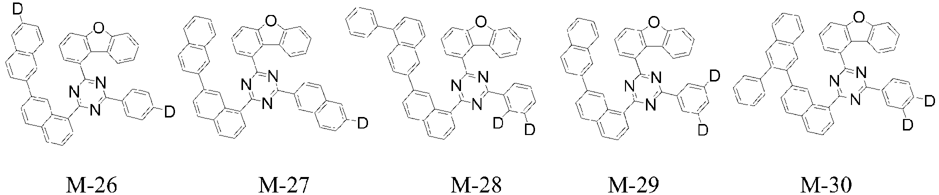

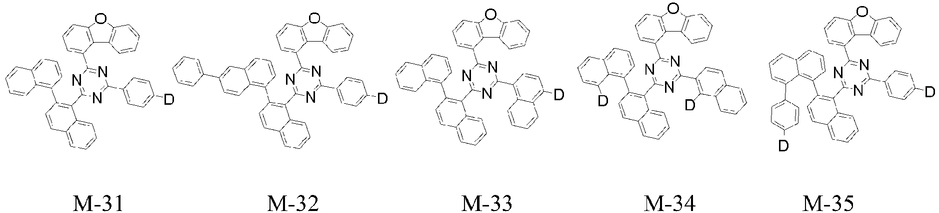

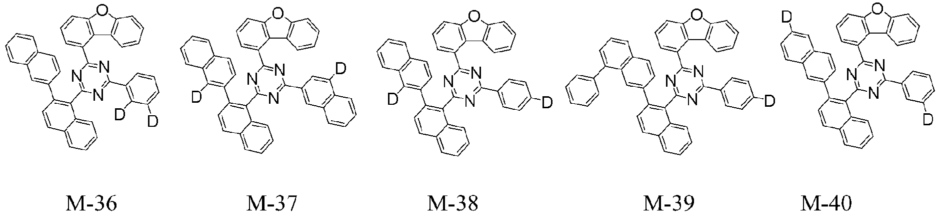

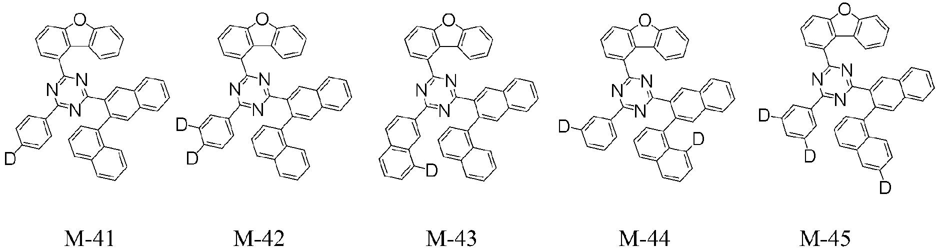

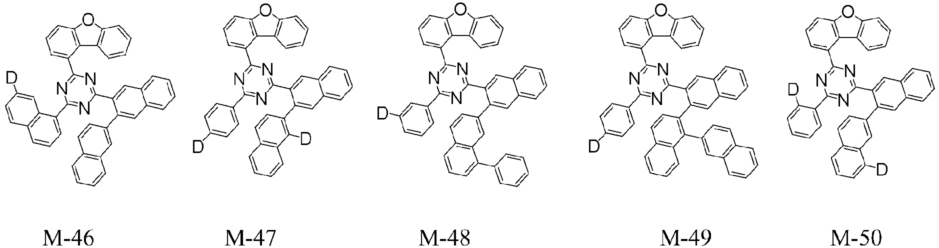

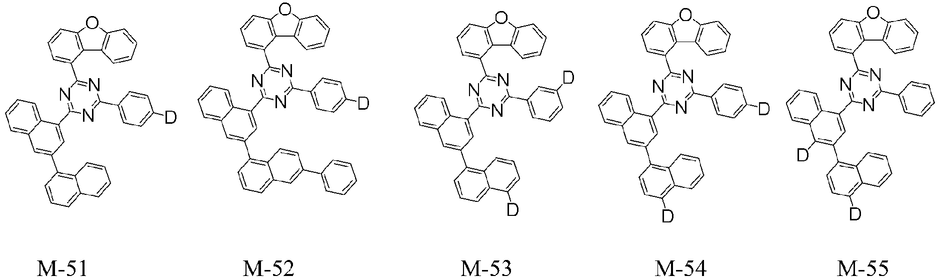

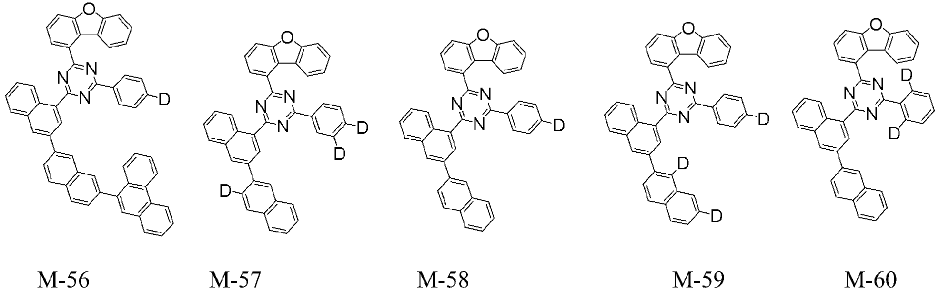

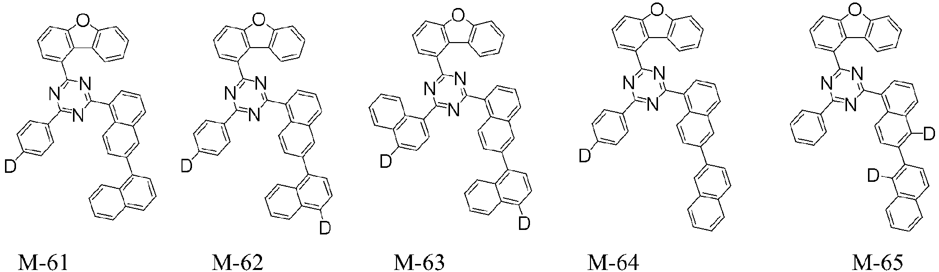

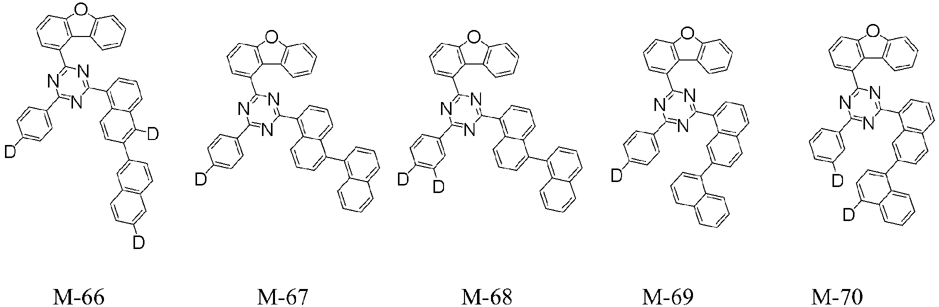

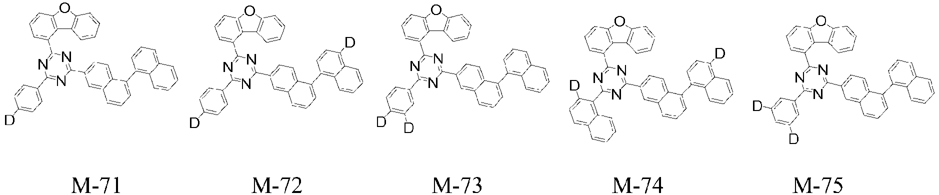

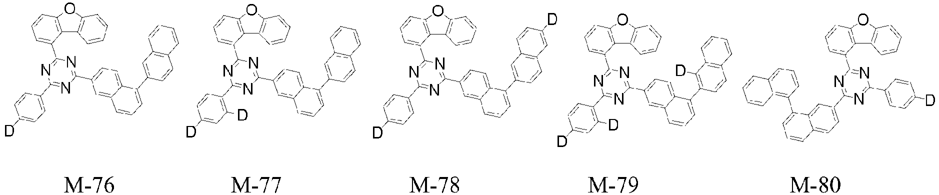

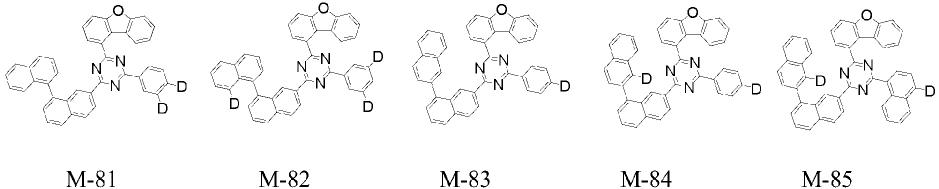

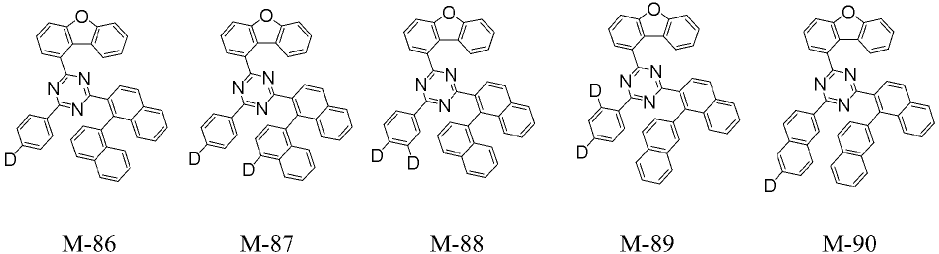

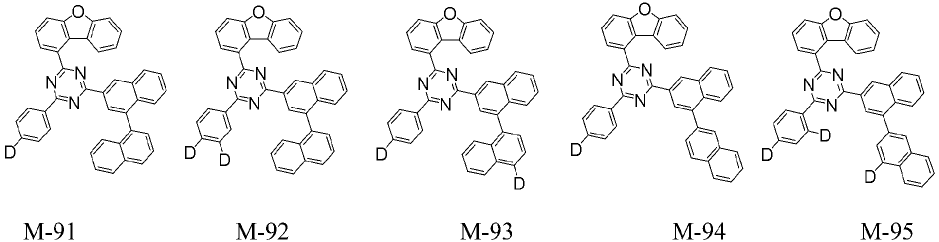

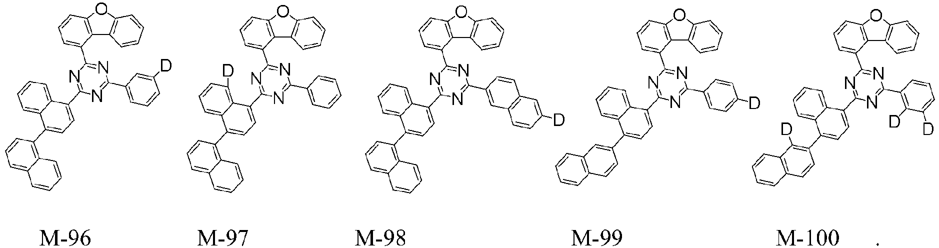

8. A light-emitting host material composition, comprising a first host material and a second host material, wherein the first host material comprises the deuterated compound according to any one of claims 1 to 7, and the second host material comprises a compound shown in Formula (2) below: 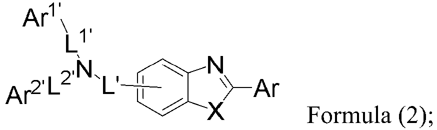
wherein X is selected from O and S;
L', L^{1'}, and L^{2'} are each independently selected from a linking bond, a substituted or unsubstituted C6-C30 arylene, and a substituted or unsubstituted C3-C30 heteroarylene, and L', L^{1'}, and L^{2'} are each independently present; and
wherein Ar^{1'}, Ar^{2'}, and Ar are each independently selected from hydrogen, deuterium, halogen, cyano, a substituted or unsubstituted C6-C60 aryl, a substituted or unsubstituted C6-C60 arylamino, a substituted or unsubstituted C3-C60 heteroarylamino, and a substituted or unsubstituted C3-C60 heteroaryl; and
the substituents on the substituted C6-C30 arylene, C3-C30 heteroarylene, substituted C6-C30 aryl, substituted C3-C30 heteroaryl, substituted C6-C60 arylamino, and substituted C3-C60 heteroarylamino are each independently selected from one or at least two of a combination of deuterium, halogen, cyano, C1-C12 alkyl, C3-C12 cycloalkyl, C6-C30 aryl, C3-C30 heteroaryl, C6-C60 arylamino, and C3-C60 heteroarylamino.

9. The light-emitting host material composition according to claim 8, wherein Ar^{1'}, Ar^{2'}, and Ar are each independently selected from hydrogen, deuterium, halogen, and a substituted or unsubstituted E group, the E group is selected from: phenyl, naphthyl, biphenyl, phenanthrenyl, fluoranthenyl, chrysenyl, terphenyl, triphenylene, phenylnaphthyl, naphthylphenyl, dimethylfluorenyl, phenylmethylfluorenyl, diphenylfluorenyl, pyridinyl, pyridinylphenyl, phenylpyridinyl, spiro-bifluorenyl, benzodimethylfluorenyl, benzodiphenylfluorenyl, benzospirobifluorenyl, dibenzofuranyl, benzonaphthofuranyl, benzonaphthothienyl and dibenzothienyl;
the substituents on the substituted E group are each independently selected from one or at least two of a combination of C1-C6 alkyl, C3-C12 cycloalkyl, C6-C25 aryl, C3-C25 heteroaryl, C6-C60 arylamino, and C3-C60 heteroarylamino;
optionally, L', L^{1'}, and L^{2'} are each independently selected from a linking bond, a substituted or unsubstituted C6-C12 arylene, and a substituted or unsubstituted C3-C12 heteroarylene;
the substituents on the substituted C6-C12 arylene, and substituted C3-C12 heteroarylene are each independently selected from one or at least two of a combination of C1-C6 alkyl, C3-C12 cycloalkyl, C6-C25 aryl, C3-C25 heteroaryl, C6-C60 arylamino, and C3-C60 heteroarylamino;
optionally, Ar^{1'}-Ar^{2'} are each independently selected from phenyl, naphthyl, biphenyl, chrysenyl, terphenyl, phenylnaphthyl, naphthylphenyl, dimethylfluorenyl, diphenylfluorenyl, pyridinyl, pyridinylphenyl, phenylpyridinyl, phenylcarbazolyl, phenylbenzocarbazolyl, phenylphenanthrocarbazolyl, spiro-bifluorenyl, spiro[fluorene-9,9'-xanthene]yl, phenylmethylfluorenyl, dibenzofuranyl, and benzonaphthofuranyl;
optionally, Ar is selected from phenyl or naphthyl;
optionally, L' is selected from naphthylene; and
optionally, L^{1'} and L^{2'} are each independently selected from a linking bond, phenylene, or naphthylene.

10. The light-emitting host material composition according to claim 8 or 9, wherein the second host material comprises a compound selected from N-1 to N-654:

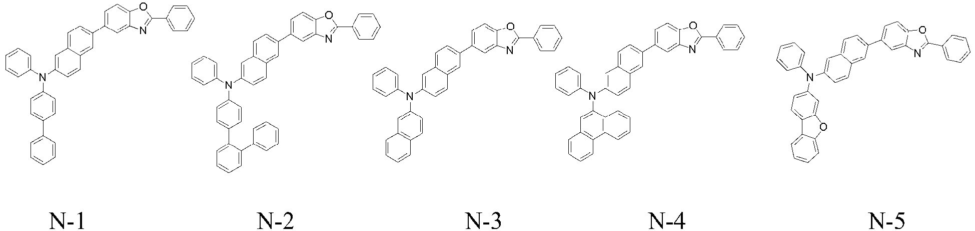

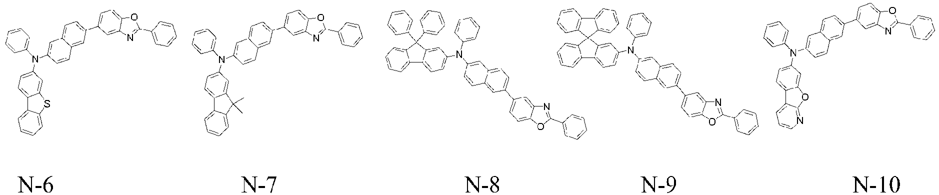

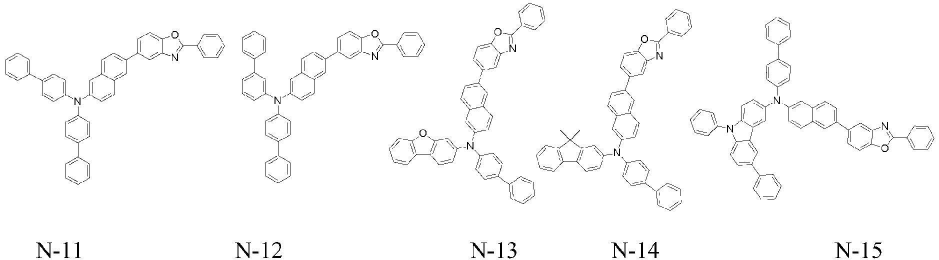

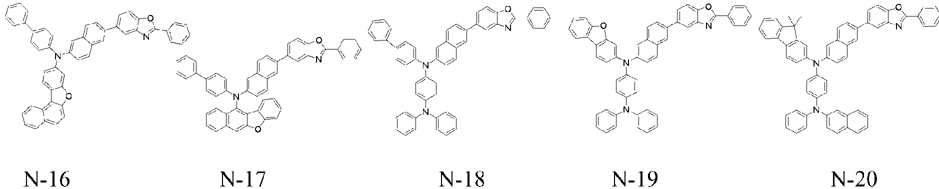

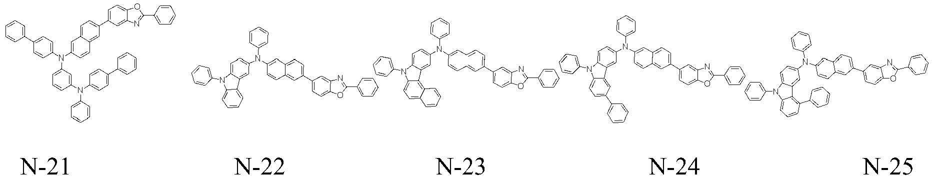

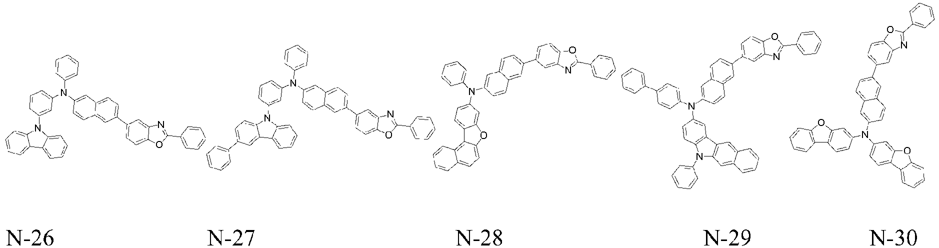

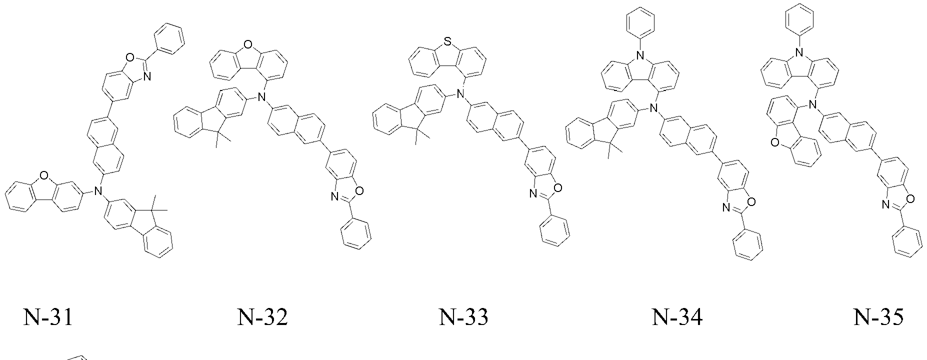

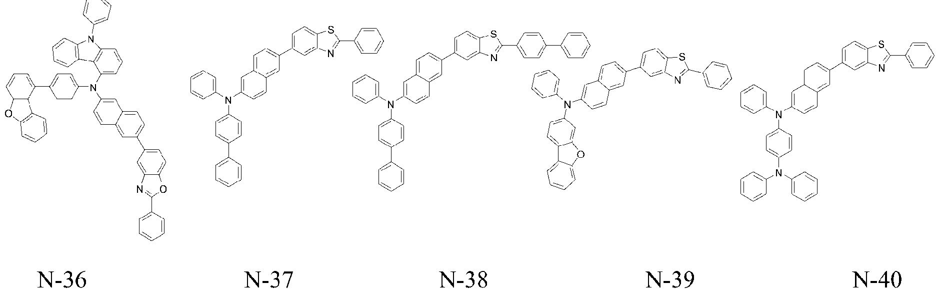

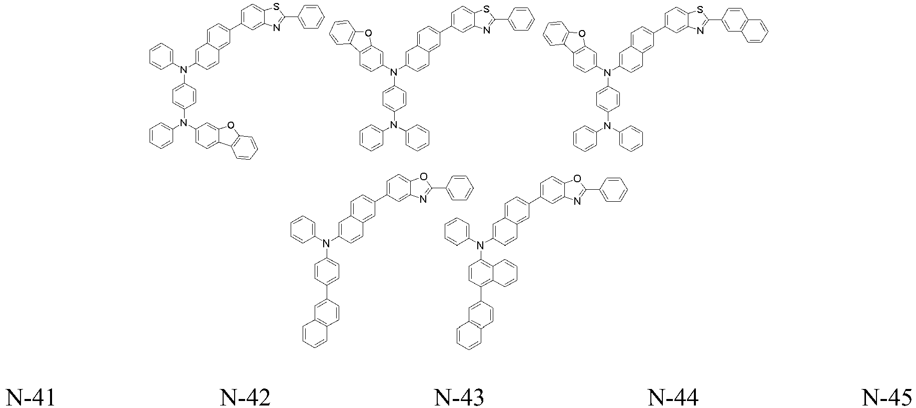

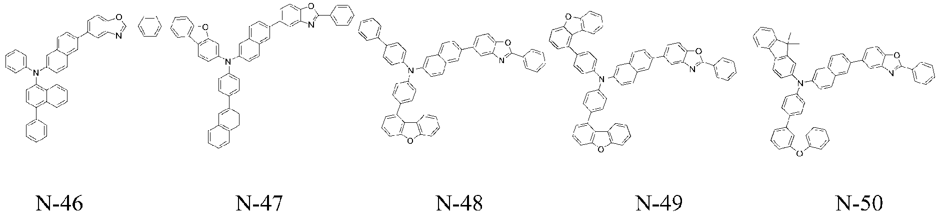

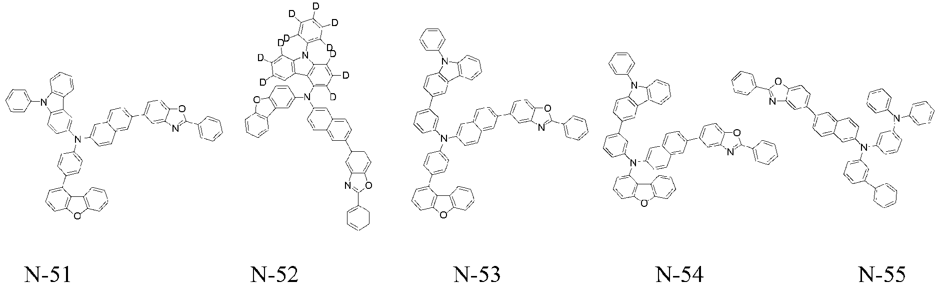

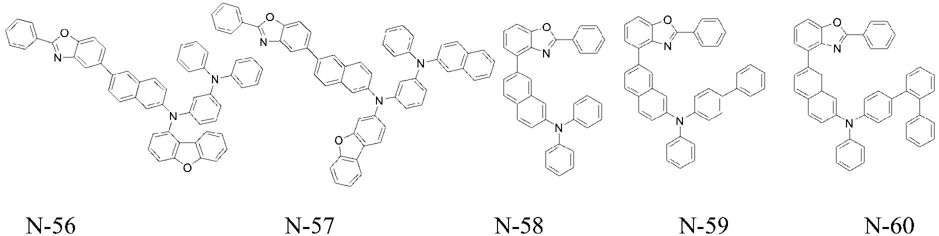

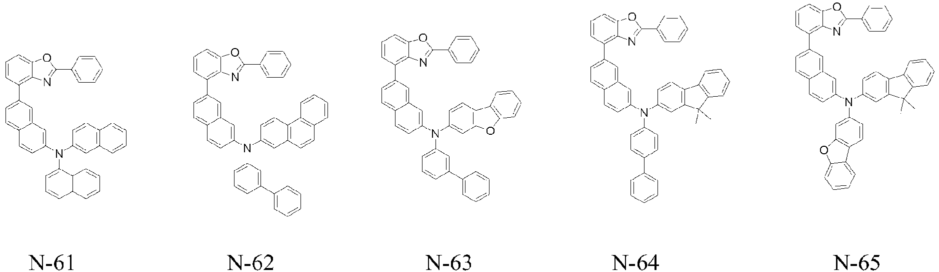

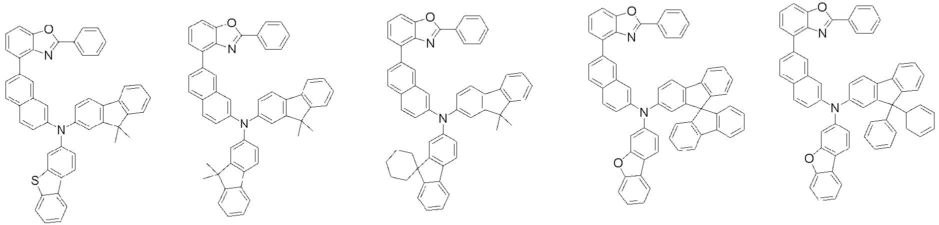

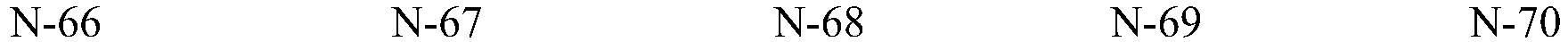

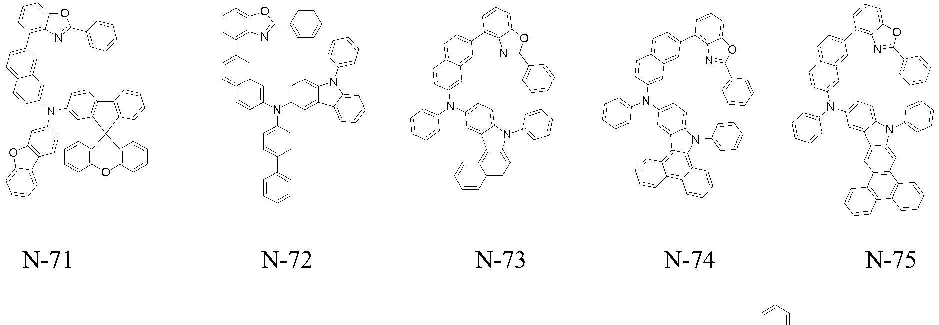

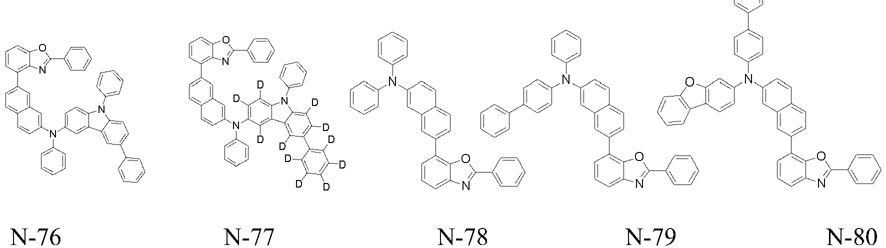

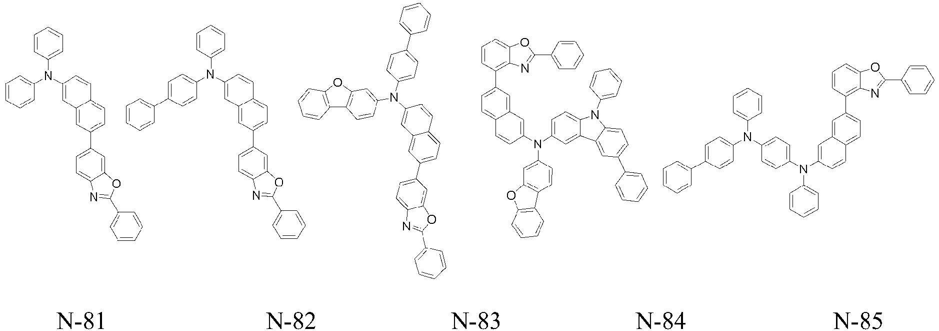

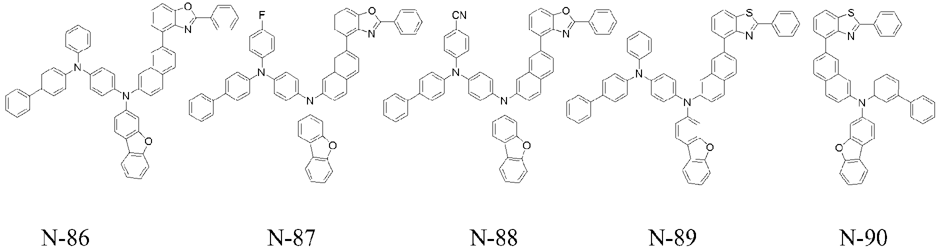

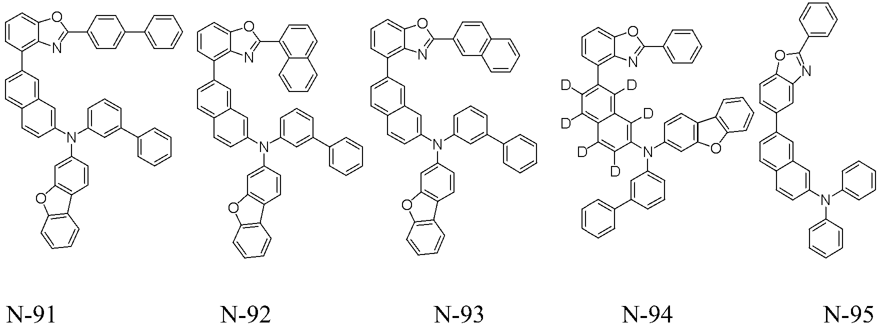

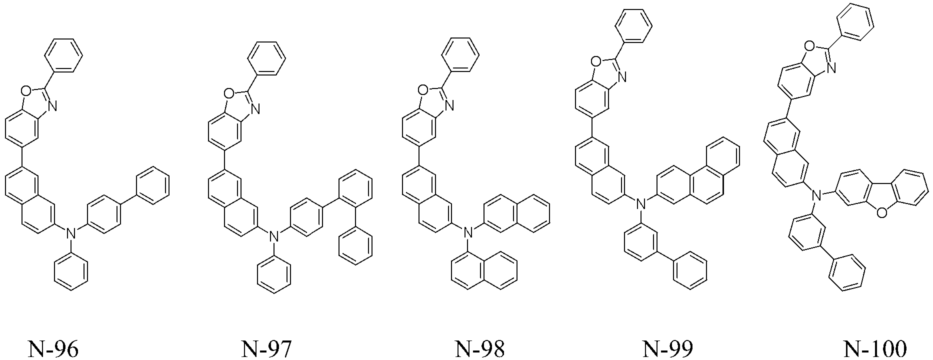

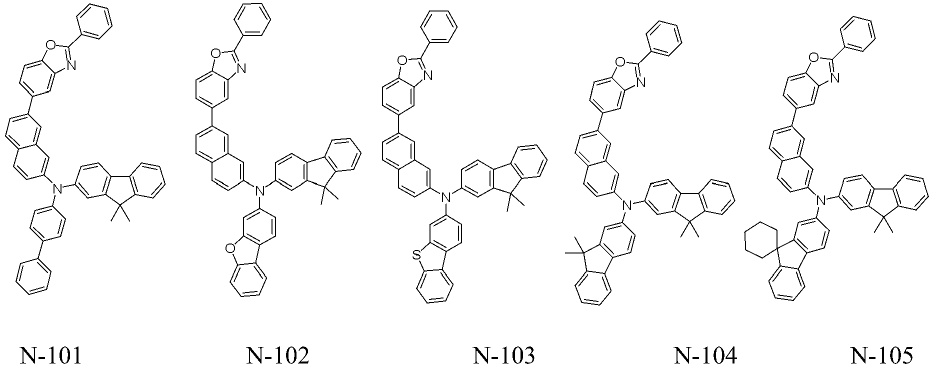

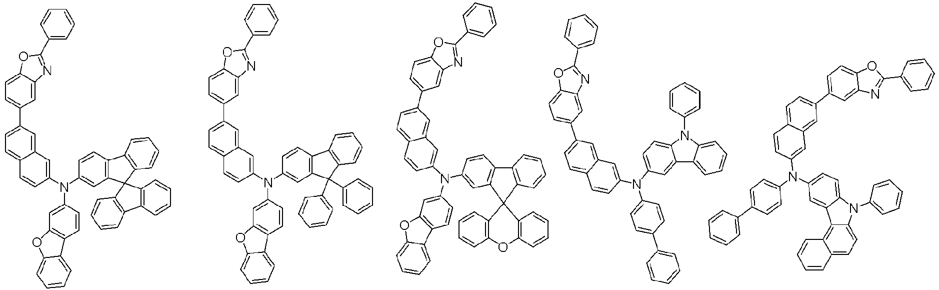

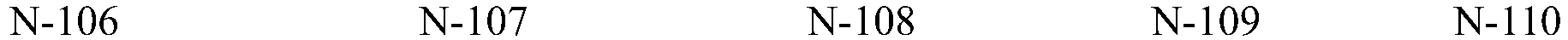

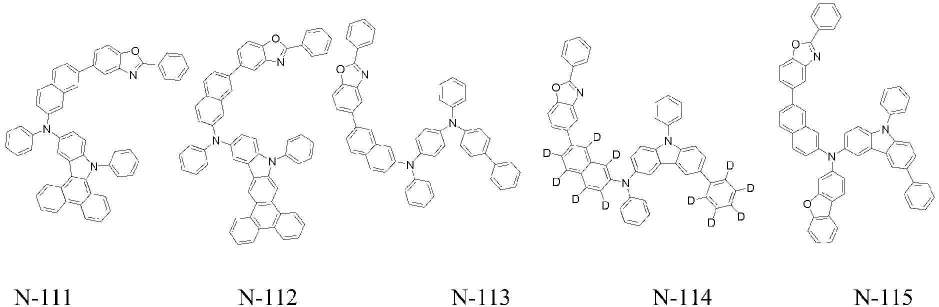

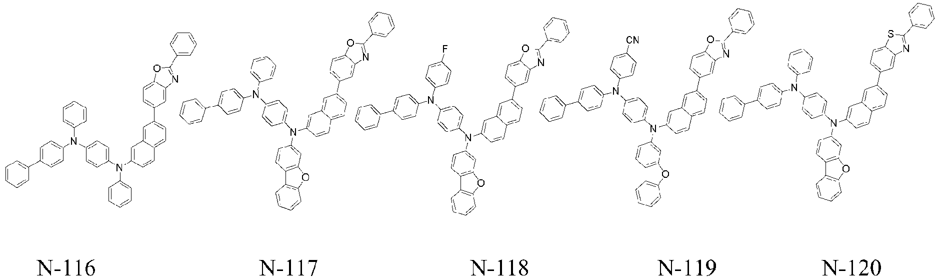

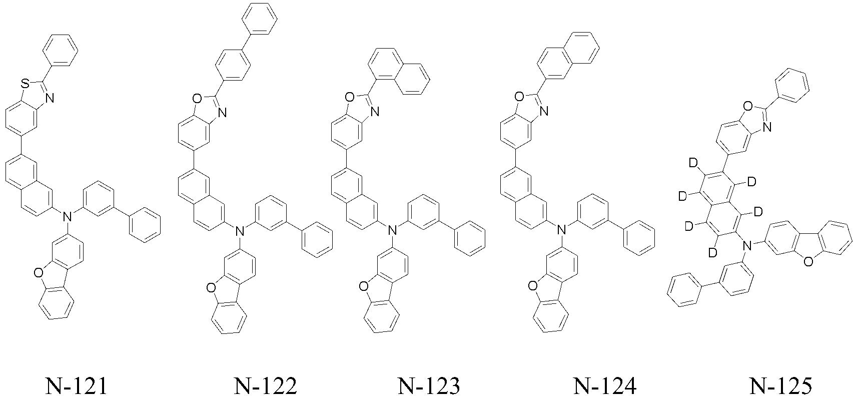

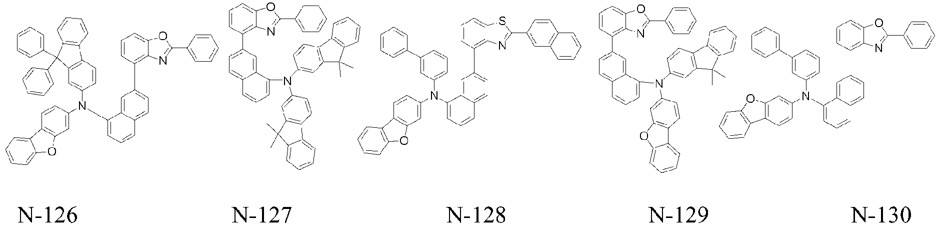

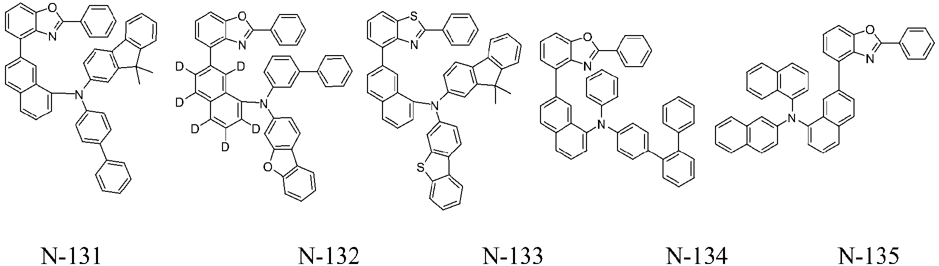

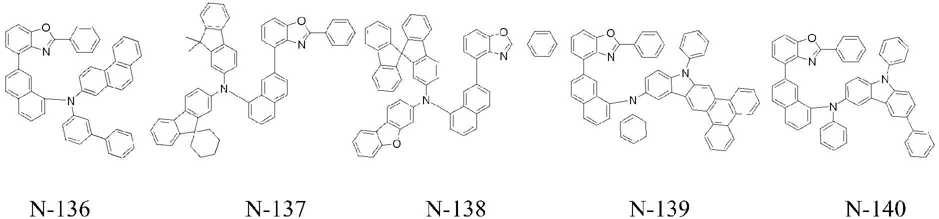

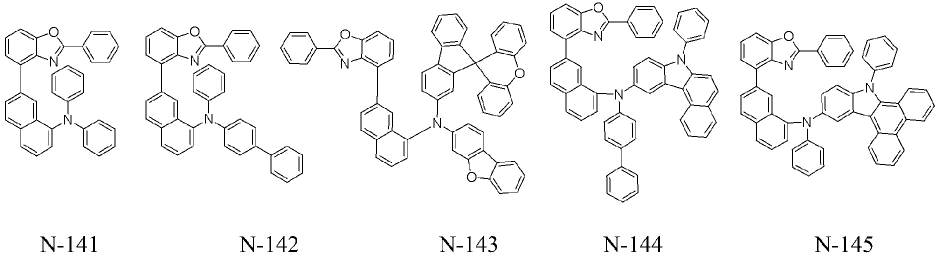

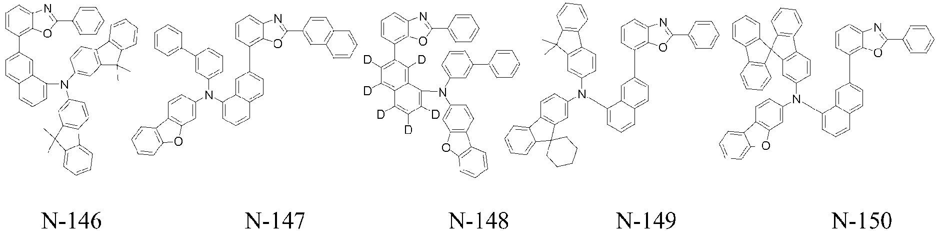

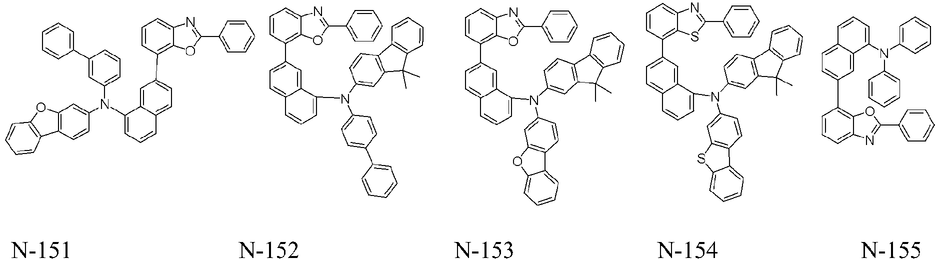

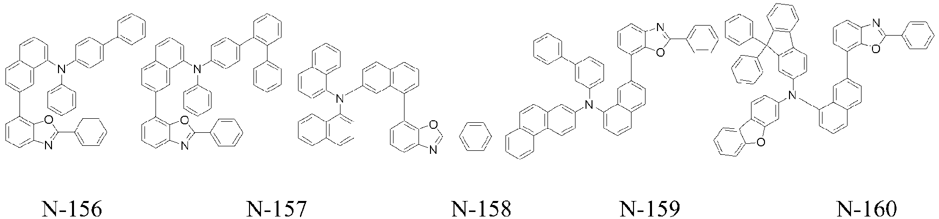

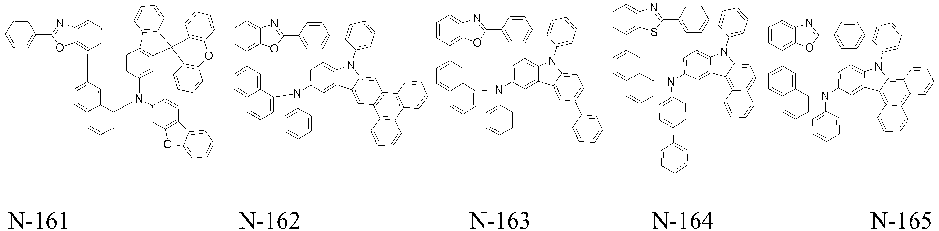

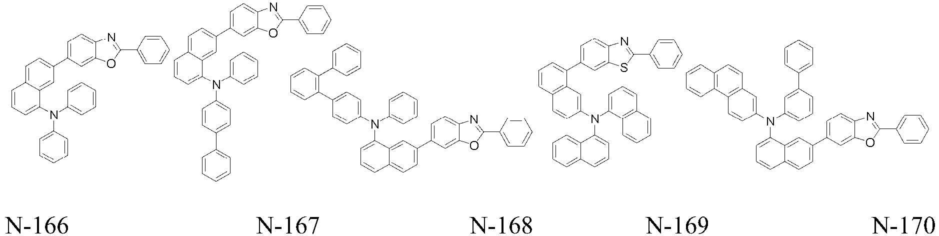

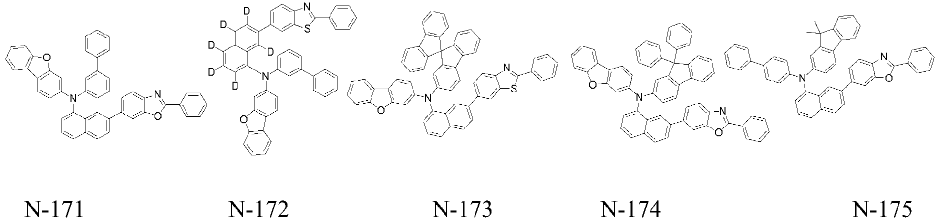

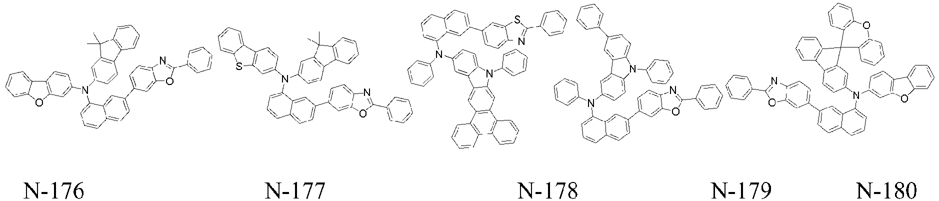

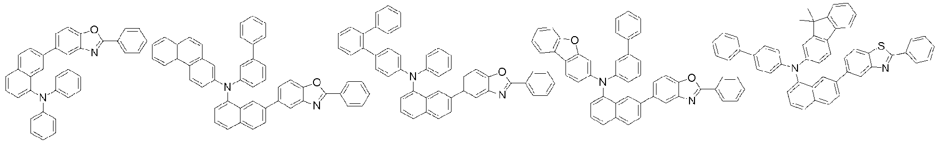

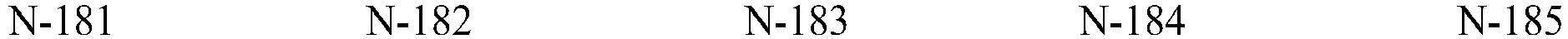

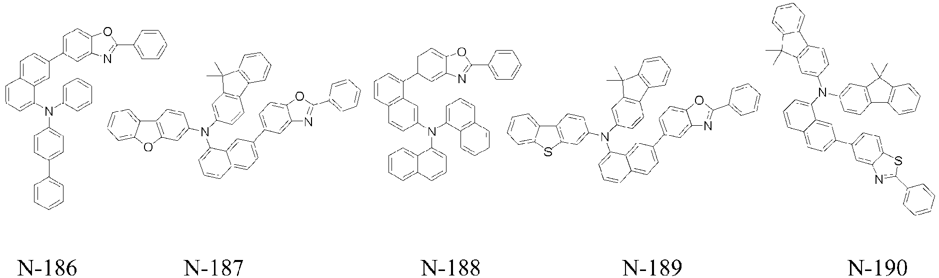

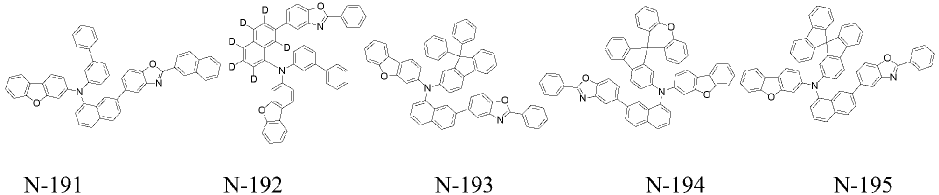

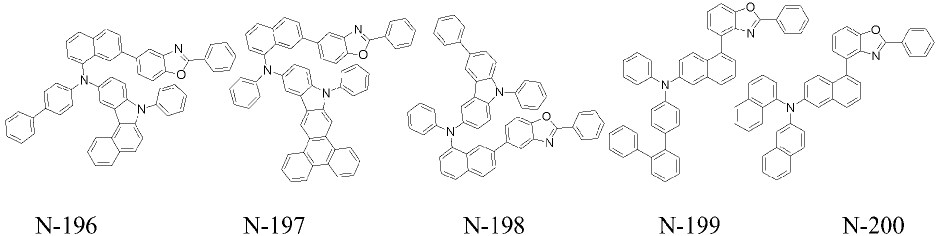

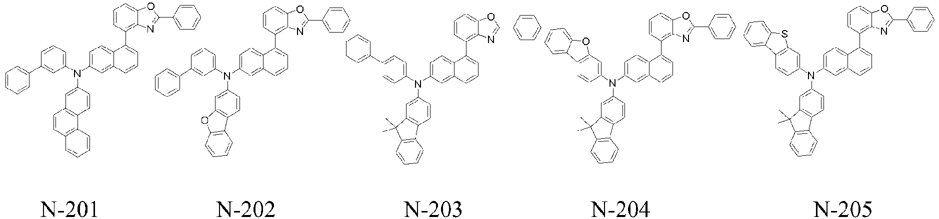

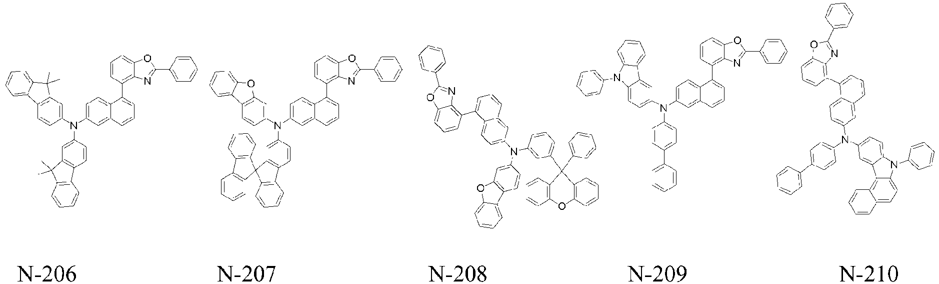

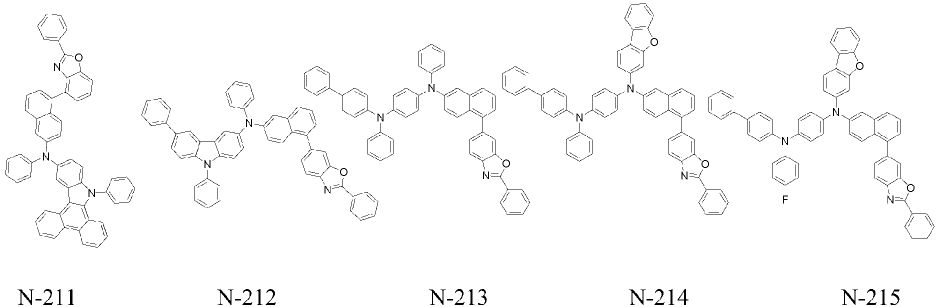

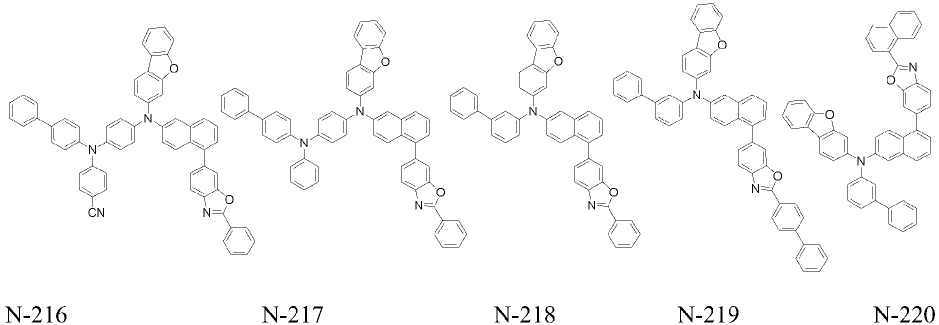

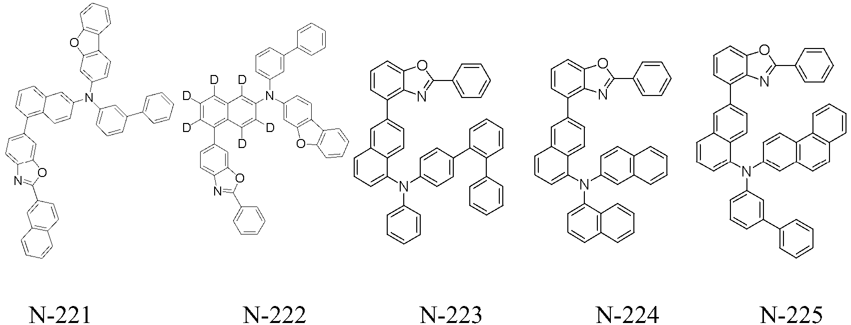

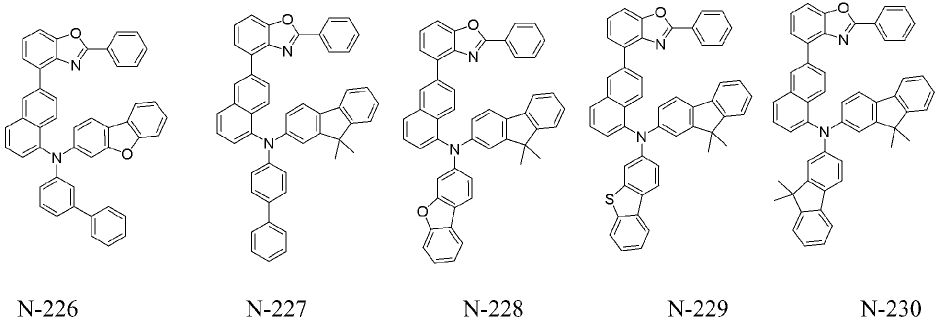

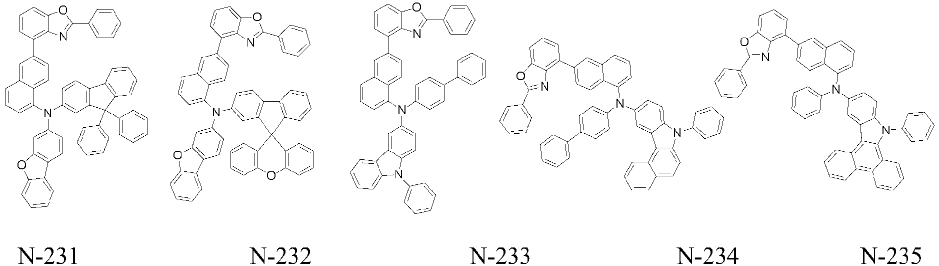

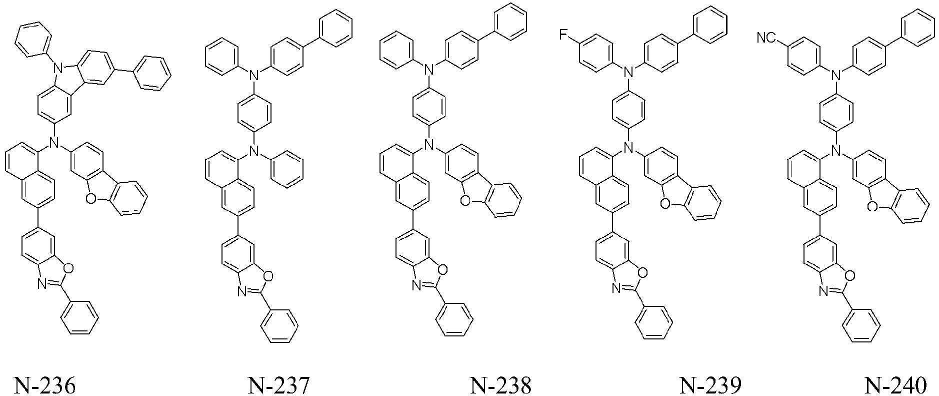

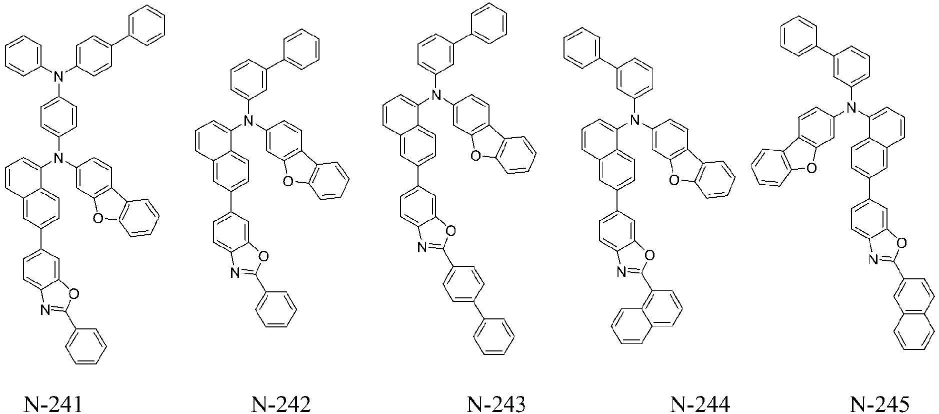

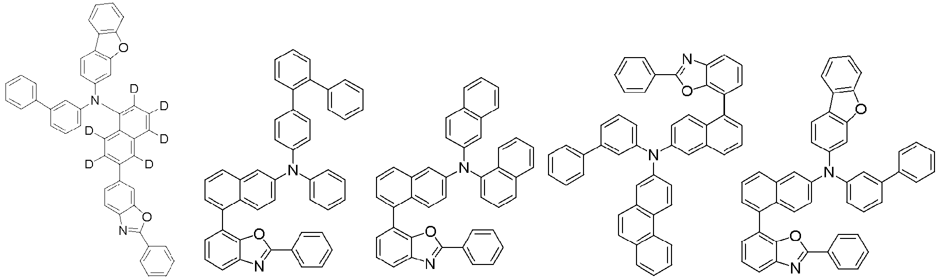

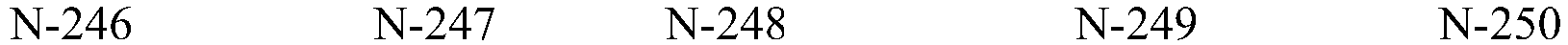

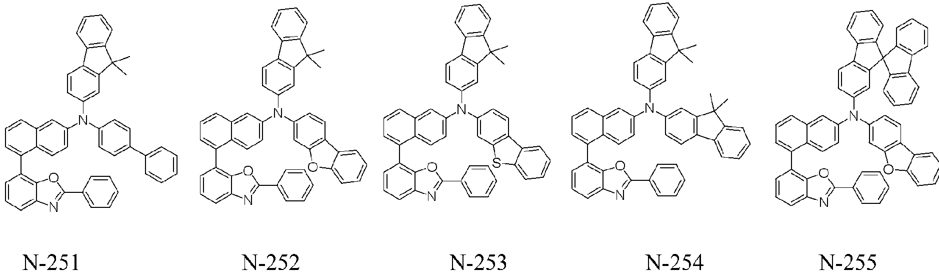

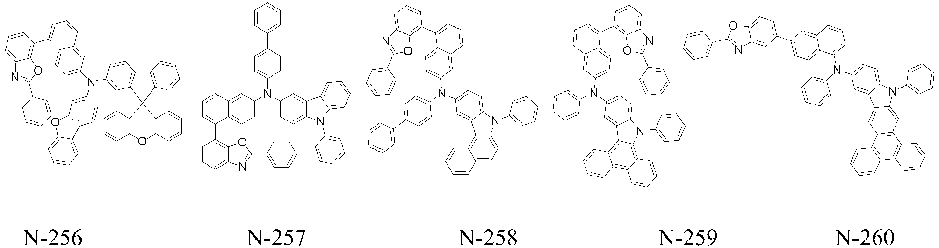

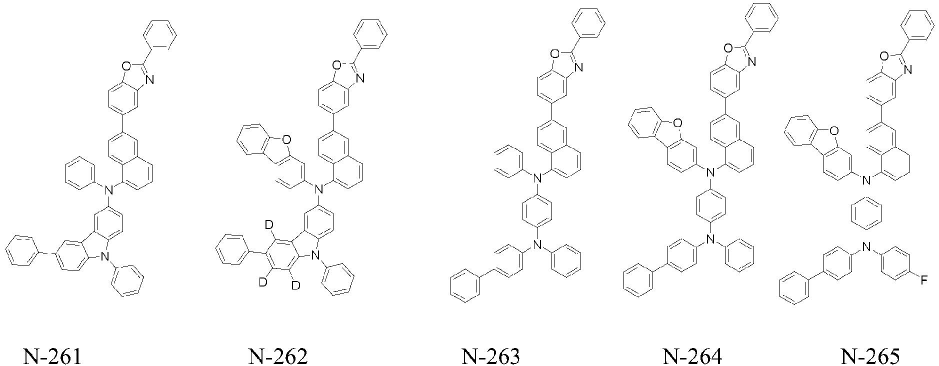

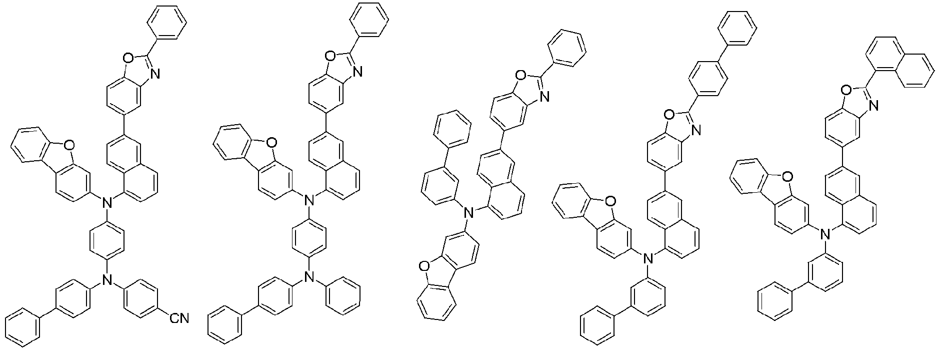

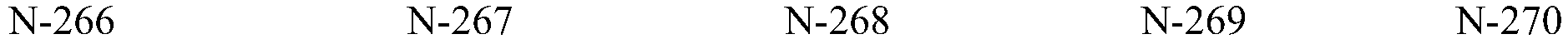

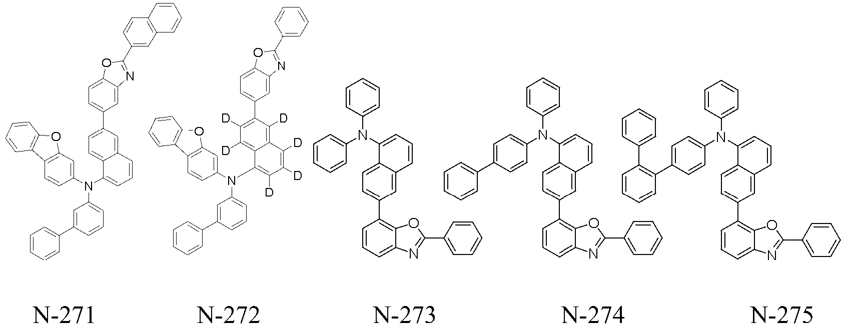

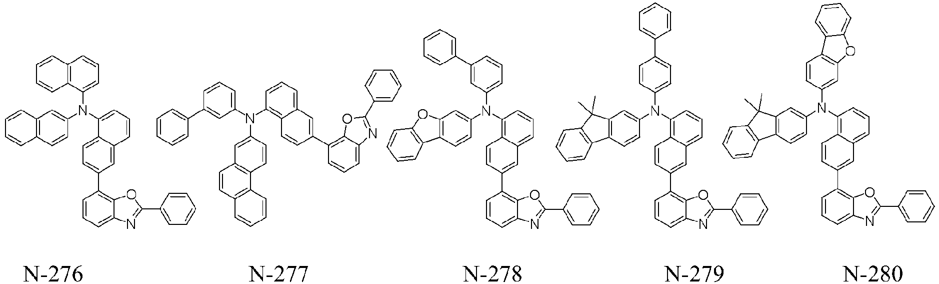

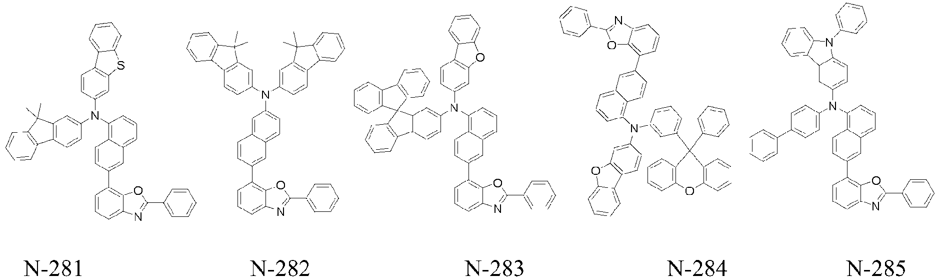

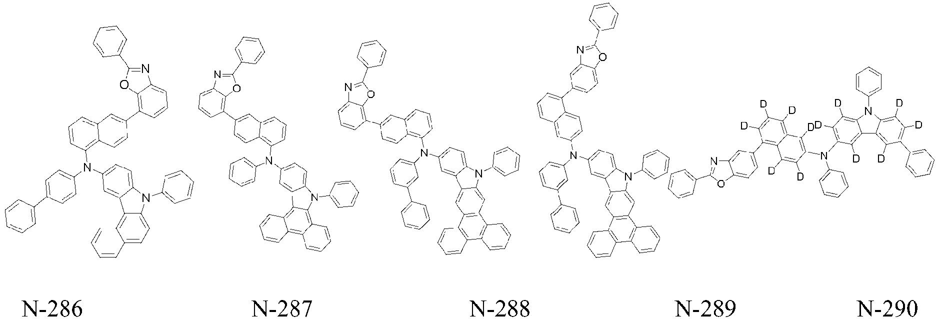

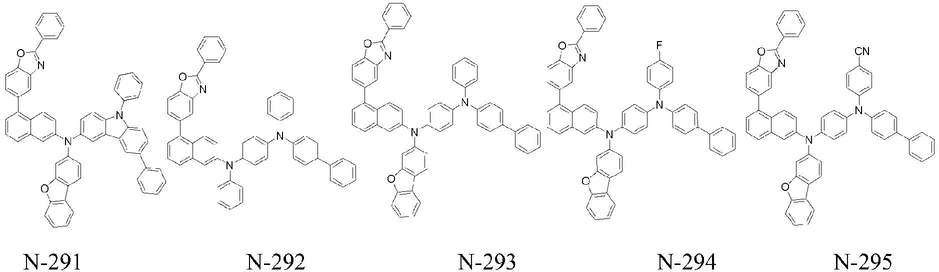

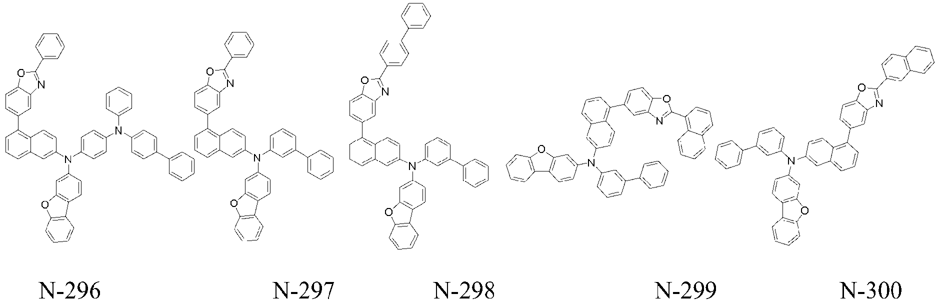

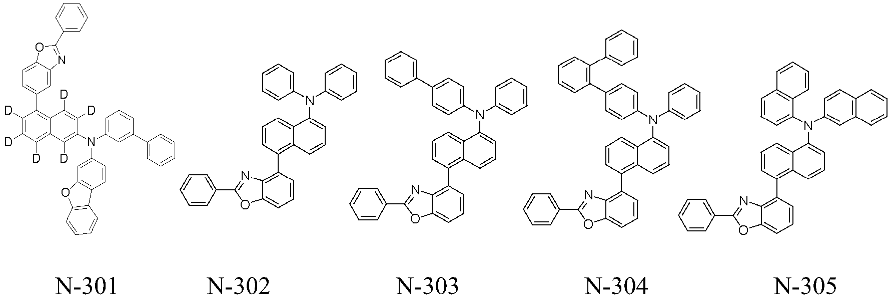

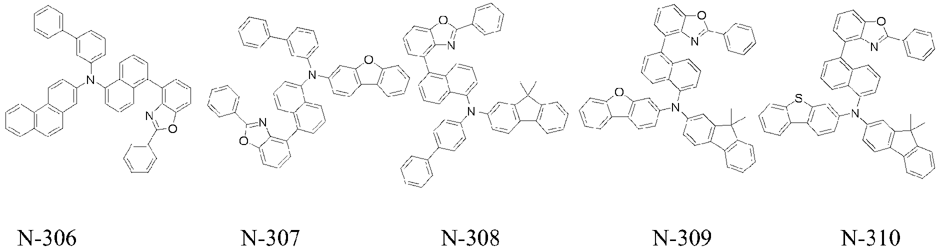

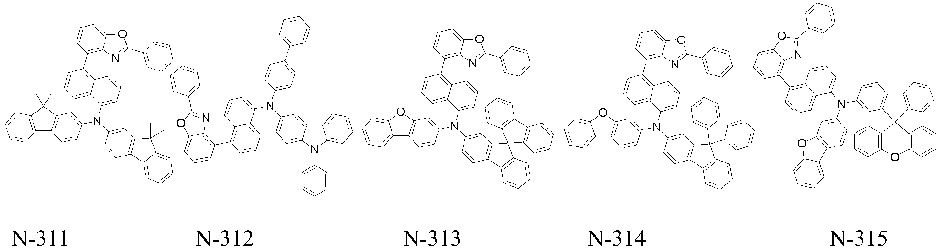

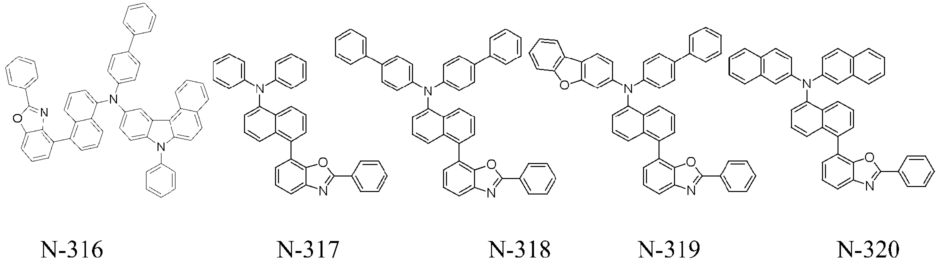

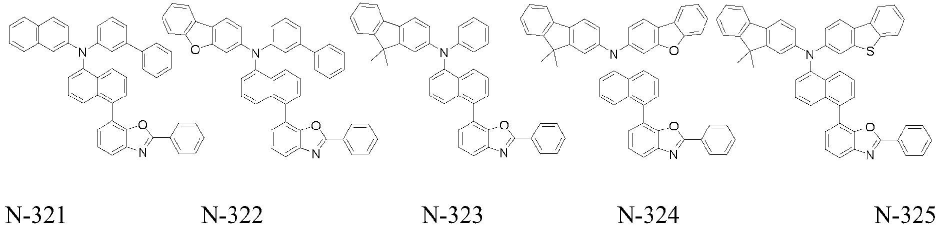

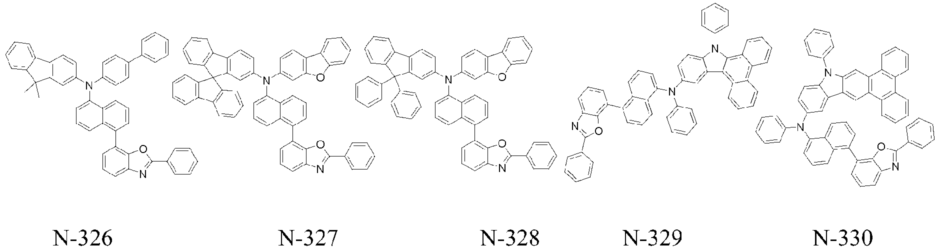

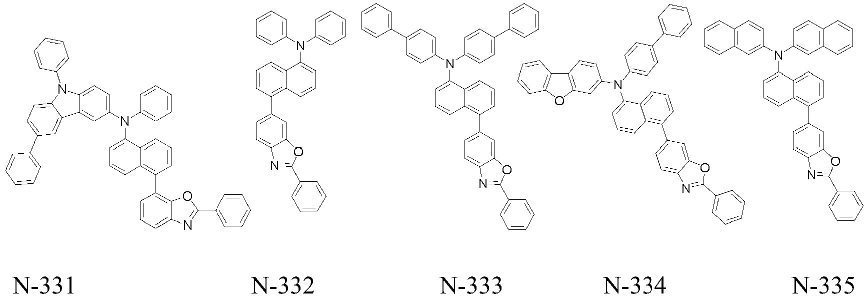

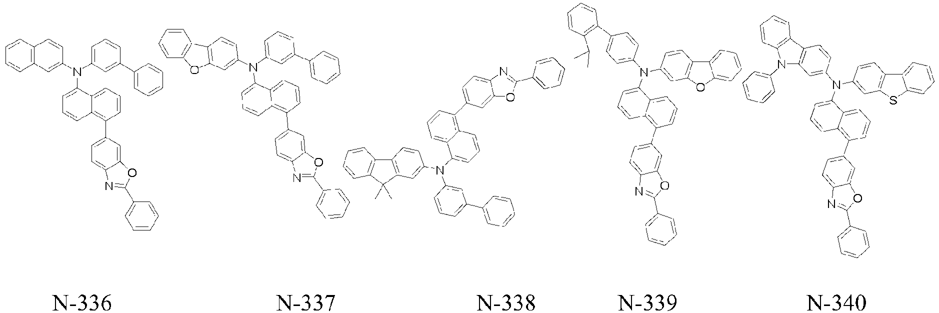

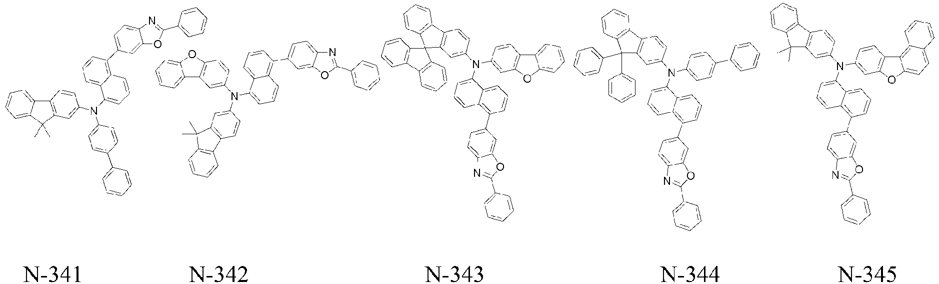

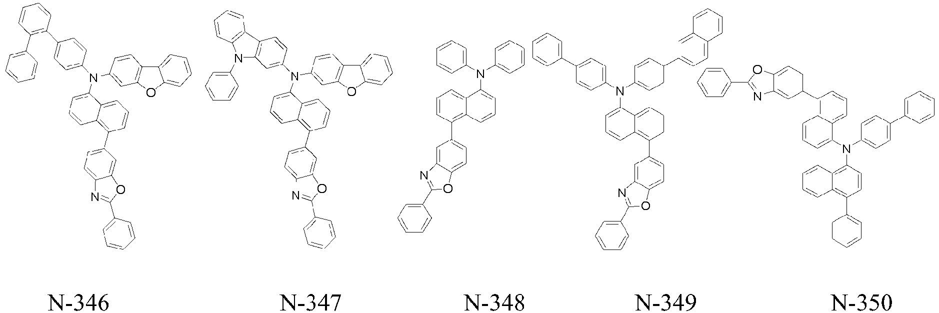

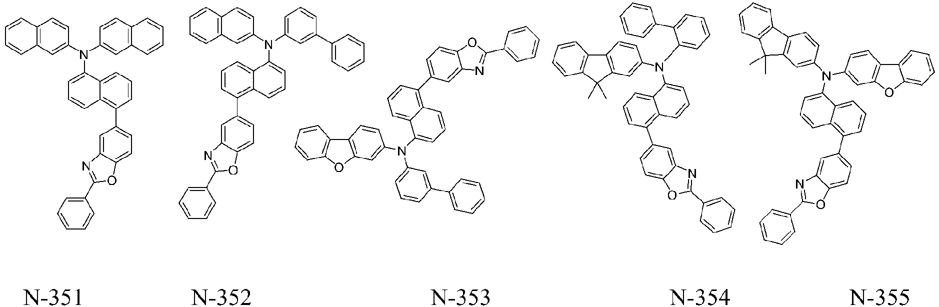

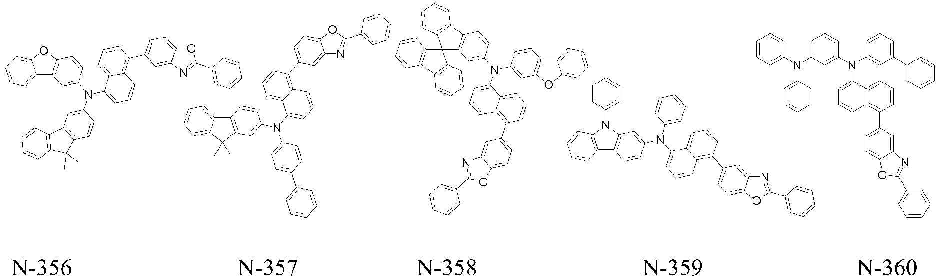

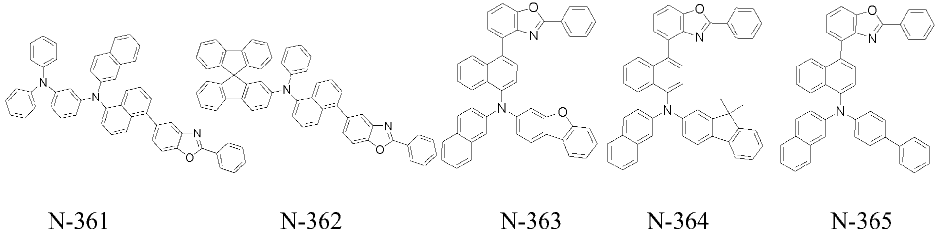

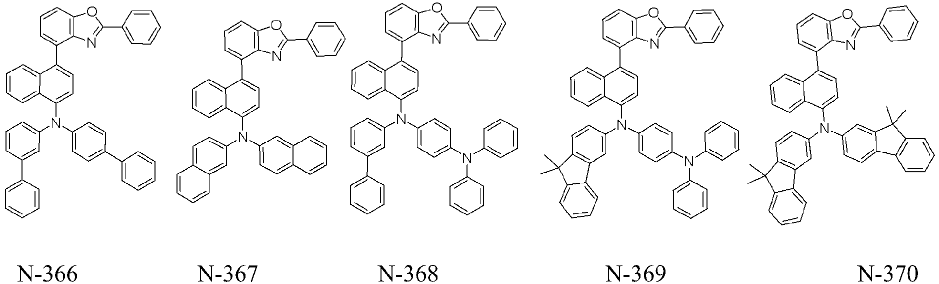

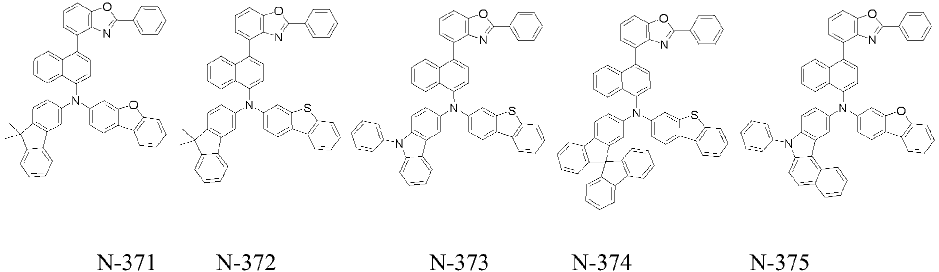

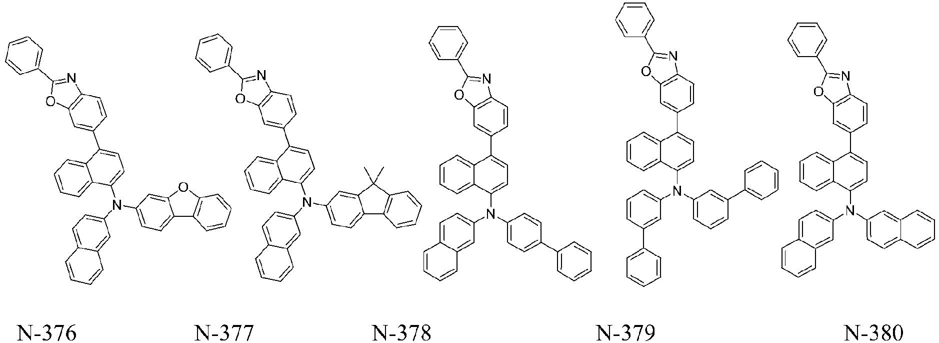

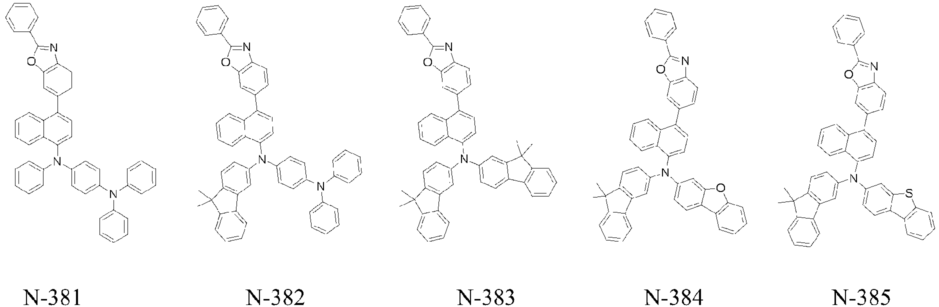

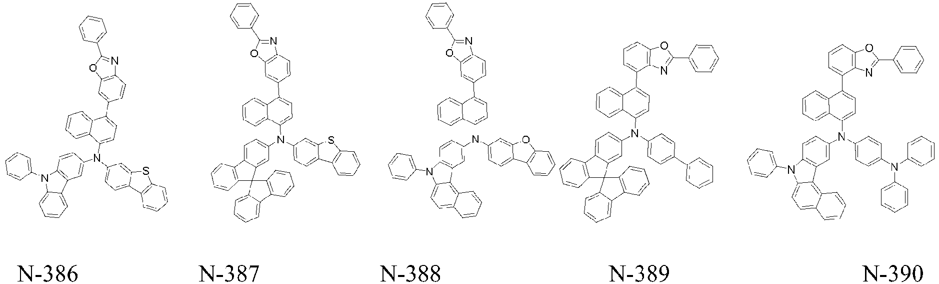

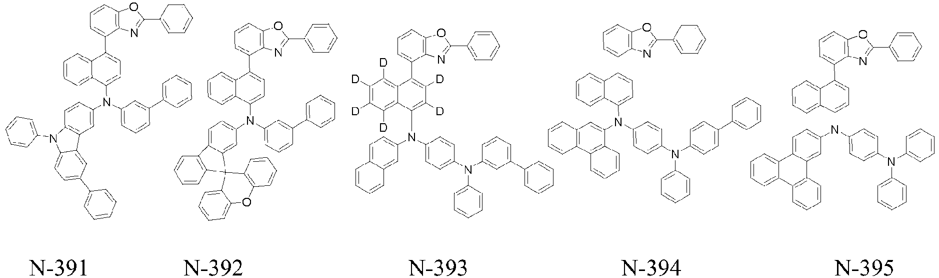

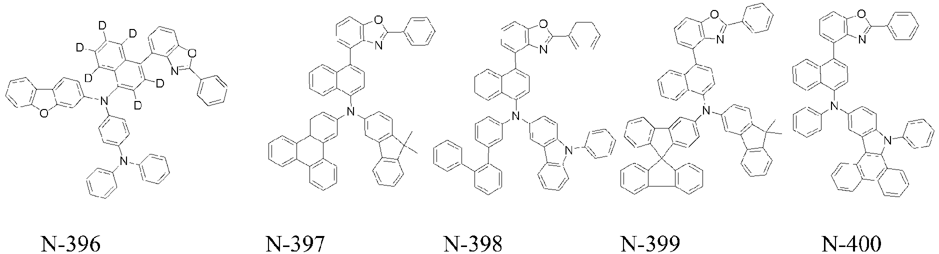

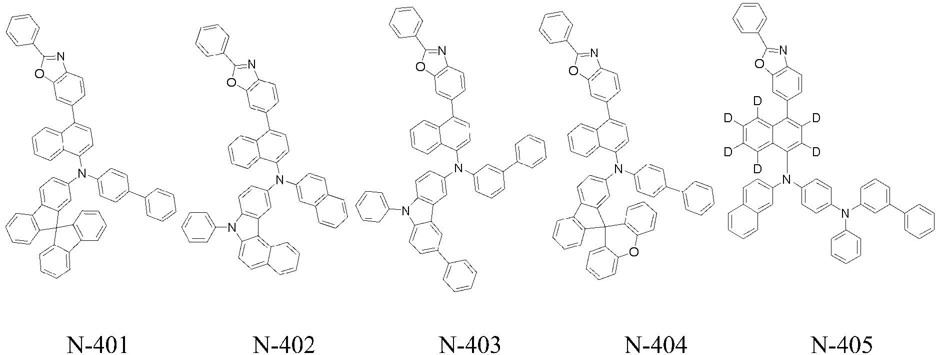

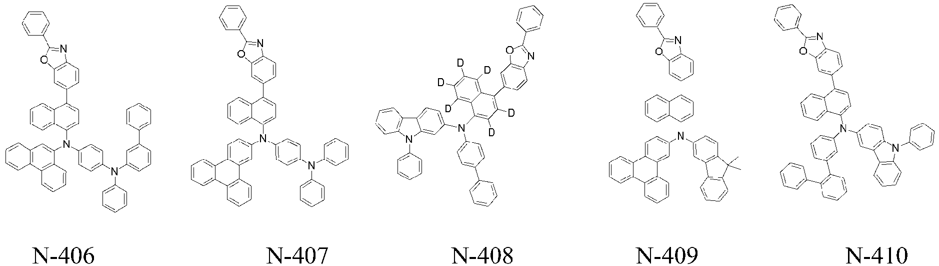

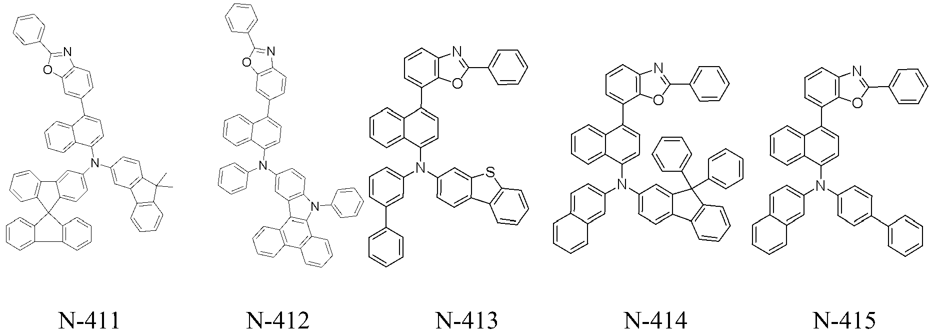

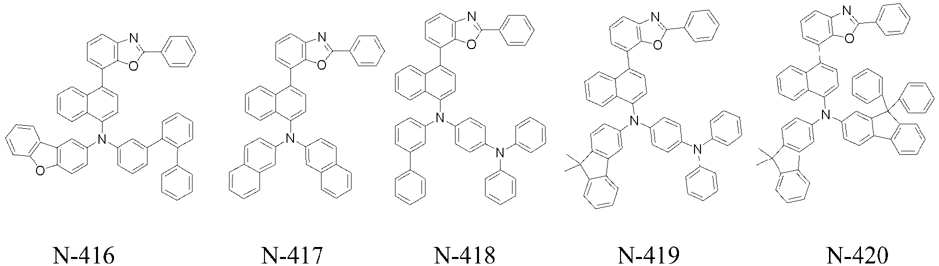

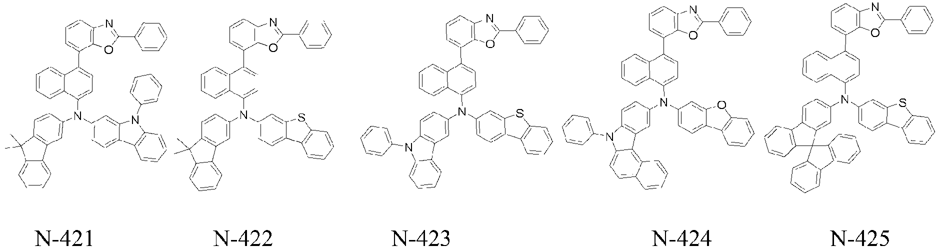

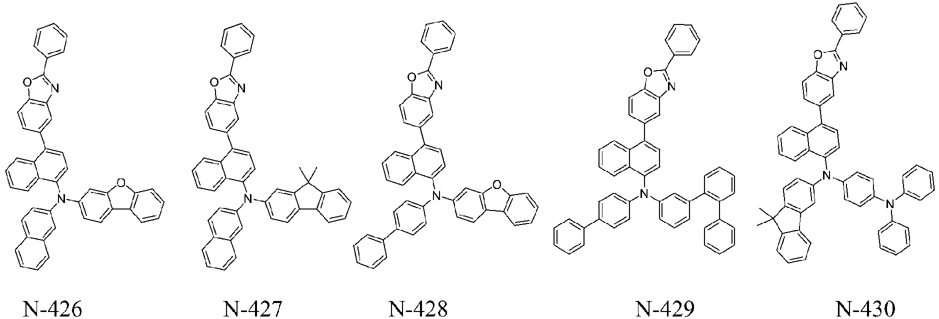

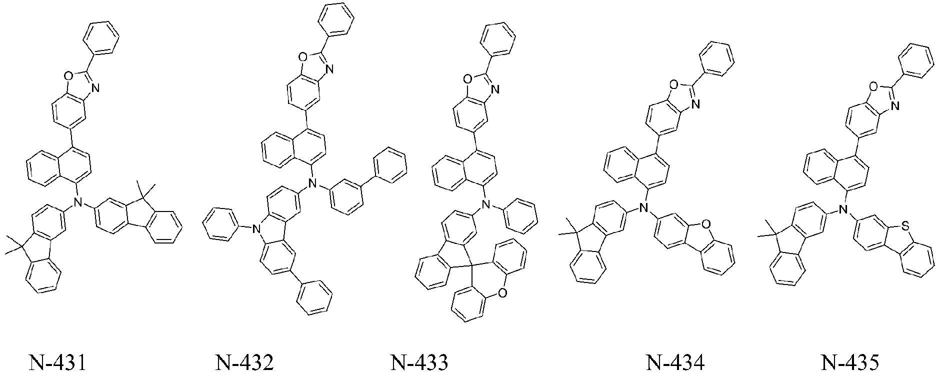

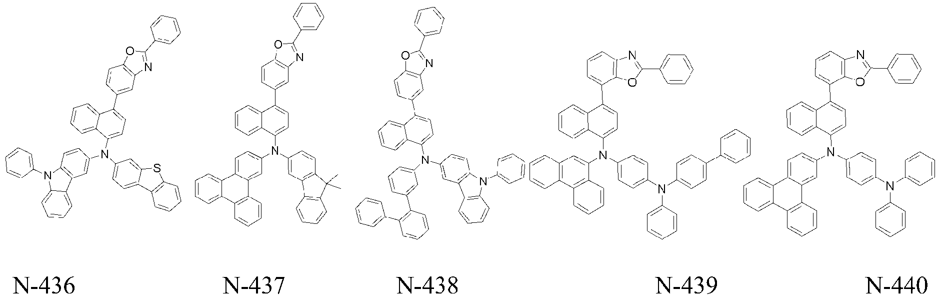

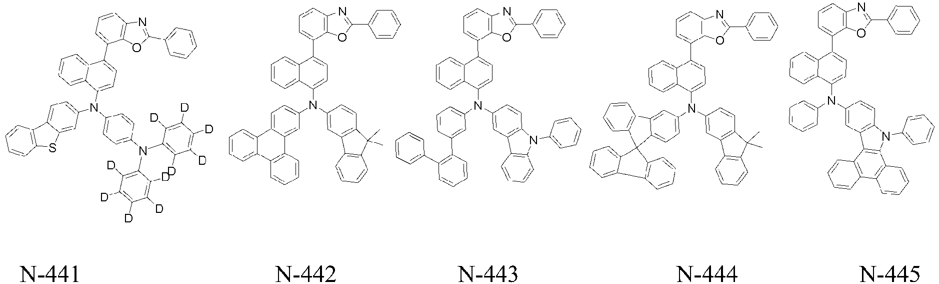

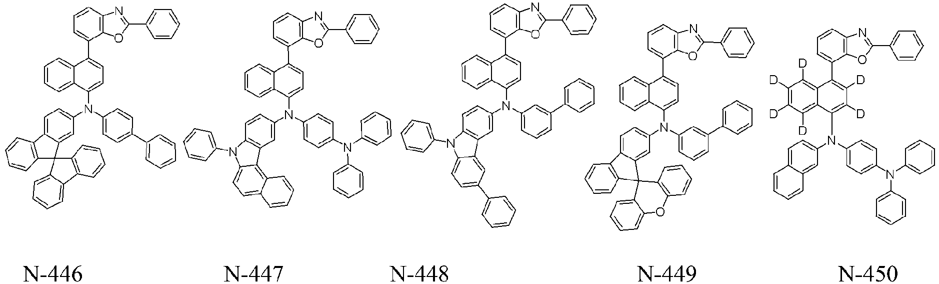

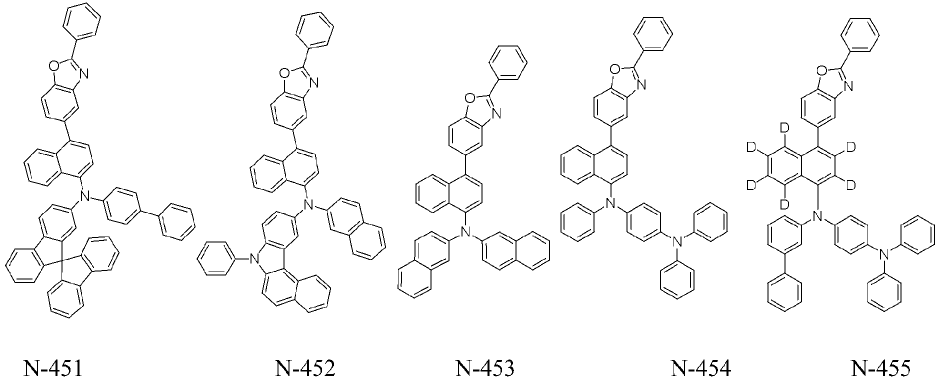

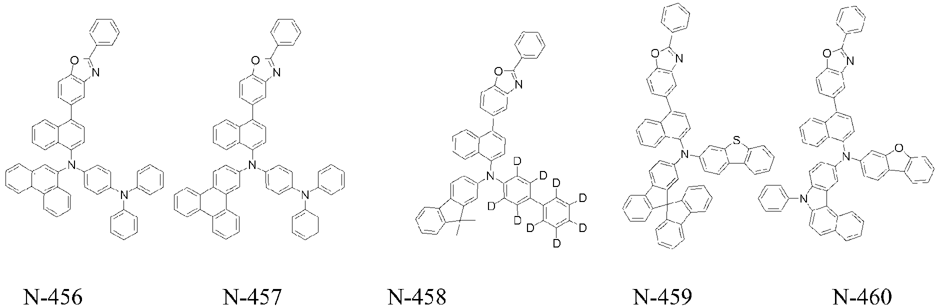

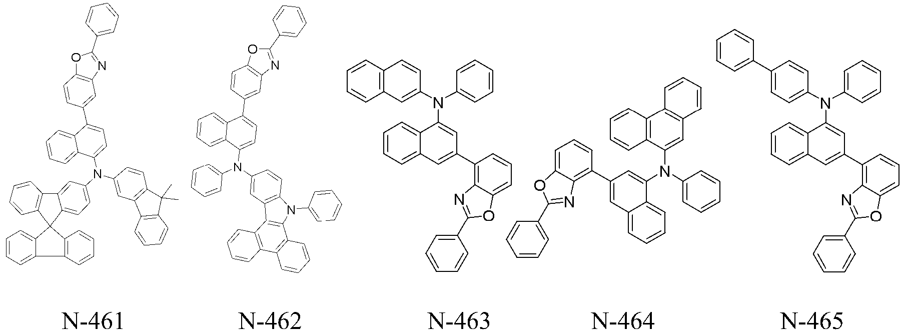

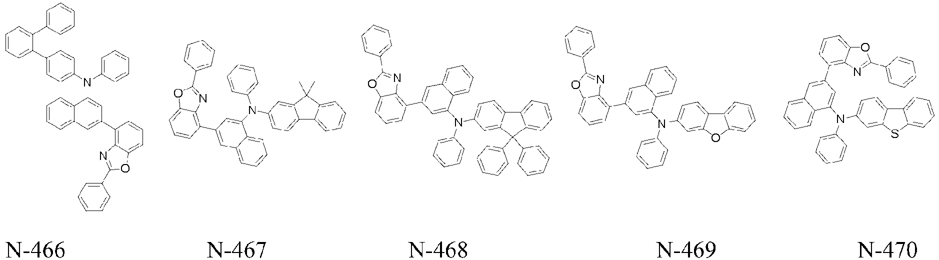

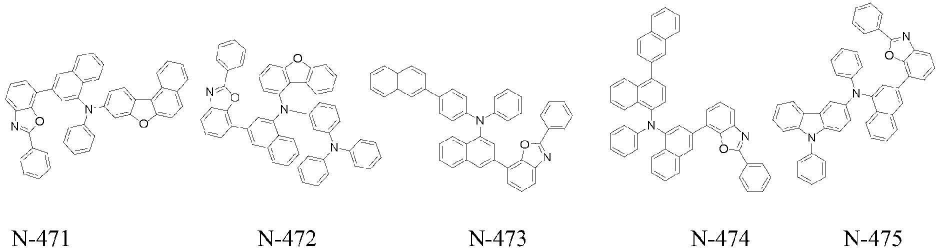

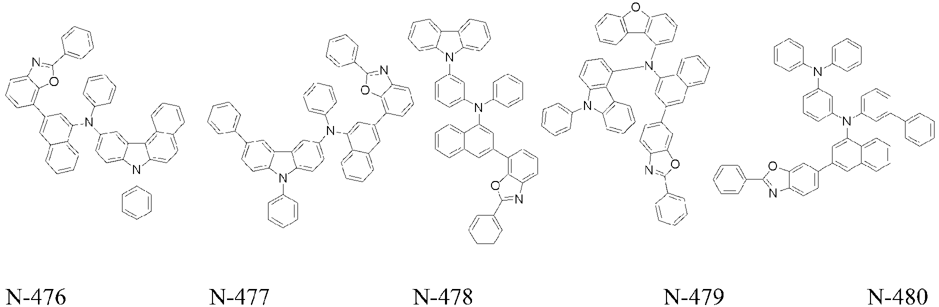

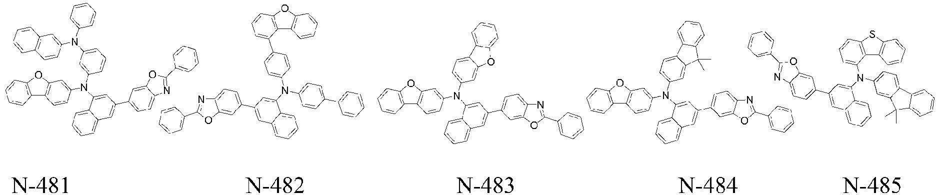

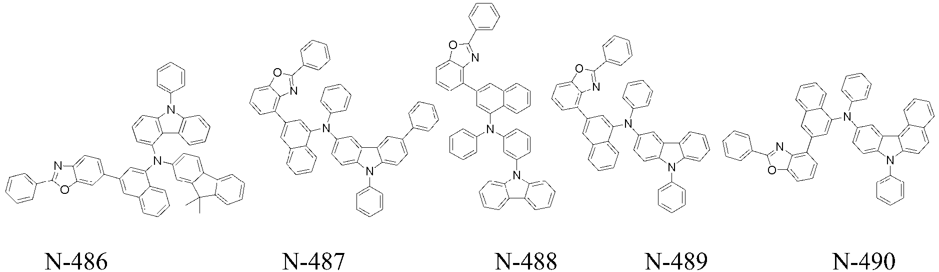

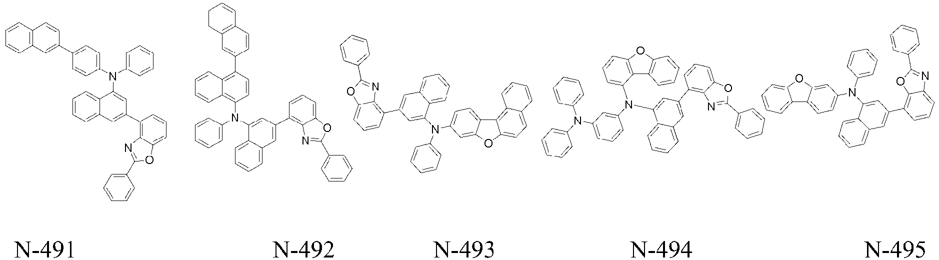

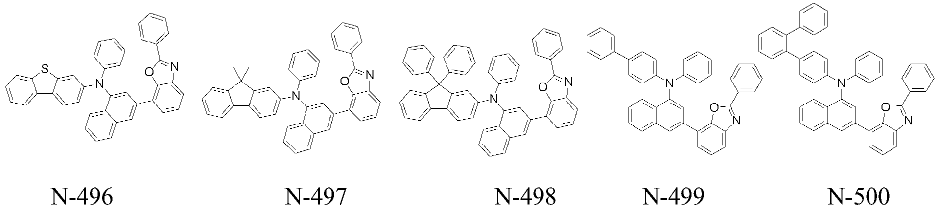

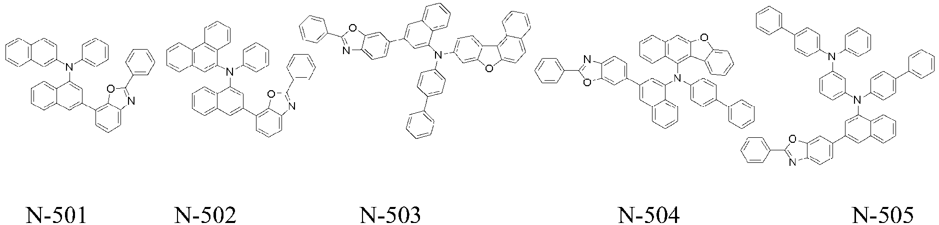

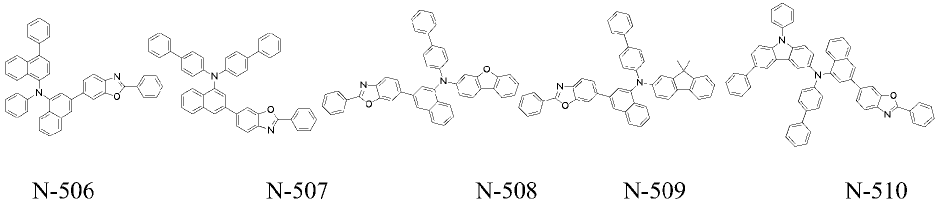

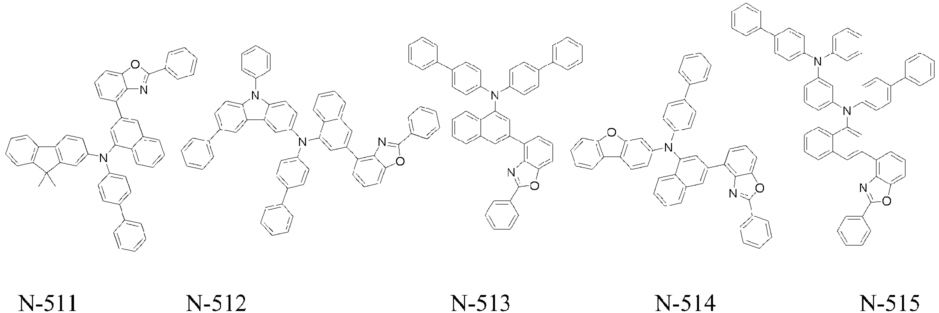

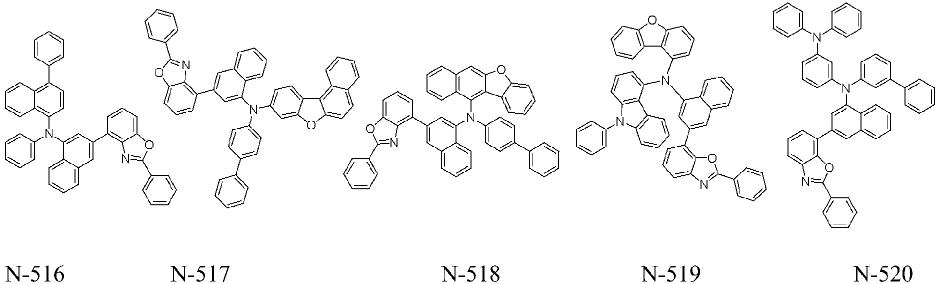

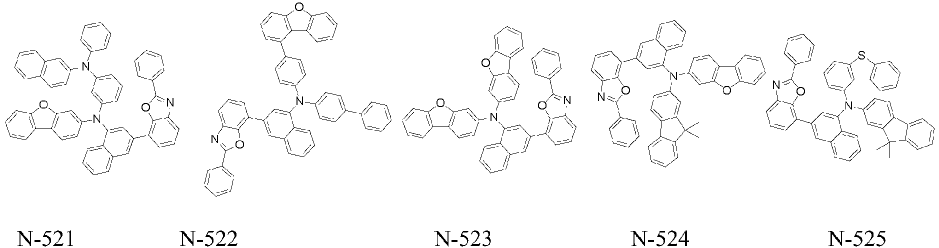

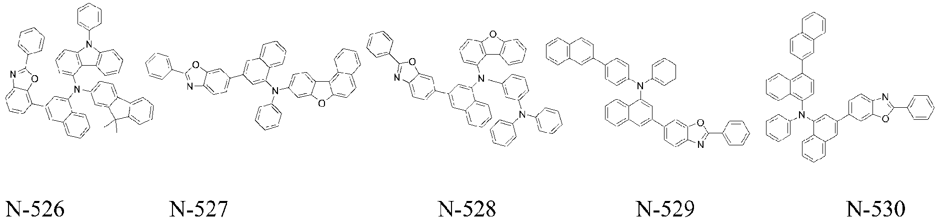

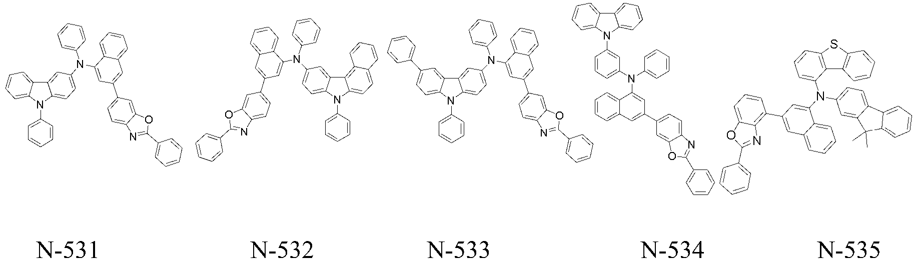

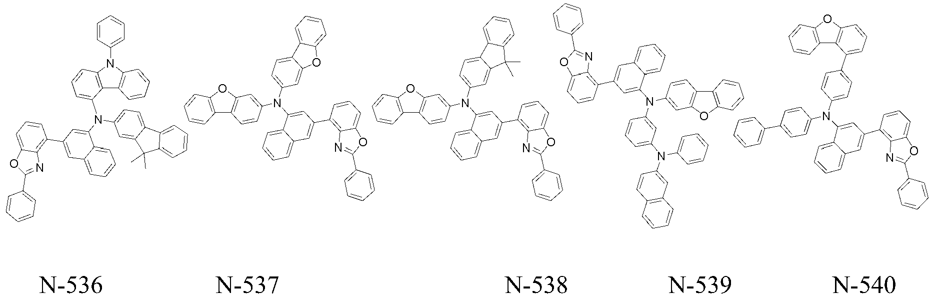

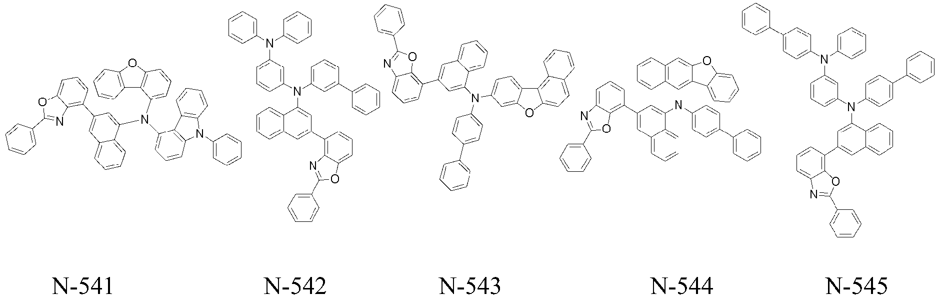

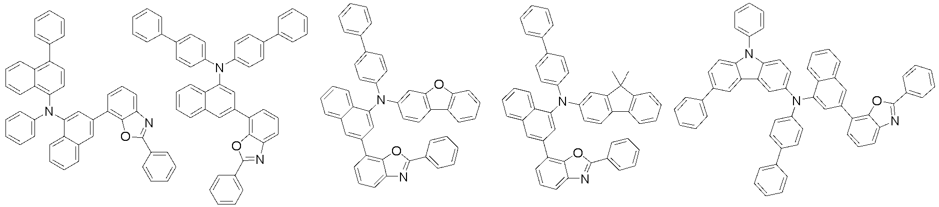

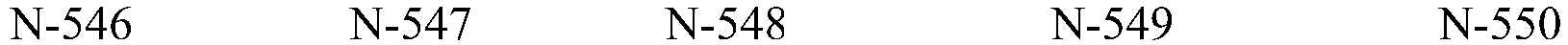

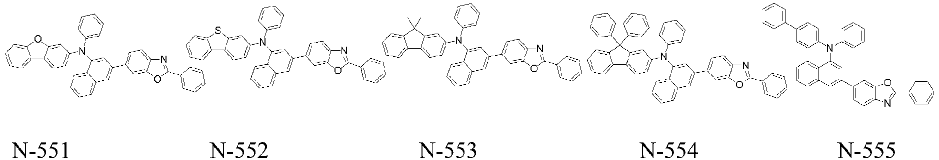

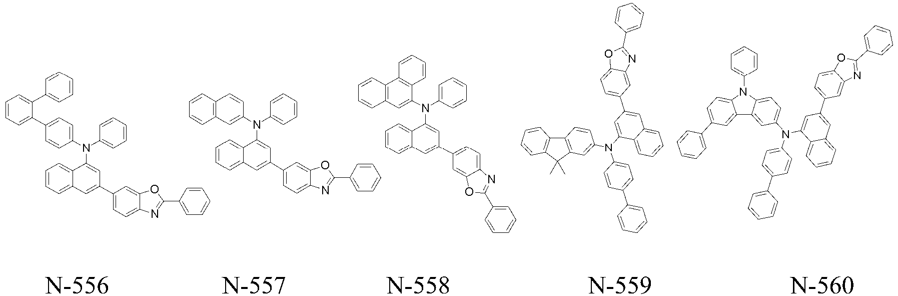

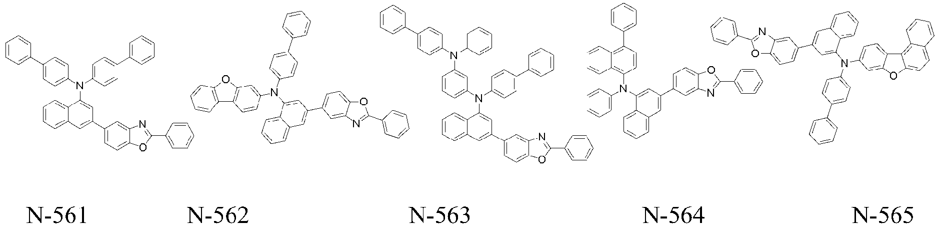

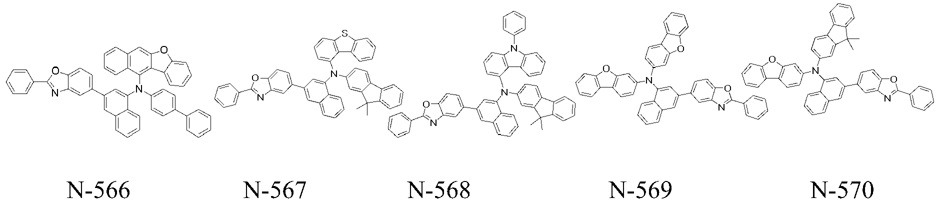

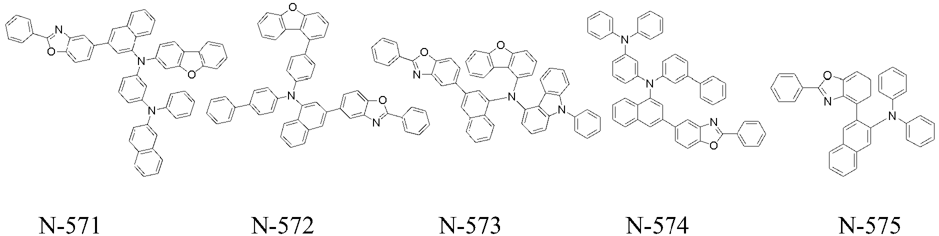

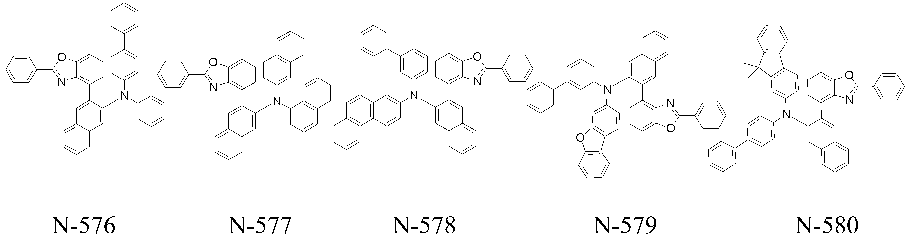

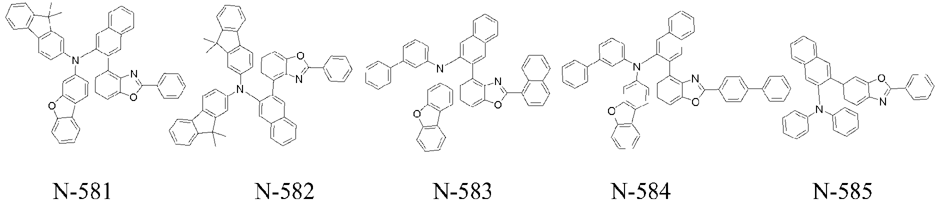

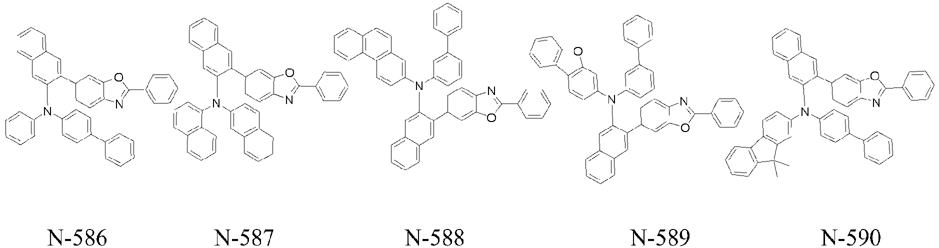

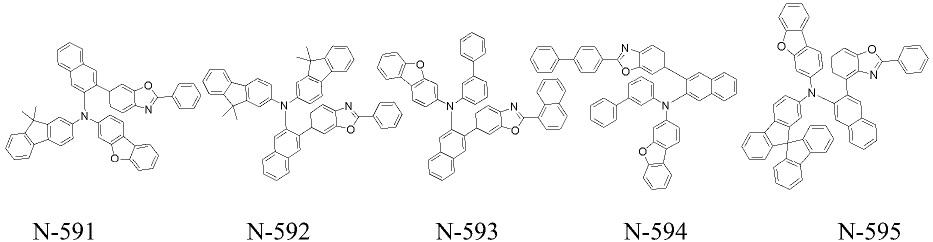

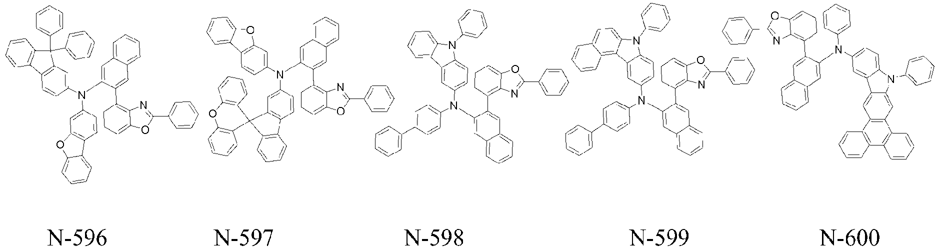

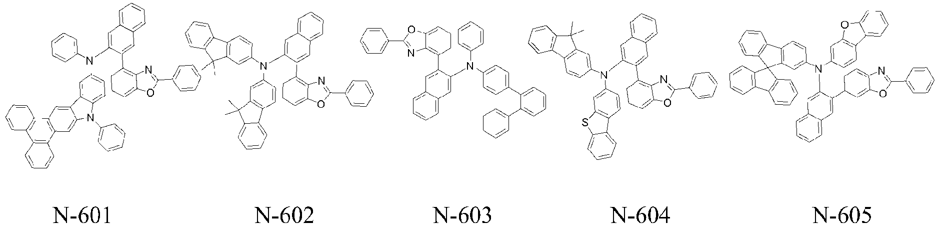

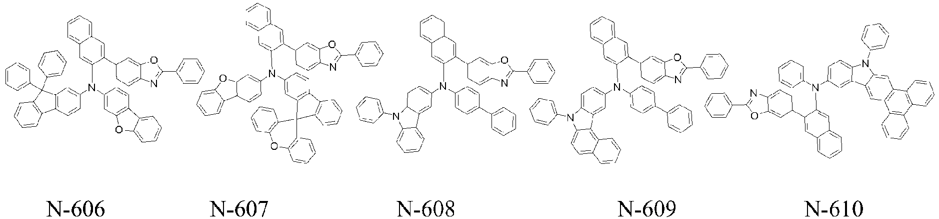

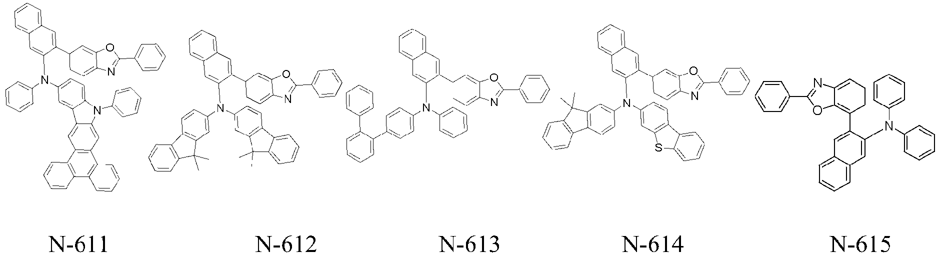

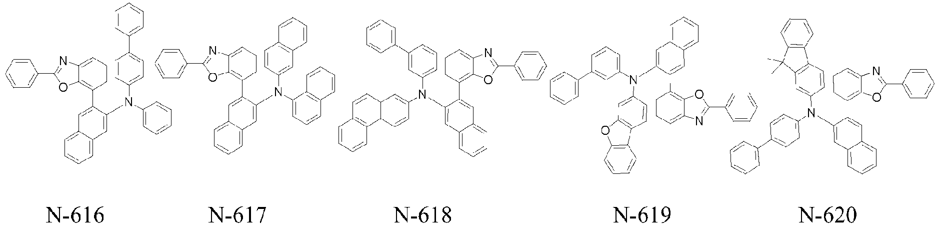

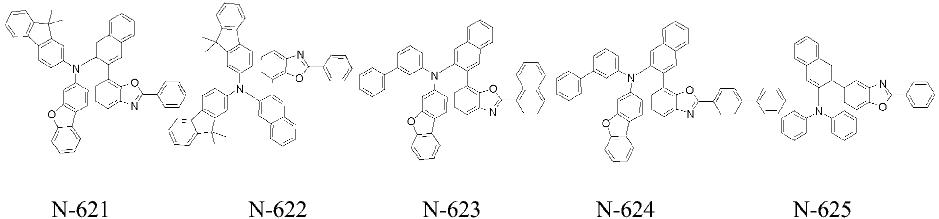

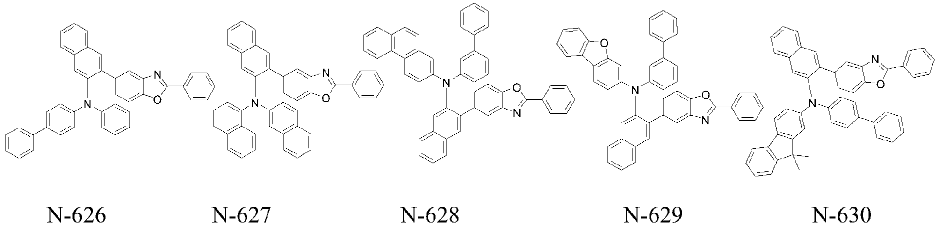

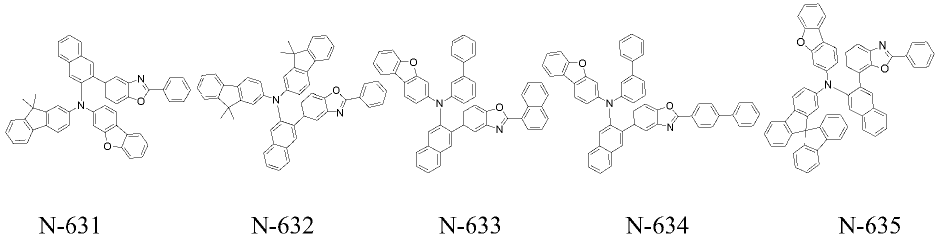

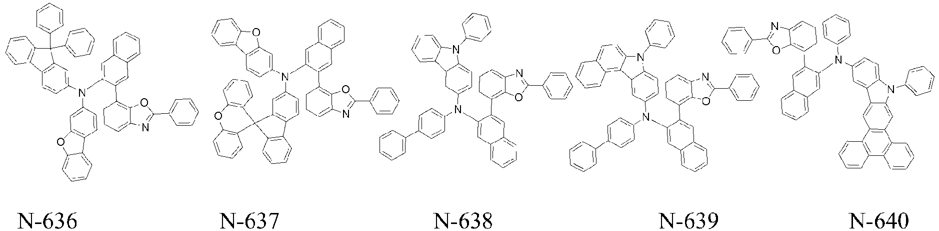

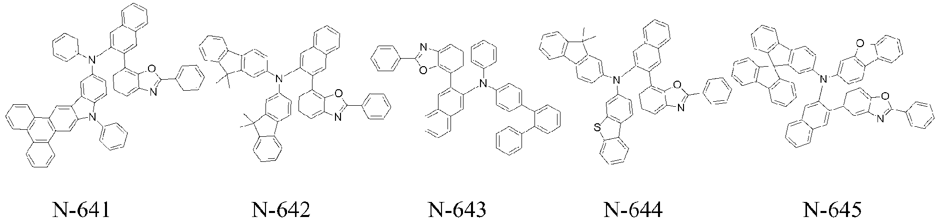

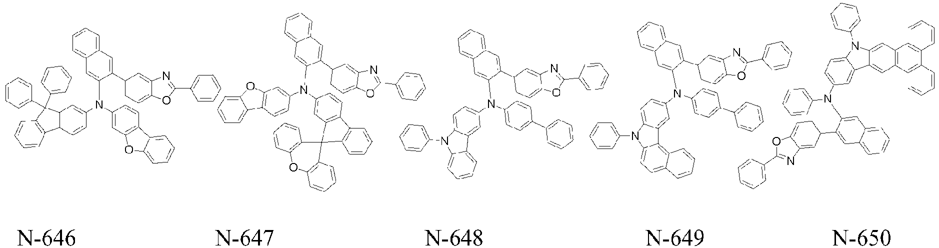

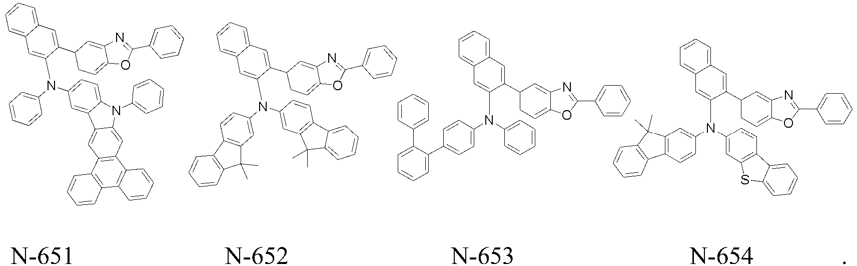

11. The light-emitting host material composition according to any one of claims 8 to 10, wherein a mass ratio of the first host material to the second host material is 9:1 to 1:9;
optionally, the mass ratio of the first host material to the second host material is 2:8 to 8:2;
optionally, the mass ratio of the first host material to the second host material is 3:7 to 7:3; and
optionally, the mass ratio of the first host material to the second host material is further 4:6 to 6:4.

12. An organic electroluminescent material, comprising the deuterated compound according to any one of claims 1 to 7 or the light-emitting host material composition according to any one of claims 8 to 11.

13. An organic electroluminescent device, comprising a cathode, an anode, and an organic layer located between the cathode and the anode, wherein the organic layer comprises the deuterated compound according to any one of claims 1 to 7 or the light-emitting host material composition according to any one of claims 8 to 11.

14. Use of the organic electroluminescent device according to claim 13 in a fiber optic equipment, an illumination equipment, an electrophotographic photoreceptor device, a photoelectric converter, an organic solar cell, a switching device, an organic light-emitting field-effect transistor, an image sensor, or a dye laser.
